# EUROPEAN PATENT APPLICATION

(11) **EP 4 450 093 A1**
(43) Date of publication of application: **23.10.2024**
(21) Application number: 23168356.6
(22) Date of filing: 17.04.2023
(51) Int. Cl.: A61K 47/68, A61P 35/00, A61P 37/00, C07K 14/54, C07K 16/28, C07K 16/32, C07K 19/00

(54) **CLEAVABLE IMMUNE CELL ENGAGERS**

(71) Applicant: Synaffix B.V., 5349 AB Oss (NL)
(72) Inventor: The designation of the inventor has not yet been filed
(74) Representative: Nederlandsch Octrooibureau

(57) **Abstract**

The present invention concerns antibody-cytokine conjugates, wherein the cytokine is conjugated to the antibody through a cleavable linker. The conjugates according to the invention can be prepared by conjugating a functionalized antibody Ab(F)ₓ containing x reactive moieties F, wherein x is an integer in the range 1 -10, and an immune cell-engaging polypeptide containing one or two reactive moieties Q, wherein the antibody is specific for a tumour cell and the immune cell-engaging polypeptide is specific for an immune cell, wherein the reaction forms a covalent linkage between the functionalized antibody and the immune cell-engaging polypeptide by reaction of Q with F. The invention further concerns the antibody-cytokine conjugate obtainable by the process according to the invention and medical uses thereof.

## Description

### Field of the invention

The present invention relates to immune cell engagers generated from antibodies and other polypeptides. More specifically the invention relates to conjugates, compositions and methods suitable for the attachment of an immune cell-binding polypeptide of interest to an antibody via a cleavable linker without requiring genetic engineering of the antibody before such attachment. The resulting antibody-immune cell engager conjugates as compounds, compositions, and methods can be useful, for example, in immunotherapy for cancer patients.

### Background of the Invention

Antibody-drug conjugates (ADC), considered as magic bullets in therapy, are comprised of an antibody to which is attached a pharmaceutical agent. The antibodies (also known as ligands) can be small protein formats (scFv's, Fab fragments, DARPins, Affibodies, etc.) but are generally monoclonal antibodies (mAbs) which have been selected based on their high selectivity and affinity for a given antigen, their long circulating half-lives, and little to no immunogenicity. Thus, mAbs as protein ligands for a carefully selected biological receptor provide an ideal delivery platform for selective targeting of pharmaceutical drugs. For example, a monoclonal antibody known to bind selectively with a specific cancer-associated antigen can be used for delivery of a chemically conjugated cytotoxic agent to the tumour, via binding, internalization, intracellular processing and finally release of active catabolite. The cytotoxic agent may be small molecule toxin, a protein toxin or other formats, like oligonucleotides. As a result, the tumour cells can be selectively eradicated, while sparing normal cells which have not been targeted by the antibody. Similarly, chemical conjugation of an antibacterial drug (antibiotic) to an antibody can be applied for treatment of bacterial infections, while conjugates of anti-inflammatory drugs are under investigation for the treatment of autoimmune diseases and for example attachment of an oligonucleotide to an antibody is a potential promising approach for the treatment of neuromuscular diseases. Hence, the concept of targeted delivery of an active pharmaceutical drug to a specific cellular location of choice is a powerful approach for the treatment of a wide range of diseases, with many beneficial aspects versus systemic delivery of the same drug.

An alternative strategy to employ monoclonal antibodies for targeted delivery of a specific protein agent is by genetic fusion of the latter protein to one (or more) of the antibody's termini, which can be the N-terminus or the C-terminus on the light chain or the heavy chain (or both). In this case, the biologically active protein of interest, e.g. a protein toxin like *Pseudomonas* exotoxin A (PE38) or an anti-CD3 single chain variable fragment (scFv), is genetically encoded as a fusion to the antibody, possibly but not necessarily via a peptide spacer, so the antibody is expressed as a fusion protein. The peptide spacer may contain a protease-sensitive cleavage site, or not.

A monoclonal antibody may also be genetically modified in the protein sequence itself to modify its structure and thereby introduce (or remove) specific properties. For example, mutations can be made in the antibody Fc-fragment in order to nihilate binding to Fc-gamma receptors, binding to the FcRn receptor or binding to a specific cancer target may be modulated, or antibodies can be engineered to lower the pl and control the clearance rate from circulation.

An emerging strategy in therapeutic treatment involves the use of an antibody that is able to bind simultaneously to multiple antigens or epitopes, a so-called bispecific antibody (simultaneously addressing two different antigens or epitopes), or a trispecific antibody (addressing three different antigens of epitopes), and so forth, as summarized in Kontermann and Brinkmann, Drug Discov. Today 2015, 20, 838-847, incorporated by reference. A bispecific antibody with 'two-target' functionality can interfere with multiple surface receptors or ligands associated, for example with cancer, proliferation or inflammatory processes. Bispecific antibodies can also place targets into close proximity, either to support protein complex formation on one cell, or to trigger contacts between cells. Examples of 'forced-connection' functionalities are bispecific antibodies that support protein complexation in the clotting cascade, or tumor-targeted immune cell recruiters and/or activators. Depending on the production method and structure, bispecific antibodies vary in the number of antigen-binding sites, geometry, half-life in the blood serum, and effector function.

A wide range of different formats for multispecific antibodies has been developed over the years, which can be roughly divided into IgG-like (bearing a Fc-fragment) and non-IgG-like (lacking a Fc-fragment) formats, as summarized by Kontermann and Brinkmann, Drug Discov. Today 2015, 20, 838-847 and Yu and Wang, J. Cancer Res. Clin. Oncol. 2019, 145, 941-956, incorporated by reference. Most bispecific antibodies are generated by one of three methods by somatic fusion of two hybridoma lines (quadroma), by genetic (protein/cell) engineering, or by chemical conjugation with cross-linkers, totalling more than 60 different technological platforms today.

IgG-like formats based on full IgG molecular architectures include but are not limited to IgG with dual-variable domain (DVD-lg), Duobody technology, knob-in-hole (KIH) technology, common light chain technology and cross-mAb technology, while truncated IgG versions include ADAPTIR, XmAb and BEAT technologies. Non-IgG-like approaches include but are not limited BITE, DART, TandAb and ImmTAC technologies. Bispecific antibodies can also be generated by fusing different antigen-binding moieties (e.g., scFv or Fab) to other protein domains, which enables further functionalities to be included. For example, two scFv fragments have been fused to albumin, which endows the antibody fragments with the long circulation time of serum albumin, as demonstrated by Müller et al., J. Biol. Chem. 2007, 282, 12650-12660, incorporated by reference. Another example is the `dock-and-lock' approach based on heterodimerization of cAMP-dependent protein kinase A and protein A kinase-anchoring protein, as reported by Rossi et al., Proc. Nat. Acad. Sci. 2006, 103, 6841-6846, incorporated by reference. These domains can be linked to Fab fragments and entire antibodies to form multivalent bispecific antibodies, as shown by Rossi et al., Bioconj. Chem. 2012, 23, 309-323. The dock-and-lock strategy requires the generation of a fusion protein between the targeting antibody and a peptide fragment for docking onto the protein A kinase-anchoring protein. Therapeutic Ab fragments (scFv, diabody) may also be fused with albumin or proteins that bind albumin, which increases the half-life of the drug in the blood up to five to six times. The construction of such molecules gives unpredictable results, thereby bispecific antibodies generated as the result of different Ab-fragment fusion or binding of Abs to other proteins have limited application in research and development of new therapeutic molecules.

Chemical conjugation to generate a non-lgG-type bispecific antibody was used for the first time by Brennan et al., Science 1985, 229, 81-83, incorporated by reference: two Fab₂ fragments obtained by pepsinolysis of rabbit IgG were reduced and then oxidized, resulting in bispecific Fab₂. Similarly, homo-and heterobifunctional reagents interacting with cysteine residues was reported by Glennie *et al.* **1987,** 139, 2367-2375, incorporated by reference. Chemical conjugation of Abs against CD3 and CD20 (rituximab) was used to obtain T cells with bispecific antibody-coated surfaces, as shown by Gall et al., Exp. Hematol. 2005, 33, 452-459, incorporated by reference. Generation of the bispecific CD20 × CD3 was ensured by treatment of OKT3 (anti-CD3) with Traut's reagent, followed by mixing with maleimide-functionalized rituximab (obtained by pretreament of rituximab with sulfosuccinimidyl 4-(N-maleimidomethyl)cyclohexane-1-carboxylate (sulfo-SMCC). By virtue of the random chemical conjugation of both antibodies, followed by random heterodimerization, the bispecific antibody is inevitable obtained as a highly heterogeneous mixture (also containing multimers). The only chemical method reported to date that is also site-specific is the CovX-Body technology, as reported by Doppalapudi et al., Bioorg. Med. Chem. Lett. 2007, 17, 501-506, incorporated by reference, based on the instalment of an aldolase catalytic antibody site into the the targeting antibody, followed by treatment with peptide fragment chemically modified with a azetidinone-motif, leading to spontaneous ligation. Bispecific antibodies were produced by the addition of two short peptides that inhibited VEGF or angiopoietin 2 with a branched linker and then with the Abs, as reported by Doppalapudi et al., Proc. Nat. Acad. Sci. 2010, 107, 22611-22616*,* incorporated by reference.

Formats of bispecific antibody generation based on chemical Ab or Ab-fragment conjugation today are not in use, in particular due to the low yield of product (of low purity) and high cost-of-goods. Besides, the advance in recombinant DNA technologies enabled the efficient generation of fusion proteins and positive clinical results were obtained therewith. Regardless, a non-genetic chemical modification approach could significantly accelerate time-to-clinic, in case proper control of site-specificity of stoichiometry can be ensured.

Examples of bispecific antibodies that have been or are currently under clinic development are catumaxomab (EpCAM × CD3), blinatumomab (CD19 × CD3), GBR1302 (Her2 × CD3), MEDI-565 (CEA × CD3), BAY2010112 (PSMA × CD3), RG7221 (angiopoietin × VEGF), RG6013 (FIX × FX), RG7597 (Her1 × Her3), MCLA128 (Her2 × Her3), MM111 (Her2 × Her3), MM141 (IGF1R × Her3), ABT122 (TNFalpha × IL17), ABT981 (IL1a × II1b), ALX0761 (IL17A × IL17F), SAR156597 (IL4 × IL13), AFM13 (CD30 × CD16) and LY3164530 (Her1 × cMET).

A popular strategy in the field of cancer therapy employs a bispecific antibody binding to an upregulated tumor-associated antigen (TAA or simply target) as well as to a receptor present on a cancer-destroying immune cell. *e.g.* a T cell or an NK cell. Such bispecific antibodies are also known as T cell or NK cell-redirecting antibodies, respectively. Although the approach of immune cell redirecting is already more than 30 years old, new technologies are overcoming the limitations of the 1^{st} generation immune cell-redirecting antibodies, especially extending half-life to allow intermittent dosing, reducing immunogenicity and improving the safety profile. Currently, there is one approved drug (blinatumomab or Blincyto^{®}) and more than 30 other bispecific formats in various stages of clinical development. The basis for the approval of blinatumomab (2014) resulted from a single-arm trial with a 32% complete remission rate and a minimal residual disease (MRD) response (31%) in all patients treated. Currently, 51 clinical trials of blinatumomab are being carried out for ALL (39 trials), NHL (10 trials), multiple myeloma (1 trial) and lymphoid cancer with Richter's transformation (1 trial). However, Blinatumomab suffers from a main drawback because of its short serum half-life (2.11 h, due to the relatively small molecule and simple structure), and patients require continuous intravenous infusion.

Like other methods of therapy for severe diseases, therapeutic bispecific antibodies cause different side effects, the most common of which are nausea, vomiting, abdominal pain, fatigue, leukopenia, neutropenia, and thrombopenia. In many patients, Abs against therapeutic bispecific antibodies appear in the blood during treatment. Most adverse events occur during the beginning of therapy, and in most cases side effects normalize under continued treatment. The majority of data on therapeutic BsAb adverse effects are available on blinatumomab and catumaxomab, since these drugs have undergone numerous clinical trials. A common side effect of blinatumomab and catumaxomab therapy is "cytokine storm", elevation of cytokine levels and some neurological events. Cytokine release-related symptoms are general side effects of many therapeutic mAbs and occur due to specific mechanisms of action: use of cytotoxic T cells as effectors. Minimizing cytokine-release syndrome is possible with a low initial dose of the drug in combination with subsequent high doses, as well as corticosteroid (dexamethasone) and antihistamine premedication.

One way to mitigate the adverse events associated with immune cell engagement therapy, in particular cytokine release syndrome, and to avoid the use of step-up-dosing regimens, was reported by Bacac et al., Clin. Cancer Res. 2018, 24, 4785-4797, incorporated by reference. It was shown that with significantly higher potency and safer administration could be achieved by generating a CD20 × CD3 T cell engager with a 2:1 molecular format, i.e. bivalent binding to CD20 and monovalent binding to CD3, which is achieved by insertion of the anti-CD3 fragment in one of the Fab arms of the full-IgG anti-CD20 antibody. The resulting bispecific antibody is associated with a long half-life and high potency enabled by high-avidity bivalent binding to CD20 and head-to-tail orientation of B- and T cell-binding domains in a 2:1 molecular format. A heterodimeric human IgG1 Fc region carrying the "PG LALA" mutations was incorporated to abolish binding to Fcg receptors and to complement component C1q while maintaining neonatal Fc receptor (FcRn) binding, enabling a long circulatory half-life. The bispecific CD20-T cell engagers displays considerably higher potency than other CD20-TCB antibodies in clinical development and is efficacious on tumor cells expressing low levels of CD20. CD20-TCB also displays potent activity in primary tumor samples with low effector:target ratios.

By far the most investigated receptor for the purpose of T cell-engagement involves the CD3 receptor on activated T cells. T cell-redirecting bispecific antibodies are amongst the most used approaches in cancer treatment and the first report in which bispecific antibodies specifically engaged CD3 on T cells on one side and the antigens of cancer cells independent of their T cell receptor (TCR) on the other side, was published 30 years ago. T cell-redirecting antibodies have made considerable progress in hematological malignancies and solid tumour treatments in the past 10 years. Catumaxomab is the first bispecific antibody of its kind targeting epithelial cell adhesion molecule (EpCAM) and CD3, which was approved in Europe (2009) for the treatment of malignant ascites (but withdrawn in 2017 for commercial reasons). This discovery was followed by another successful bispecific targeting CD19 and CD3 (blinatumomab), which was given marketing permission by the FDA for relapsed or refractory precursor B-cell acute lymphoblastic leukemia (ALL) treatment in 2014. At present, although many patients benefit from blinatumomab, there are a number of T cell-redirecting antibodies with different formats and characteristics showing potential anti-tumour efficacy in clinical studies.

The concept of redirecting T cells to the tumor is currently expanded to other receptors, which are at the same time costimulatory, such as CD137 (4-1 BB), CD134 (OX40), CD27 or ICOS.

In the field of CD137 targeting, agonistic monoclonal antibodies (so not bispecific) have shown much preclinical promise but their clinical development has been slow due to a poor therapeutic index, in particular liver toxicity. CD137 is expressed on T cells that are already primed to recognize tumor antigen through MHC/TCR interaction. It is a TNFRSF (tumor necrosis factor receptor super family) member which requires clustering to deliver an activating signal to T cells. Monospecific monoclonal antibodies that can agonise CD137 are in the clinic and known to be potent T cell activators but suffer from treatment-limiting hepatotoxicity due to Fc-receptor and multivalent format-driven clustering. Bispecific tumor-targeted antibodies that are monovalent for CD137, are unable to cause CD137 clustering in normal tissue. Only upon binding of the bispecific antibody to a tumor-associated antigen on tumor cells, clustering of co-engaged CD137 on tumor-associated T cells is induced. This drives a highly potent but tumor-specific T cell activation. The tumor-targeted cross-linking of Cd137/4-1 BB might provide a safe and effective way for co-stimulation of T cells for cancer immunotherapy and its combination with T cell bispecific antibodies may provide a convenient "off-the-shelf," systemic cancer immunotherapy approach for many tumor types. Examples of anti-CD137-based bispecific antibodies in clinical development include MP0310 (FAP × CD137), RG7827 (FAP × CD137), ALG.APV-527 (5T4 × CD137), MCLA145 (PD-1 × CD137), PRS342 (glypican-3 × CD137), PRS-343 (Her2 × CD137), CB307 (PSMA × CD137). Various of the above bispecifics are deliberately chosen as monovalent for CD137 and as such is unable to cause CD137 clustering in normal tissue. For example, only after binding of the bispecific CB307 to PSMA on tumor cells, it causes clustering of co-engaged CD137 on tumour-associated T cells, thereby driving a highly potent but tumor-specific T cell activation.

Antibodies known to bind T cells are known in the art, highlighted by Martin et al., Clin. Immunol. 2013, 148, 136-147 and Rossi et al., Int. Immunol. 2008, 20, 1247-1258, both incorporated by reference, for example OKT3, UCHT3, BMA031 and humanized versions thereof. Antibodies known to bind to V□9V□2 T cells are also known, see for example de Bruin et al., J. Immunol. 2017, 198, 308-317, incorporated by reference.

Similar to T cell engagement, NK cell recruitment to the tumor microenvironment is under broad investigation. NK cell engagement is typically based on binding CD16, CD56, NKp46, or other NK cell specific receptors, as summarized in Konjevic et al., **2017,** http://dx.doi.org/10.5772/intechopen.69729, incorporated by reference. NK cell engagers can be generated by fusion or insertion of an NK-binding antibody (fragment) to a full IgG binding to a tumor-associated antigen. Alternatively, specific cytokines can also be employed, given that NK cell antitumor activity is regulated by numerous activating and inhibitory NK cell receptors, alterations in NK cell receptor expression and signaling underlie diminished cytotoxic NK cell function. Based on this and on predictive in vitro findings, cytokines including IFNα, IL-2, IL-12, IL-15, and IL-18 have been used systemically or for ex vivo activation and expansion of NK cells and have led to improved NK cells antitumor activity by increasing the expression of NK cell activating receptors and by inducing cytotoxic effector molecules. Moreover, this cytokine-based therapy enhances NK cell proliferation and regulatory function, and it has been shown that it induces NK cells exhibiting cytokine induced memory-like properties that represent a newly defined NK cell subset with improved NK cell activity and longevity. Both for cancer therapy as well as for the treatment of chronic inflammation, several cytokine payloads have been developed and tested in preclinical trials. Proinflammatory cytokines such as IL-2, TNF and IL-12 have been investigated for tumor therapy, as they have been found to increase and activate the local infiltrate of leukocytes at the tumor site. For example, IL-2 monotherapy has been approved as aldesleukin (Proleukin^{®}) and is in phase III clinical trials in combination with nivolumab (NKTR-214). Similarly, various recombinant versions of IL-15 are under clinical evaluation (rhIL-15 or ALT-803). Specific mutants of IL-15 have been reported, for example by Behár et al., Prot. Engin. Des. Sel. 2011, 24, 283-290 and Silva et al., Nature 2019, 565, 186-191, both incorporated by reference, and the complex of IL-15 with IL-15 receptor (IL-15R), as reported by Rubinstein et al., Proc. Nat. Acad. Sci. 2006, 103, 9166-9171, incorporated by reference and fusion constructs of IL-15 and IL-15R (Sushi domain) have also been evaluated for antitumor activity, see for example Bessard et al., Mol. Canc. Ther. 2009, 8, 2736-2745, incorporated by reference. By contrast immunosuppressive cytokines such as IL-10 may be considered as payloads for the treatment of chronic inflammatory conditions or of other diseases (e.g., endometriosis).

Systemic administration of pro-inflammatory cytokines can lead to severe off-target-related adverse effects, which may limit the dose and prevent escalation to therapeutically active regimens. Certain cytokine products (e.g., IL-2, TNF, IL-12) have exhibited recommended doses in the single-digit milligram range (per person) or even below. Adverse effects associated with the intravenous administration of pro-inflammatory cytokines may include hypotension, fever, nausea or flu-like symptoms, and may occasionally also cause serious haematologic, endocrine, autoimmune or neurologic events. In view of these considerations, there is a clear biomedical need for the development of 'next-generation' cytokine products, which are better tolerated and which display a preferential action at the site of disease, helping to spare normal tissues, as summarized in Murer and Neri, New Biotechnol. 2019, 52, 42-53, incorporated by reference. Thus, the targeted delivery of cytokines to the tumor aims at inducing a local pro-inflammatory environment, which may activate and recruit immune cells. A list of antibody-cytokine fusions described in the literature has been reported by Hutmacher and Neri, Adv. Drug Deliv. Rev. 2018, 141, 67-91, incorporated by reference. A list of clinical cytokine fusions is provided in Murer and Neri, New Biotechnol. 2019, 52, 42-53, incorporated by reference. Various IL-15 fusions proteins are under preclinical evaluation, as summarized in "T-cell & NK-Cell Engaging Bispecific Antibodies 2019: A Business, Stakeholder, Technology and Pipeline Analysis", 2019, released by La Merie publishing, incorporated by reference, for example OXS-3550 (CD33-IL-15-CD16 fusion) prepared by Trike technology close to phase I evaluation.

A common strategy in the field of immune cell engagement employs nihilation or removal of binding capacity of the antibody to Fc-gamma receptors, which has multiple pharmaceutical implications. The first consequence of removal of binding to Fc-gamma receptors is the reduction of Fc-gamma receptor-mediated uptake of antibodies by e.g. macrophages or megakaryocytes, which may lead to dose-limiting toxicity as for example reported for Kadcyla^{®} (trastuzumab-DM1) and LOP628. Selective deglycosylation of antibodies in vivo affords opportunities to treat patients with antibody-mediated autoimmunity. Removal of high-mannose glycoform in a recombinant therapeutic glycoprotein may be beneficial, since high-mannose glycoforms are known to compromise therapeutic efficacy by aspecific uptake by endogenous mannose receptors and leading to rapid clearance, as for example described by Gorovits and Krinos-Fiorotti, Cancer Immunol. Immunother. 2013, 62, 217-223 and Goetze et al, Glycobiology 2011, 21, 949-959 (both incorporated by reference). In addition, Van de Bovenkamp et al, J. Immunol. 2016, 196, 1435-1441 (incorporated by reference) describe how high mannose glycans can influence immunity. It was described by Reusch and Tejada, Glycobiology 2015, 25, 1325-1334 (incorporated by reference), that inappropriate glycosylation in monoclonal antibodies could contribute to ineffective production from expressed Ig genes.

In the field of immune therapy, binding of glycosylated antibodies to Fc-gamma receptors on immune cells may induce systemic activation of the immune system, prior to binding of the antibody to the tumor-associated antigen, leading to cytokine storm (cytokine release syndrome, CRS). Therefore, in order to reduce the risk of CRS, the vast majority of immune cell engagers in the clinic are based on Fc-silenced antibodies, lacking the capacity to bind to Fc-gamma receptors. In addition, various companies in the field of bispecific antibodies are tailoring molecular architectures with defined ratios with regard to target-binding versus immune cell-engaging antibody domains. For example, Roche is developing T cell-engagers based on asymmetric monoclonal antibodies that retain bivalent binding capacity to the TAA (for example CD20 or CEA) by both CDRs, but with an additional anti-CD3 fragment engineered into one of the two heavy chains only (2:1 ratio of target-binding:CD3-binding). Similar strategies can be employed for engagement/activation of T cells with anti-CD137 (4-1BB), anti-OX40, anti-CD27 or NK cell-engagement/activation with anti-CD16, CD56, NKp46, or other NK cell specific receptors.

Abrogation of binding to Fc-gamma receptor can be achieved in various ways, for example by specific mutations in the antibody (specifically the Fc-fragment) or by removal of the glycan that is naturally present in the Fc-fragment (C_{H}2 domain, around N297). Glycan removal can be achieved by genetic modification in the Fc-domain, e.g. a N297Q mutation or T299A mutation, or by enzymatic removal of the glycan after recombinant expression of the antibody, using for example PNGase F or an endoglycosidase. For example, endoglycosidase H is known to trim high-mannose and hybrid glycoforms, while endoglycosidase S is able to trim complex type glycans and to some extent hybrid glycan. Endoglycosidase S2 is able to trim both complex, hybrid and high-mannose glycoforms. Endoglycosidase F2 is able to trim complex glycans (but not hybrid), while endoglycosidase F3 can only trim complex glycans that are also 1 ,6-fucosylated. Another endoglycosidase, endoglycosidase D is able to hydrolyze Man5 (M5) glycan only. An overview of specific activities of different endoglycosidases is disclosed in Freeze et al. in Curr. Prot. Mol. Biol., 2010, 89:17.13A.1-17, incorporated by reference herein. An additional advantage of deglycosylation of proteins for therapeutic use is the facilitated batch-to-batch consistency and significantly improved homogeneity.

Inspiration may be taken from the field of ADC technologies to prepare antibody-protein conjugates for the generation of bispecific antibodies or antibody-cytokine fusions.

Many technologies are known for bioconjugation, as summarized in G.T. Hermanson, "Bioconjugate Techniques", Elsevier, 3rd Ed. 2013, incorporated by reference. Two main technologies can be recognized for the preparation of ADCs by random conjugation, either based on acylation of lysine side-chain or based on alkylation of cysteine side-chain. Acylation of the □-amino group in a lysine side-chain is typically achieved by subjecting the protein to a reagent based on an activated ester or activated carbonate derivative, for example SMCC is applied for the manufacturing of Kadcyla^{®}. Main chemistry for the alkylation of the thiol group in cysteine side-chain is based on the use of maleimide reagents, as is for example applied in the manufacuting of Adcetris^{®}. Besides standard maleimide derivatives, a range of maleimide variants are also applied for more stable cysteine conjugation, as for example demonstrated by James Christie et al., J. Contr. Rel. 2015, 220, 660-670 and Lyon et al., Nat. Biotechnol. 2014, 32, 1059-1062, both incorporated by reference. Another important technology for conjugation to cysteine side-chain is by means of disulfide bond, a bioactivatable connection that has been utilized for reversibly connecting protein toxins, chemotherapeutic drugs, and probes to carrier molecules (see for example Pillow et al., Chem. Sci. 2017, 8, 366-370. Other approaches for cysteine alkylation involve for example nucleophilic substitution of haloacetamides (typically bromoacetamide or iodoacetamide), see for example Alley et al., Bioconj. Chem. 2008, 19, 759-765, incorporated by reference, or various approaches based on Michael addition on unsaturated bonds, such as reaction with acrylate reagents, see for example Bernardim et al., Nat. Commun. 2016, 7, DOI: 10.1038/ncomms13128 and Ariyasu et al., Bioconj. Chem. 2017, 28, 897-902, both incorporated by reference, reaction with phosphonamidates, see for example Kasper et al., Angew. Chem. Int. Ed. 2019, 58, 11625-11630, incorporated by reference, reaction with allenamides, see for example Abbas et al., Angew. Chem. Int. Ed. 2014, 53, 7491-7494, incorporated by reference, reaction with cyanoethynyl reagents, see for example Kolodych et al., Bioconj. Chem. 2015, 26, 197-200, incorporated by reference, reaction with vinylsulfones, see for example Gil de Montes et al., Chem. Sci. 2019, 10, 4515-4522, incorporated by reference, or reaction with vinylpyridines, see for example https://iksuda.com/science/permalink/ (accessed Jan. 7th, 2020). Reaction with methylsulfonylphenyloxadiazole has also been reported for cysteine conjugation by Toda et al., Angew. Chem. Int. Ed. 2013, 52, 12592-12596, incorporated by reference.

A number of processes have been developed that enable the generation of an antibody-drug conjugate with defined drug-to-antibody ratio (DAR), by site-specific conjugation to a (or more) predetermined site(s) in the antibody. Site-specific conjugation is typically achieved by engineering of a specific amino acid (or sequence) into an antibody, serving as the anchor point for payload attachment, see for example Aggerwal and Bertozzi, Bioconj. Chem. 2014, 53, 176-192, incorporated by reference, most typically engineering of cysteine. Besides, a range of other site-specific conjugation technologies has been explored in the past decade, most prominently genetic encoding of a non-natural amino acid, *e.g. p-*acetophenylalanine suitable for oxime ligation, or *p*-azidomethylphenylalanine suitable for click chemistry conjugation. The majority of approaches based on genetic reengineering of an antibody lead to ADCs with a DAR of ~2. An alternative approach to antibody conjugation without reengineering of antibody involves the reduction of interchain disulfide bridges, followed addition of a payload attached to a cysteine cross-linking reagent, such as bis-sulfone reagents, see for example Balan et al., Bioconj. Chem. 2007, 18, 61-76 and Bryant et al., Mol. Pharmaceutics 2015, 12, 1872-1879, both incorporated by reference, mono- or bis-bromomaleimides, see for example Smith et al., J. Am. Chem. Soc. 2010, 132, 1960-1965 and Schumacher et al., Org. Biomol. Chem. 2014, 37, 7261-7269, both incorporated by reference, bismaleimide reagents, see for example WO2014114207, bis(phenylthio)maleimides, see for example Schumacher et al., Org. Biomol. Chem. 2014, 37, 7261-7269 and Aubrey et al., Bioconj. Chem. 2018, 29, 3516-3521, both incorporated by reference, bis-bromopyridazinediones, see for example Robinson et al., RSC Advances 2017, 7, 9073-9077, incorporated by reference, bis(halomethyl)benzenes, see for example Ramos-Tomillero et al., Bioconj. Chem. 2018, 29, 1199-1208, incorporated by reference or other bis(halomethyl)aromatics, see for example WO2013173391. Typically, ADCs prepared by cross-linking of cysteines have a drug-to-antibody loading of ~4 (DAR4).

Ruddle et al., ChemMedChem 2019, 14, 1185-1195 have recently shown that DAR1 conjugates can be prepared from antibody Fab fragments (prepared by papain digestion of full antibody or recombinant expression) by selective reduction of the C_{H}1 and C_{L} interchain disulfide chain, followed by rebridging the fragment by treatment with a symmetrical PDB dimer containing two maleimide units. The resulting DAR1-type Fab fragments were shown to be highly homogeneous, stable in serum and show excellent cytotoxicity. In a follow-up publication, White etal., MAbs 2019, 11, 500-515, and also in WO2019034764, incorporated by reference, it was shown that DAR1 conjugates can also be prepared from full IgG antibodies, after prior engineering of the antibody: either an antibody is used which has only one intrachain disulfide bridge in the hinge region (Flexmab technology, reported in Dimasi et al., J. Mol. Biol. 2009, 393, 672-692, incorporated by reference) or an antibody is used which has an additional free cysteine, which may be obtained by mutation of a natural amino acid (e.g. HC-S239C) or by insertion into the sequence (*e.g*. HC-i239C, reported by Dimasi et al., Mol. Pharmaceut. 2017, 14, 1501-1516). Either engineered antibody was shown to enable the generation of DAR1 ADCs by reaction of the resulting cysteine-engineered ADC with a bismaleimide derived PBD dimer. It was shown that the Flexmab-derived DAR1 ADCs was highly resistant to payload loss in serum and exhibited potent antitumor activity in a HER2-positive gastric carcinoma xenograft model. Moreover, this ADC was tolerated in rats at twice the dose compared to a site-specific DAR2 ADC prepared using a single maleimide-containing PBD dimer. However, no improvement in therapeutic window was noted, since the minimal effective dose (MED) of the DAR1 ADC versus the DAR2 ADC increased with the same factor 2.

It has been shown in WO2014065661, and van Geel et al., Bioconj. Chem. 2015, 26, 2233-2242, both incorporated by reference, that antibodies can be site-specifically conjugated based on enzymatic remodeling of the native antibody glycan at N297 (trimming by endoglycosidase and introduction of azido-modified GalNAc derivative under the action of a glycosyltransferase) followed by attachment of a cytotoxic payload using click chemistry. It was demonstrated by and Verkade et al., Antibodies 2018, 7, 12, that the introduction of an acylated sulfamide further improves the glycan remodeling technology in terms of therapeutic index and the DAR of the resulting antibody-drug conjugates could be tailored towards DAR2 or DAR4 by choice of specific linker. It was also demonstrated that glycan trimming before conjugation leads to nihilation of binding of the resulting antibody-drug conjugates (ADCs) to Fc-gamma receptors (Fc-silencing). ADCs prepared by this technology were found to display a significantly expanded therapeutic index versus a range of other conjugation technologies and the technology of glycan-remodeling conjugation currently clinically applied in for example ADCT-601 (ADC Therapeutics).

A similar enzymatic approach to convert an antibody into an azido-modified antibody with concomitant Fc-silencing, reported by Lhospice etal., Mol. Pharmaceut. 2015, 12, 1863-1871, incorporated by reference, employs the bacterial enzyme transglutaminase (BTG or TGase). It was shown that deglycosylation of the native glycosylation site N297 with PNGase F liberates the neighbouring N295 to become a substrate for TGase-mediated introduction, which converts the deglycosylated antibody into a bis-azido antibody upon subjection to an azide-bearing molecule in the presence of TGase. Subsequently, the bis-azido antibody was reacted with DBCO-modified cytotoxins to produce ADCs with DAR2. A genetic method based on C-terminal TGase-mediated azide introduction followed by conversion in ADC with metal-free click chemistry was reported by Cheng etal., Mol. Cancer Therap. 2018, 17, 2665-2675, incorporated by reference.

Besides the attachment of small molecules, it has also been amply demonstrated that various click chemistries are suitable for the generation of protein-protein conjugates. For example, Witte et al., Proc. Nat. Acad. Sci. 2012, 109, 11993-11998, incorporated by reference, have shown the unnatural N-to-N and C-to-C protein dimers can be obtained by a combination of sortase-mediated introduction of two complementary click probes (azide and DBCO) into two different proteins, followed by seamless ligation based on metal-free click chemistry (strain-promoted azide-alkyne cycloaddition or SPAAC). Wagner et al., Proc. Nat. Acad. Sci. 2014, 111, 16820-16825, incorporated by reference, have applied this approach to prepare a bispecific antibody based on C-terminal sortagging with an anti-influenza scFv, which was further extended to metal-free click chemistry based on inverse eletron-demand Diels-Alder cycloaddition with tetrazines by Bartels et al., Methods 2019, 154, 93-101, incorporated by reference. Tetrazine ligation had been applied earlier also by for example Devaraj et al., Angew. Chem. Int. Ed. 2009, 48, 7013-7016 and Robillard et al., Angew. Chem. Ed. Engl. 2010, 49, 3375-3378, both incorporated by reference, for antibody modification by first (random) chemical installment of a trans-cyclooctene (TCO) onto an antibody. In contrast, site-specific introduction of TCO (or tetrazine or cyclopropene other click moieties for tetrazine ligation) onto antibodies can be achieved by a multitude of methods based on prior genetic modification of the antibody as described above and for example reported by Lang et al., J. Am. Chem. Soc. 2012, 134, 10317-10320, Seitchik et al., J. Am. Chem. Soc. 2012, 134, 2898-2901 and Oller-Salvia, Angew. Chem. Int. Ed. 2018, 57, 2831-2834, all incorporated by reference.

Sortase is a suitable enzyme for site-specific modification of proteins after prior introduction of a sortase recognition sequence, as first reported by Popp et al., Nat. Chem. Biol. 2007, 3, 707-708). Many other enzyme-enzyme recognition sequence combinations are also known for site-specific protein modification, as for example summarized by Milczek, Chem. Rev. 2018, 118, 119-141, incorporated by reference, and specifically applied to antibodies as summarized by Falck and Müller, Antibodies 2018, 7, 4 (doi:10.3390/antib7010004) and van Berkel and van Delft, Drug Discov. Today: Technol. 2018, 30, 3-10, both incorporated by reference. Besides, a wide array of methods is available for non-genetic modification of native proteins, as summarized by Koniev and Wagner, Chem. Soc. Rev. 2015, 44, 5495-5551, incorporated by reference and for N-terminal modification by Rosen and Francis, Nat. Chem. Biol. 2017, 13, 697-705 and Chen et al., Chem. Sci. 2017, 8, 27172722, both incorporated by reference. Any of the above approaches could be employed to install a proper click probe into a polypeptide/protein, as for example summarized by Chen et al., Acc. Chem. Res. 2011, 44, 762-773 and Jung and Kwon, Polymer Chem. 2016, 7, 4585-4598, both incorporated by reference, and applied to an immune cell engager or a cytokine. Upon installation of the complementary click probe into the antibody targeting the tumor-associated antigen, an immune cell engager can be readily generated while the stoichiometry of tumor-binding antibody to immune cell binder can be tailored by proper choice of technology.

Chemical approaches have also been developed for site-specific modification of antibodies without prior genetic modification, as for example highlighted by Yamada and Ito, ChemBioChem. 2019, 20, 2729-2737.

Chemical conjugation by affinity peptide (CCAP) for site-specific modification has been developed by Kishimoto et al., Bioconj. Chem. 2019**,** by using a peptide that binds with high affinity to human IgG-Fc, thereby enabling selective modification of a single lysine in the Fc-fragment with a biotin moiety or a cytotoxic payload. Similarly, Yamada et al., Angew. Chem. Int. Ed. 2019, 58, 5592-5597 and Matsuda et al., ACS Omega 2019, 4, 20564-20570, both incorporated by reference, have demonstrated that a similar approach (AJICAP^{™} technology) can be applied for the site-specific introduction of thiol groups on a single lysine in the antibody heavy chain. CCAP or AJICAP^{™} technology may also be employed for the site-specific introduction of azide groups or other functionalities.

Chemical conjugation of immune cell engagers to antibodies has been applied but normally leads to heterogenous mixtures. The preparation of homogenous bispecific antibodies or antibody-cytokine fusions that do not require prior reengineering of the full-length IgG and/or enables tailoring of the number of immune cell-engaging polypeptides as well as the spacer length and structure between IgG and polypeptide has been reported in WO2021144315.

Fusion proteins comprising a cleavable linker have been described in for example WO2021198490, WO2021188835 and WO2019/173832. The cleavable linker may connect a masking agent with the rest of the fusion protein as described in WO2021188835 or WO2019/173832. To date, no methods have been reported for the preparation of a homogenous antibody-cytokine conjugate that is connected via a chemical linker, wherein the cytokine is released upon cleavage by a protease.

### Summary of the invention

The present invention concerns an antibody-cytokine conjugate in which a cytokine is connected to an antibody via a chemical linker, the antibody-cytokine conjugate enables for targeted delivery of the cytokine by release of the cytokine at the target. The antibody-cytokine conjugate can be produced without requiring genetic modification of the IgG. The current invention enables tailoring of the molecular format of the antibody-cytokine conjugate to be defined in 2:1 or 2:2 ratio, i.e. the ratio of complement-dependent regions in full IgG CDR (2) versus immune cell-engaging polypeptide (1 or 2).

The inventors have surprisingly found that the conjugates according to the present invention have a higher efficacy and therapeutic window than a genetic fusion of an antibody and an immune cell engager.

Without being bound to a theory, the inventors believe that the immune cell-engaging polypeptide of a fusion protein is glycosylated which results in a heterogeneous product having variable efficacy, the average efficacy of said heterogenous immune-cell engaging polypeptide is believed to be lower than that of a homogeneous aglycosylated product.

Thus, a first aspect of the present invention relates to a cleavable antibody-cytokine conjugate. In a second aspect, the present invention relates to the cleavable antibody immunocytokine conjugate for medical treatment. In a third aspect, the present invention relates to a process for preparing the antibody-cytokine conjugate.

### Detailed description

### Definitions

The verb "to comprise", and its conjugations, as used in this description and in the claims is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there is one and only one of the elements. The indefinite article "a" or "an" thus usually means "at least one".

The compounds disclosed in this description and in the claims may comprise one or more asymmetric centres, and different diastereomers and/or enantiomers may exist of the compounds. The description of any compound in this description and in the claims is meant to include all diastereomers, and mixtures thereof, unless stated otherwise. In addition, the description of any compound in this description and in the claims is meant to include both the individual enantiomers, as well as any mixture, racemic or otherwise, of the enantiomers, unless stated otherwise. When the structure of a compound is depicted as a specific enantiomer, it is to be understood that the invention of the present application is not limited to that specific enantiomer.

The compounds may occur in different tautomeric forms. The compounds according to the invention are meant to include all tautomeric forms, unless stated otherwise. When the structure of a compound is depicted as a specific tautomer, it is to be understood that the invention of the present application is not limited to that specific tautomer.

The compounds disclosed in this description and in the claims may further exist as exo and *endo* diastereoisomers. Unless stated otherwise, the description of any compound in the description and in the claims is meant to include both the individual exo and the individual *endo* diastereoisomers of a compound, as well as mixtures thereof. When the structure of a compound is depicted as a specific *endo* or exo diastereomer, it is to be understood that the invention of the present application is not limited to that specific *endo* or exo diastereomer.

Furthermore, the compounds disclosed in this description and in the claims may exist as *cis* and *trans* isomers. Unless stated otherwise, the description of any compound in the description and in the claims is meant to include both the individual *cis* and the individual *trans* isomer of a compound, as well as mixtures thereof. As an example, when the structure of a compound is depicted as a *cis* isomer, it is to be understood that the corresponding *trans* isomer or mixtures of the *cis* and *trans* isomer are not excluded from the invention of the present application. When the structure of a compound is depicted as a specific *cis* or *trans* isomer, it is to be understood that the invention of the present application is not limited to that specific *cis* or *trans* isomer.

The compounds according to the invention may exist in salt form, which are also covered by the present invention. The salt is typically a pharmaceutically acceptable salt, containing a pharmaceutically acceptable anion. The term "salt thereof' means a compound formed when an acidic proton, typically a proton of an acid, is replaced by a cation, such as a metal cation or an organic cation and the like. Where applicable, the salt is a pharmaceutically acceptable salt, although this is not required for salts that are not intended for administration to a patient. For example, in a salt of a compound the compound may be protonated by an inorganic or organic acid to form a cation, with the conjugate base of the inorganic or organic acid as the anionic component of the salt.

The term "pharmaceutically accepted" salt means a salt that is acceptable for administration to a patient, such as a mammal (salts with counter ions having acceptable mammalian safety for a given dosage regime). Such salts may be derived from pharmaceutically acceptable inorganic or organic bases and from pharmaceutically acceptable inorganic or organic acids. "Pharmaceutically acceptable salt" refers to pharmaceutically acceptable salts of a compound, which salts are derived from a variety of organic and inorganic counter ions known in the art and include, for example, sodium, potassium, calcium, magnesium, ammonium, tetraalkylammonium, etc., and when the molecule contains a basic functionality, salts of organic or inorganic acids, such as hydrochloride, hydrobromide, formate, tartrate, besylate, mesylate, acetate, maleate, oxalate, etc.

The term "protein" is herein used in its normal scientific meaning. Herein, polypeptides comprising about 10 or more amino acids are considered proteins. A protein may comprise natural, but also unnatural amino acids.

The term "monosaccharide" is herein used in its normal scientific meaning and refers to an oxygen-containing heterocycle resulting from intramolecular hemiacetal formation upon cyclisation of a chain of 5-9 (hydroxylated) carbon atoms, most commonly containing five carbon atoms (pentoses), six carbon atoms (hexose) or nine carbon atoms (sialic acid). Typical monosaccharides are ribose (Rib), xylose (Xyl), arabinose (Ara), glucose (Glu), galactose (Gal), mannose (Man), glucuronic acid (GlcA), N-acetylglucosamine (GIcNAc), N-acetylgalactosamine (GaINAc) and N-acetylneuraminic acid (NeuAc).

The term "cytokine" is herein used in its normal scientific meaning and are small molecule proteins (5-20 kDa) that modulate the activity of immune cells by binding to their cognate receptors and by triggering subsequent cell signalling. Cytokines include chemokines, interferons (IFN), interleukins, monokines, lymphokines, colony-stimulating factors (CSF) and tumour necrosis factors (TNF). Examples of cytokines are IL-1 alpha (IL1a), IL-1 beta (IL1b), IL-2 (IL2), IL-4 (IL4), IL-5 (IL5), IL-6 (IL6) , IL8 (IL-8), IL-10 (IL10), IL-12 (IL12), IL-15 (IL15), IFN-alpha (IFNA), IFN-gamma (IFN-G), and TNF-alpha (TNFA).

The term "antibody" is herein used in its normal scientific meaning. An antibody is a protein generated by the immune system that is capable of recognizing and binding to a specific antigen. An antibody is an example of a glycoprotein. The term antibody herein is used in its broadest sense and specifically includes monoclonal antibodies, polyclonal antibodies, dimers, multimers, multi-specific antibodies (e.g. bispecific antibodies), antibody fragments, and double and single chain antibodies. The term "antibody" is herein also meant to include human antibodies, humanized antibodies, chimeric antibodies and antibodies specifically binding cancer antigen. The term "antibody" is meant to include whole immunoglobulins, but also antigen-binding fragments of an antibody. Furthermore, the term includes genetically engineered antibodies and derivatives of an antibody. Antibodies, fragments of antibodies and genetically engineered antibodies may be obtained by methods that are known in the art. Typical examples of antibodies include, amongst others, abciximab, rituximab, basiliximab, palivizumab, infliximab, trastuzumab, efalizumab, alemtuzumab, adalimumab, tositumomab-1131, cetuximab, ibrituximab tiuxetan, omalizumab, bevacizumab, natalizumab, ranibizumab, panitumumab, eculizumab, certolizumab pegol, golimumab, canakinumab, catumaxomab, ustekinumab, tocilizumab, ofatumumab, denosumab, belimumab, ipilimumab and brentuximab.

An "antibody fragment" is herein defined as a portion of an intact antibody, comprising the antigen-binding or variable region thereof. Examples of antibody fragments include Fab, Fab', F(ab')₂, and Fv fragments, diabodies, minibodies, triabodies, tetrabodies, linear antibodies, single-chain antibody molecules, scFv, scFv-Fc, multispecific antibody fragments formed from antibody fragment(s), a fragment(s) produced by a Fab expression library, or an epitope-binding fragments of any of the above which immunospecifically bind to a target antigen (e.g., a cancer cell antigen, a viral antigen or a microbial antigen.

An "antigen" is herein defined as an entity to which an antibody specifically binds.

The term "substantial" or "substantially" is herein defined as a majority, i.e. >50% of a population, of a mixture or a sample, preferably more than 50%, 55%, 60%, 65%, 70%, 75%, 80%, 85%, 90%, 91%, 92%, 93%, 94%, 95%, 96%, 97%, 98%, or 99% of a population.

A "linker" is herein defined as a moiety that connects two or more elements of a compound. For example in an antibody-conjugate, an antibody and a payload are covalently connected to each other via a linker. A linker may comprise one or more linkers and spacer-moieties that connect various moieties within the linker.

A "polar linker" is herein defined as a linker that contains structural elements with the specific aim to increase polarity of the linker, thereby improving aqueous solubility. A polar linker may for example comprise one or more units, or combinations thereof, selected from ethylene glycol, a carboxylic acid moiety, a sulfonate moiety, a sulfone moiety, an acylated sulfamide moiety, a phosphate moiety, a phosphinate moiety, an amino group or an ammonium group.

A "spacer" or spacer-moiety is herein defined as a moiety that spaces (i.e. provides distance between) and covalently links together two (or more) parts of a linker. The linker may be part of e.g. a linker-construct, the linker-conjugate or a bioconjugate, as defined below.

The term "click probe" refers to a functional moiety that is capable of undergoing a click reaction, i.e. two compatible click probes mutually undergo a click reaction such that they are covalently linked in the product. Compatible probes for click reactions are known in the art, and preferably include (cyclic) alkynes and azides. In the context of the present invention, click probe Q in the compound according to the invention is capable of reacting with click probe F on the (modified) protein, such that upon the occurrence of a click reaction, a conjugate is formed wherein the protein is conjugated to the compound according to the invention. Herein, F and Q are compatible click probes.

The term "(hetero)alkyl" refers to alkyl groups and heteroalkyl groups. Heteroalkyl groups are alkyl groups wherein one or more carbon units in the alkyl chain (e.g. CH₂, CH or C) are replaced by heteroatoms, such as O, S, S(O), S(O)₂ or NR⁴. In other words, the alkyl chain is interrupted with one ore more elements selected from O, S, S(O), S(O)₂ and NR⁴. Such interruptions are distinct from substituents, as they occur within the chain of an alkyl group, whereas substituents are pendant groups, monovalently attached to e.g. a carbon atom of an alkyl chain. In a preferred embodiment, the (hetero)alkyl group is an alkyl group, e.g. ethyl (Et), isopropyl (i-Pr), n-propyl (n-Pr), tert-butyl (t-Bu), isobutyl (i-Bu), n-butyl (n-Bu) or n-pentyl.

Likewise, the term "(hetero)aryl" refers to aryl groups and heteroaryl groups. Heteroaryl groups are aryl groups wherein one or more carbon units in the ring (e.g. CH) are replaced by heteroatoms, such as O, S, N or NR⁴.

An "acylsulfamide moiety" is herein defined as a sulfamide moiety (H₂NSO₂NH₂) that is N-acylated or N-carbamoylated on one end of the molecule and N-alkylated (mono or bis) at the other end of the molecule. In the context of the present invention, especially in the examples, this group is also referred to as "HS".

A "domain" may be any region of a protein, generally defined on the basis of sequence homologies and often related to a specific structural or functional entity. CEACAM family members are known to be composed of Ig-like domains. The term domain is used in this document to designate either individual Ig-like domains, such as "N-domain" or for groups of consecutive domains, such as "A3-B3 domain".

A "coding sequence" or a sequence "encoding" an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

The term "gene" means a DNA sequence that codes for, or corresponds to, a particular sequence of amino acids which comprises all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

A "biomolecule" is herein defined as any molecule that can be isolated from nature or any molecule composed of smaller molecular building blocks that are the constituents of macromolecular structures derived from nature, in particular nucleic acids, proteins, glycans and lipids. Examples of a biomolecule include an enzyme, a (non-catalytic) protein, a polypeptide, a peptide, an amino acid, an oligonucleotide, a monosaccharide, an oligosaccharide, a polysaccharide, a glycan, a lipid and a hormone.

The term "payload" refers to the moiety that is covalently attached to a targeting moiety such as an antibody. Payload thus refers to the monovalent moiety having one open end which is covalently attached to the targeting moiety via a linker. A payload may be small molecule or a biomolecule. In the present invention, the payload is an immune cell engager polymer.

The term "complement-dependent region" or "CDR" refers to the variable fragment of an antibody that is able to bind a specific receptor or antigen.

### The invention

The present invention provides an antibody-cytokine conjugate with prolonged in vivo half-life and increased in vitro and in vivo activity, and capable of activating an immune response in the tumour microenvironment, thereby conferring protective anti-tumour immunity.

### Conjugates of general structure (1) or structure (2)

The invention concerns antibody-cytokine conjugates of general structure (1) or (2):
An antibody-cytokine conjugate having structure (1) or structure (2) 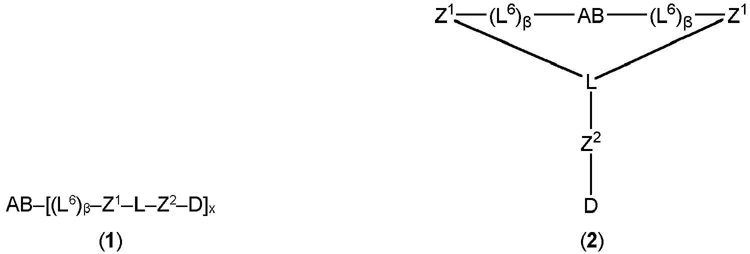 wherein:
   - AB is an antibody;
   - L is a linker that connects AB to D;
   - x is an integer in the range of 1-10;
   - Z¹ and Z² are connecting groups, wherein at least Z¹ comprises the product of a cycloaddition or a nucleophilic reaction;
   - β is 0 or 1;
   - L⁶ is -(H)ᵥ-S-(L⁷)_{w'}- wherein:
      ∘ H is a monosaccharide,
      ∘ v is an integer in the range of 1 - 10,
      ∘ S is a sugar or a sugar derivative,
      ∘ w' is 0 or 1, and
      ∘ L⁷ is -N(H)C(O)-(CH₂-O)_{z}(CH₂CH₂-O)γ-CH₂- or CH₂-(O-CH₂-CH₂)_{γ}-,wherein γ is an integer in the range of 0-100 and z is 0 or 1.
   - D is a polypeptide according to -(D¹)_{α}-D², wherein:
      ∘ D¹ is a cleavable peptide linker;
      ∘ D² is an immune cell engager polypeptide specific for an immune cell receptor;
      ∘ α is 0 or 1;
   - wherein L is a cleavable linker if α = 0.

Preferably, L⁶ is present and β is 1.

### The antibody AB

AB is an antibody. Antibodies are known in the art and include IgA, IgD, IgE, IgG, IgM, Fab, VHH, scFv, diabody, minibody, affibody, affylin, affimers, atrimers, fynomer, Cys-knot, DARPin, adnectin/centryin, knottin, anticalin, FN3, Kunitz domain, OBody, bicyclic peptides and tricyclic peptides. Preferably, the antibody is a monoclonal antibody, more preferably selected from the group consisting of IgA, IgD, IgE, IgG and IgM antibodies. Even more preferably AB is an IgG antibody. The IgG antibody may be of any IgG isotype. The antibody may be any IgG isotype, e.g. IgG1, IgG2, IgI3 or IgG4. Preferably AB is a full-length antibody, but AB may also be a Fc fragment.

The antibody Ab is typically specific for an extracellular receptor on a tumour cell, preferably wherein the extracellular receptor on the tumour cell is selected from the group consisting of 5T4 (TPBG), ADAM9, ALPP, ALPPL2, AMHRII, ASCT2 (SLC1A5), ASLG659, ASPHD1, av-integrin, avb3-integrin/ITGAV/CD51, Axl, B7-H3 (CD276), B7-H4 (VTCN1), BAFF-R, BCMA (CD269), BMPR1B, Brevican, c-KIT (CD117), c-Met, C4.4a (LYPD3), CA-IX (CA9)/MN, Cadherin-6, CanAg, CCR7, CD117 (c-KIT), CD123 (IL-3Rα), CD13, CD133, CD138/syndecan-1, CD166 (ALCAM), CD19, CD20, CD203C, CD205, Ly75, CD21, CD22, CD228 (P79, SEMF), CD25 (IL-2R-α), CD30 (TNFRSF8), CD324 (CDH1/E-cadherin), CD33, CD352 (SLAMF6, NTB-A), CD37, CD38, CD44v6, CD45, CD46, CD47, CD48a (SLAMF2), CD56 (NCAM), CD70, CD71 (TF-R), CD72, CD74, CD79a, CD79b, CDH6, Cadherin-6, CEACAM5 (CD66e), claudin, CLDN18.2, CLDN6 (claudin-6, Skullin), CLEC12A, CLL-1/CLEC12A, Cripto, CS1 (SLAMF7, CD319), CXCR5, DKL-1, DLL3 (delta-like 3), DPEP3, E16, EGFR, EGFRvlll, ENPP3, CD203c (AGS-16), EpCAM, EphA2, EphB2R, Ephrin-A4 (EFNA4), ETBR, FAP, FGFR2, FGFR3, fibronectin EDB , FLT3, FOLR1 (FR-a), Gal-3BP, GD3, GDNF-Ra1, GEDA, GFRA1, Globo H, GPC3 (glypican-3), gpNMB, GPR172A, GPR19, GPR54, GRP20, guanyl cyclase C (GCC), HER2, HER3, HLA-DOB, IGF-1R, IL13R, IL20Ra, Integrin avb6, KAAG-1, LAMP-1 (CD107a), Lewis Y (CD174), LGR5, LIV-1 (SLC39A6, ZIP6), LRRC15, LY64, Ly6E (lymph. antig 6), Ly6G6D, LY6K, mesothelin (MSLN), MFI2 (TAA), MICA/B, MOSPD2, MPF, MUC1 (CA6), MUC16/CA-125 , MUC1c, NaPi2b (SLC34A2), NCA, Nectin-4 (PVRL4), Notch3, P-cadherin (pCAD, CDH3), P2X5, PD-L1 (CD274, B7-H1), PMEL17, PRLR (prolactin), PSCA, PSMA, PTK7 (CCK4), RET, RNF43, RON, ROR1, ROR2, Serna 5b, SEZ6, SLITRK6 (SLC44A4), STEAP1, STEAP2, STn, TAG72, TENB2, TF (CD142, thromoplastin), TIM-1, TM4SF, TMEFF, TMEM118, TMEM46, TNF-alpha, TROP-2 (TACSTD2), TWEAKR, receptor tyrosine kinases (RTK), tenascin, or antibody is specific for an extracellular protein resulting from a viral infection and/or for a tumour-associated carbohydrate antigen (TACA).

The antibody may also be specific to an extracellular protein resulting from a viral infection, e.g. human polio virus (HPV), human cytomegalovirus (HCMV) or human papillomavirus (HPV). The antibody may also be specific for a tumour-associated carbohydrate antigen (TACA) that is selected from the group of Tn, STn, T-antigen, LDN, Lewis^{c} (Le^{c}), Sialyl-Lewis^{c} (SLe^{c}), 6-Sialyl-Lewis^{c} (6SLe^{c}), LN, alpha-Gal, 3SLN, 6SLN, H-antigen, A-antigen, B-antigen, Lewis^{a} (Le^{a}), Sialyl-Lewis^{a} (SLe^{a}), 6-Sialyl-Lewis^{a} (6SLe^{a}), Lewis^{b} (Le^{b}), Sialyl-Lewis^{b} (SLe^{b}), 6-Sialyl-Lewis^{b} (6SLe^{b}), Lewis^{x} (Le^{x}), Sialyl-Lewis^{x} (SLe^{x}), 6-Sialyl-Lewis^{x} (6SLe^{x}), Lewis^{y} (Le^{y}), Sialyl-Lewis^{y} (SLe^{y}), 6-Sialyl-Lewisy (6SLe^{y}) and or combinations thereof. The antibody may also be specific to both an extracellular protein and a TACA at the same time.

The number of payloads D attached to a single antibody is known in the art as the DAR (drug-to-antibody ratio). In the present invention the drug is an immune cell engager polypeptide specific for an immune cell receptor. In the present invention the DAR values are an integer in the range of 1-10, preferably DAR values are 1, 2 or 4, more preferably the DAR values are 1 or 2.

Part of the antibody may be a linker L⁶ that connects the reactive moiety F¹ or connecting group Z¹ to the peptide part of the cell-binding agent. Preferably, the connecting group Z¹ is connected to the antibody via a glycan.

### Linker L⁶

Linker L⁶ is preferably present, wherein reactive group F¹ may be introduced at a specific position of the antibody. This is for example the case for conjugation via an artificially introduced reactive group F¹, such as for example using transglutaminase, using sortase or by enzymatic glycan modification (e.g. glycosyltransferase or a-1 ,3-mannosyl-glycoprotein-2-b-N-acetylglucosaminyl-transferase). For example, a modified sugar residue S(F¹)₂ may be introduced at the glycan, extending the glycan with one monosaccharide residue S, which introduces two reactive groups F¹ on the glycan of an antibody. In a most preferred embodiment, conjugation occurs via the glycan of the antibody. The site of conjugation is preferably at the heavy chain of the antibody.

All recombinant antibodies, generated in mammalian host systems, contain the conserved N-glycosylation site at the asparagine residue at or close to position 297 of the heavy chain (Kabat numbering), which is modified by a glycan of the complex type. This naturally occurring glycosylation site of antibodies is preferably used, but other glycosylation sites, including artificially introduced ones, may also be used for the connection of linker L⁶. Thus, in a preferred embodiment, L⁶ is connected to an amino acid of the antibody which is located at a position in the range of 250 - 350 of the heavy chain, preferably in the range of 280 - 310 of the heavy chain, more preferably in the range of 295 - 300 of the heavy chain, most preferably at position 297 of the heavy chain. Using this conserved glycosylation position of the antibody, the obtained conjugates are formed as symmetrical dimers, wherein each half antibody contains one F¹. Some antibodies may have a second glycosylation site per half antibody, which is preferably not used as conjugation site. The skilled person is able to perform the enzymatic conversions in such a way that only the main glycosylation site is utilized for conjugation. Alternatively, the skilled person is able to perform the enzymatic conversion in such a way that also the second glycosylation site is utilized for conjugation, thereby doubling the DAR of the antibody-drug conjugate.

L⁶ is a linker that connects AB to F¹ or Z¹, and is represented by -(H)ᵥ-S-(L⁷)_{w'}-, wherein H is a monosaccharide, v is an integer in the range of 0 - 10, S is a sugar or a sugar derivative, w' is 0 or 1 and L⁷ is -N(H)C(O)CH₂-, -N(H)C(O)CF₂- or -CH₂-. (H)ᵥ may be linear or branched, S may be connected to any monosaccharide of (H)ᵥ. Typically, L⁶ is at least partly formed by the glycan of an antibody.

The -(H)ᵥ₋ part of L⁶ is the glycan, or part thereof. The -(H), of the glycan thus typically originates from the original antibody. (H)ᵥ is preferably selected from structures (H1), (H2) and (H3).

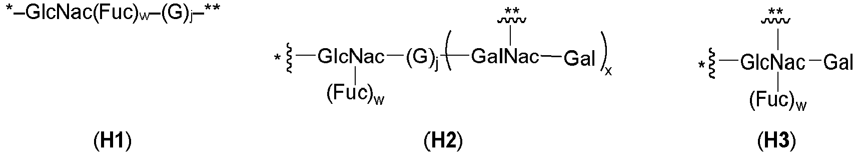

Herein, w is 0 or 1, j is an integer in the range of 0 - 9, GIcNAc is an N-acetylglucosamine moiety, Fuc is a fucose moiety, Gal is a galactose moiety, the wavy bond to * represent the connection to the peptide of the antibody and the wavy bond ** represents the connection to S. Fuc is typically bound to GIcNAc via an α-1 ,6-glycosidic bond. Normally, antibodies may (w = 1) or may not be fucosylated (w = 0). In the context of the present invention, the presence of a fucosyl moiety is irrelevant, and similar effects are obtained with fucosylated (w = 1) and non-fucosylated (w = 0) antibody conjugates. The GIcNAc residue may also be referred to as the core-GIcNAc residue and is the monosaccharide that is directly attached to the peptide part of the antibody.

S may be directly connected to the core-GlcNAc(Fuc)_{w} moiety, i.e. j = 0, meaning that the remainder of the glycan is removed from the core-GlcNAc(Fuc)_{w} moiety before S is attached. Such trimming of glycans is well-known in the art and can be achieved by the action of an endoglycosidase. (H3) may be obtained by from a trimmed glycan by introducing a galactose with a galactosyltransferase and introducing S fucose with a fucosyltransferase as described in WO2022037665. Alternatively, there are one or more monosaccharide residues present in between the core-GlcNAc(Fuc)_{w} moiety and S, i.e. j is an integer in the range of 1 - 9 , preferably j = 1 - 5. In one preferred embodiment, (G)ⱼ is an oligosaccharide fraction comprising j monosaccharide residues G, wherein j is an integer in the range of 2 - 5. In another preferred embodiment, (G)ⱼ is a monosaccharide fraction comprising j monosaccharide residues G, wherein j is 1. (G)ⱼ is connected to the GIcNAc moiety of GlcNAc(Fuc)_{w}, typically via a α-1,4 bond. In a preferred embodiment, j is 0, 1, 3, 4 or 5, more preferably, j is 0 or 1, most preferably j is 0.

Although any monosaccharide that may be present in a glycan may be employed as G, each G is preferably individually selected from the group consisting of galactose, glucose, N-acetylgalactosamine, *N-*acetylglucosamine, mannose and *N*-acetylneuraminic acid. More preferred options for G are galactose, *N-*acetylglucosamine, mannose. In case j = 1, it is preferred that G = galactose and S = *N*-acetylneuraminic acid.

When j is 3 - 10, (G)ⱼ may be linear or branched. Preferred examples of branched oligosaccharides (G)ⱼ are (**a**), (**b**), (**c**), (**d**), (**e**), (**f**) and (h) as shown below.

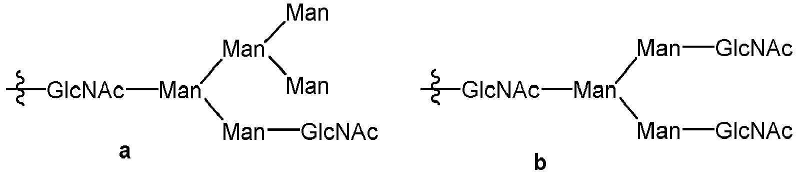

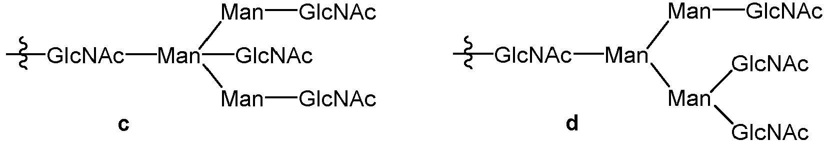

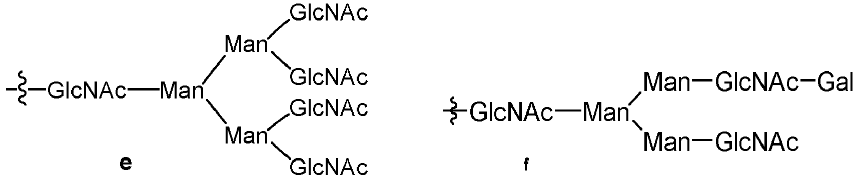

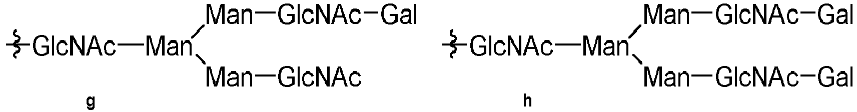

Herein, the wavy lines represent the connection to the core GlcNac(Fuc)_{w}. In case (G)ⱼ is present with j > 2, it is preferred that it ends in GIcNAc or Gal, preferably GlcNAc. In other words, the monosaccharide residue directly connected to S is preferably GIcNAc or Gal. The presence of a GIcNAc moiety facilitates the synthesis of the functionalized antibody, as monosaccharide derivative S can readily be introduced by glycosyltransfer onto a terminal GlcNAc residue. The presence of a Gal moiety facilitates the synthesis of the functionalized antibody, as monosaccharide derivative S sialic acid can readily be introduced by sialyltransferase onto a terminal Gal residue. Alternatively, the presence of a Gal moiety enables the introduction of monosaccharide derivative S fucose by fucosyltransferase onto the adjacent GIcNac as described in WO2022037665. In the above preferred embodiments for (G)ⱼ, comprising structure (**a**) - (**h**), moiety S may be connected to any of the terminal GlcNAc residues, i.e. not the one with the wavy bond, which is connected to the core GlcNAc residue on the antibody.

Antibodies and antibody conjugates having j = 0 or 1 show no or significantly reduced binding to Fc-gamma receptors, while antibodies and antibody conjugates having j in the range of 4 - 10 do bind to Fc-gamma receptors. Thus, by selecting a certain value for j, the desired extent of binding to Fc-gamma receptors can be obtained. It is thus preferred that j = 0, 1, 4, 5, 6, 7, 8, 9 or 10, more preferably j = 0, 1, 4 or 5, most preferably the antibody is trimmed and j = 0.

S is a sugar or sugar derivative. The term "sugar derivative" is herein used to indicate a derivative of a monosaccharide sugar, i.e. a monosaccharide sugar comprising substituents and/or functional groups. Suitable examples for S include glucose (Glc), galactose (Gal), mannose (Man), fucose (Fuc), amino sugars and sugar acids, e.g. glucosamine (GlcNH₂), galactosamine (GalNH₂) N-acetylglucosamine (GlcNAc), N-acetylgalactosamine (GalNAc), sialic acid (Sia) which is also referred to as N-acetylneuraminic acid (NeuNAc), and N-acetylmuramic acid (MurNAc), glucuronic acid (GlcA) and iduronic acid (IdoA). Preferably, S is selected from Gal, GalNAc and NeuNAc. In an especially preferred embodiment, S is GaINAc.

Connecting group Z¹ or reactive group F¹ may be attached directly to S, or there may be a linker L⁷ present in between S and Z¹ or F¹. Thus, L⁷ may be present (w' = 1 or 2) or absent (w' = 0). Typically, each moiety Z may be connected to S via a linker L⁷, thus in one embodiment w' = 0 of x. Preferably, L⁷ is absent and each connecting moiety Z is directly attached to S. If present, L⁷ may be selected from -N(H)C(O)CH₂-, -N(H)C(O)CF₂- or -CH₂-. In a preferred embodiment, x = 1 and w' = 0 or 1, most preferably x = 1 and w' = 0.

### The immune cell engager polypeptide D

The payload of the conjugates of the invention is the immune cell engager polypeptide D, also referred to as a cytokine. Payload D is connected to the antibody via a cleavable linker. The cleavable unit may be present in linker L (i.e. in one embodiment L is cleavable) or within the peptide chain of D (i.e. α = 1 and cleavable peptide linker D¹ is present). Typically, if α = 1 then L is not cleavable.

The polypeptide D or the precursor to polypeptide D is preferably produced by microbial recombinant expression. It is especially preferred that recombinant expression occurs in a prokaryote, in a cell-free protein expression system, by a synthetic method or by a combination of a synthetic method and a protein ligation approach. Proteins obtained by microbial expression of prokaryotes or by cell-free methods or by synthetic methods are not glycosylated, hence when D or the precursor to D is obtained by microbial expression of a prokaryote, by cell-free expression of a synthetic method it is more homogeneous than when obtained from an eukaryote. Most preferably, D is obtained by microbial recombinant expression by an E. coli bacteria. Alternatively worded, it is preferred that the peptide chain of D is not glycosylated, i.e. the D is an unglycosylated polypeptide.

### Mode of action

D is a polypeptide according to according to -(D¹)_{α}-D², wherein D¹ is a cleavable peptide linker; D² is an immune cell engager polypeptide specific for an immune cell receptor, and α is 0 or 1. D¹ is present if L is not cleavable. Preferably, α is 1 and D¹ is present, more preferably D is connected to Z² via the N-terminus or C-terminus of D¹, most preferably the N-terminus.

D² is specific for an immune cell receptor and by activating a receptor it triggers the subsequent cell signalling. The ability of D² bind to at least one of its associated receptors is decreased or reduced completely by its connection to the antibody. Therefore, D² becomes activated by cleavage of D¹ or linker L. Since the cleavable site has an enhanced probability of being cleaved at a particular target, the cell signalling occurs primarily at target. Since, the antibody sterically hinders the ability of D² to at least one of the associated receptor it is preferred that that the distance between D² and AB is as short as possible. More preferably, the shortest chain of atoms between D² and AB is 10-100 atoms, more preferably the atoms are selected from B, C, O, N, S and P. In a preferred embodiment, the chain of atoms consists of 15 - 50 atoms selected from C, O, N, S and P, more preferably from C, O, N and S.

### Cleavable Linker D¹

D¹ is a cleavable peptide linker as known in the art. D¹ contains a cleavable site for a protease. In therapeutic applications, the protease cleavage site can be cleaved by a protease that is overexpressed near or at the target cells, for example cancer cells, infected cells or pathogens. The overexpressed proteases may be extracellular enzymes produced by the target cells, or intracellular enzymes that are leaked outside of the target cells. Preferably, the target is a cancer cell and the enzymes are overexpressed in the tumor microenvironment.

In a preferred embodiment, D¹ contains a cleavable site that is recognized by one of a serine protease, a cysteine protease, an aspartate protease, a threonine protease, a glutamic acid protease, a metalloproteinase, a gelatinase, and a asparagine peptide lyase.

In another preferred embodiment, D¹ contains a cleavable site that is recognized by one of a cathepsin B, a cathepsin C, a cathepsin D, a cathepsin E, a cathepsin G, a cathepsin K, a cathepsin L, a kallikrein, a hKI, a hKIO, a hKT5, a plasmin, a collagenase, a type IV collagenase, a stromelysin, a factor Xa, a chymotrypsin-like protease, a trypsin-like protease, a elastase-like protease, a subtilisin-like protease, an actinidain, a bromelain, a calpain, a caspase, a caspase-3, a mirl-CP, a papain, a HIV-I protease, a HSV protease, a CMV protease, a chymosin, a renin, a pepsin, a matriptase, a membrane type serine protease 1 (MT-SP1), a legumain, a plasmepsin, a nepenthesin, a metalloexopeptidase, a metalloendopeptidase, a matrix metalloprotease (MMP), a MMP1, a MMP2, a MMP3, a MMP7, a MMP8, a MMP9, a MMP1O, a MMP11, a MMP12, a MMP13, a MMP14, an ADAM10, an ADAM12, an urokinase plasminogen activator (uPA), an enterokinase, a prostate-specific target (PSA, hK3), an interleukin-1b converting enzyme, a thrombin, a FAP (FAP-α), a type II transmembrane serine protease (TTSP), a neutrophil elastase, a proteinase 3, a neutrophil serine protease 4, a mast cell chymase, a mast cell tryptase, a dipeptidyl peptidase and a dipeptidyl peptidase IV (DPPIV/CD26).

### Immune cell-engaging polypeptide D²

The immune cell-engaging polypeptide D² is specific for an immune cell receptor. It is understood that a reference to a cytokine includes mutated variants, cytokines with an attached cofactor. For example IL-15 refers to normal IL-15, but also to IL15α, sushi-IL15, IL15 with point mutations etc, unless specifically indicated otherwise. Preferably, the cytokine is without co-factor. In the case D² is a mutant immune cell-engaging polypeptide, preferably the mutations enhance or decrease binding affinity to a receptor. Preferably the immune cell-engaging polypeptide is selected from the group consisting of IL-1, IL-2, IL-3, IL-4, IL-5, IL-6, IL-7, IL-8, IL-9, IL-10, IL-11, IL-12, IL-13, IL-15, IL-16, IL-17, IL-18, IL-19, IL-20, IL-21, IL-22, IL-23,, IL-24, IL-26, IL-28, IL-29, IL-33, IL-36, IL37, IL-38, IFN-α (including IFN-α1/13, IFN-α2, IFN-α4, IFN-α5, IFN-α6, IFN-α7, IFN-α8, IFN-α10, IFN-α14, IFN-α16, IFN-α17, and IFN-α21), IFN- β, IFN-Y, IFN-A, TFN-α, TNF-β, TGF-β1, M-CSF, G-CSF, GM-CSF, and CXL10, wherein mutated variants are included more preferably the immune cell engaging polypeptide is IL-2 or IL-15, even more preferably IL-15.

Thus, in one embodiment D² is IL-15, preferably IL-15 is mutated to enhance or decrease the binding affinity to a receptor, more preferably IL-15 is mutated to decrease the binding affinity to IL15Ra. IL15Ra-binding results in a quick clearance, hence the mutant with decreased IL15Ra binding affinity results in a better PK and higher potency. More preferably, IL15 according to sequence SEQ ID NO: 3, comprises an amino acid substitution on E46 and V49, even more preferably, the amino acid substitution is E46G,V49R, most preferably there are no other substitutions. Advantageously, the binding affinity to IL15Rβ and γ_{c} is decreased by the steric hindrance of the antibody. However, after cleavage the binding sites to IL15Rβ and γ_{c} become available and the immune response is activated.

### Connecting group Z¹ and Z²

Z¹ and Z² are connecting groups, which covalently connect the antibody with the payloads of the conjugate according to the invention. The term "connecting group" herein refers to the structural element, resulting from a reaction, here between Q and F, connecting one part of the conjugate with another part of the same conjugate. Z¹ is formed by a cycloaddition or a nucleophilic substitution between Q¹ and F¹. Preferably, Z¹ is formed by a cycloaddition. Likewise, Z² is preferably formed by a cycloaddition or a nucleophilic substitution between Q² and F². Preferably, Z² is formed by a cycloaddition. Herein, Z refers to Z¹ and Z², Q refers to Q¹ and Q², and F refers to F¹ and F².

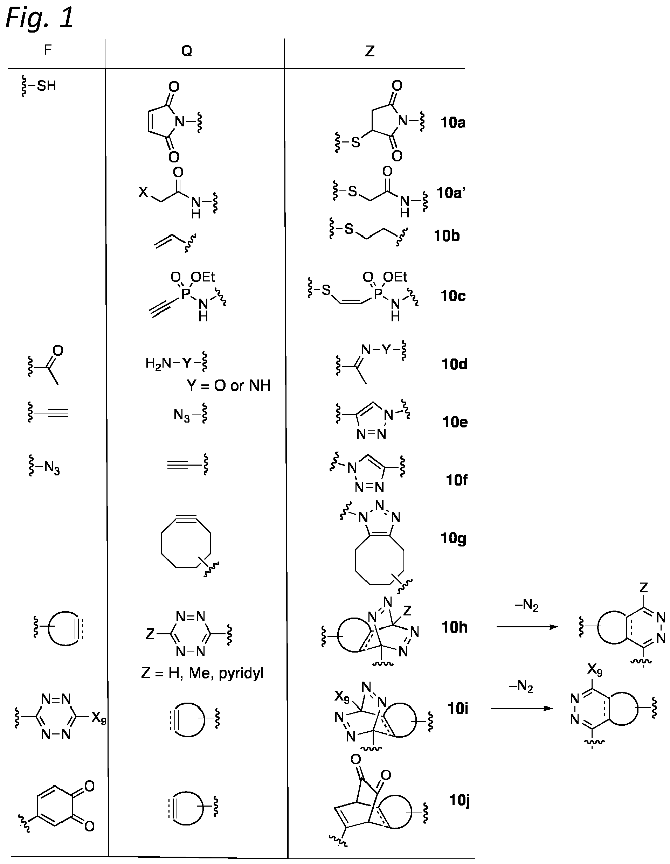

As will be understood by the person skilled in the art, the exact nature of the connecting group depends on the exact structure of the reactive moieties Q and F. The skilled person is aware of complementary click probes which are reactive towards each other and form suitable reaction partners Q¹/F¹ and Q²/F². For example, when F comprises or is an alkynyl group, complementary groups Q include azido groups. For example, when F comprises or is an azido group, complementary groups Q include alkynyl groups. For example, when F comprises or is a cyclopropenyl group, a trans-cyclooctene group, a cycloheptyne or a cyclooctyne group, complementary groups Q include tetrazinyl groups. In these particular cases, Z is only an intermediate structure and will expel N₂, thereby generating a dihydropyridazine (from the reaction with alkene) or pyridazine (from the reaction with alkyne) as shown in Figure 1.

In a preferred embodiment, connecting groups Z are obtained by a cycloaddition reaction, preferably wherein the cycloaddition is a [4+2] cycloaddition or a 1,3-dipolar cycloaddition. Conjugation reactions via cycloadditions are known to the skilled person, and the skilled person is capable of selecting appropriate reaction partners F and Q, and will understand the nature of the resulting connecting group Z. Preferred cycloadditions are a [4+2]-cycloaddition (e.g. a Diels-Alder reaction) or a [3+2]-cycloaddition (e.g. a 1,3-dipolar cycloaddition). Preferably, the cycloaddition is the Diels-Alder reaction or the 1,3-dipolar cycloaddition. The preferred Diels-Alder reaction is the inverse electron-demand Diels-Alder cycloaddition. In another preferred embodiment, the 1,3-dipolar cycloaddition is used, more preferably the alkyne-azide cycloaddition. Cycloadditions, such as Diels-Alder reactions and 1 ,3-dipolar cycloadditions are known in the art, and the skilled person knows how to perform them.

Preferably, Z contains a moiety selected from the group consisting of a triazole, a cyclohexene, a cyclohexadiene, a [2.2.2]-bicyclooctadiene, a [2.2.2]-bicyclooctene, an isoxazoline, an isoxazolidine, a pyrazoline, a piperazine, a thioether, an amide or an imide group. Triazole moieties are especially preferred to be present in Z. In one embodiment, Z comprises a (hetero)cycloalkene moiety, i.e. formed from Q comprising a (hetero)cycloalkyne moiety. In an alternative embodiment, Z comprises a (hetero)cycloalkane moiety, i.e. formed from Q comprising a (hetero)cycloalkene moiety. Herein, aromatic rings such as a triazole ring are considered a heterocycloalkane ring, since it is formed by reaction of an alkyne moiety and an azide moiety.

In a preferred embodiment, Z has the structure (Z1): 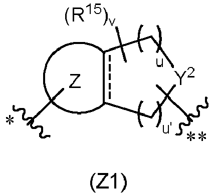 Herein, the bond depicted as --- is a single bond or a double bond. Furthermore:
- ring Z is obtained by a cycloaddition, preferably ring Z is selected from (Za) - (Zm) defined below, wherein the carbon atoms labelled with ** correspond to the two carbon atoms of the bond depicted as --- of (Z1) to which ring Z is fused;
- R¹⁵ is independently selected from the group consisting of hydrogen, halogen, -OR¹⁶, -NO₂, -CN, -S(O)₂R¹⁶, -S(O)₃⁽⁻⁾, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkylgroups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇- C₂₄ (hetero)arylalkyl groups;
- Y² is C(R³¹)₂, O, S, S⁽⁺⁾R³¹, S(O)R³¹, S(O)=NR³¹ or NR³¹, wherein S⁽⁺⁾ is a cationic sulphur atom counterbalanced by B⁽⁻⁾, wherein B⁽⁻⁾ is an anion, and wherein each R³¹ individually is R¹⁵ or a connection with D, connected via L;
- u is 0, 1, 2, 3, 4 or 5;
- u' is 0, 1, 2, 3, 4 or 5, wherein u + u' = 0, 1, 2, 3, 4, 5, 6, 7 or 8;
- v = an integer in the range 8-16;
Ring Z is formed by the cycloaddition, and is preferably selected from (Za) - (Zm)

In a preferred embodiment, u + u' = 0, 4, 5, 6, 7 or 8, more preferably 0, 4 or 5. In case the bond depicted as --- is a double bond, it is preferred that u + u' = 4, 5, 6, 7 or 8, more preferably u + u' = 4 or 5. In case the bond depicted as --- is a single bond, it is preferred that u + u' = 0 or 5. Preferably, the wavy bond labelled with * is connected to AB, optionally via L⁶, and the wavy bond labelled with ** is connected to L.

It is especially preferred that Z comprises a (hetero)cycloalkene moiety, i.e. the bond depicted as - - - is a double bond. In a preferred embodiment, Z is selected from the structures (Z2) - (Z20c), depicted here below:

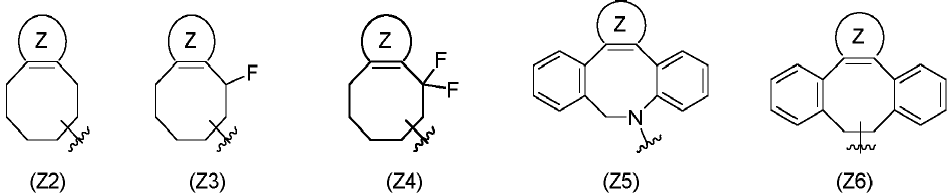

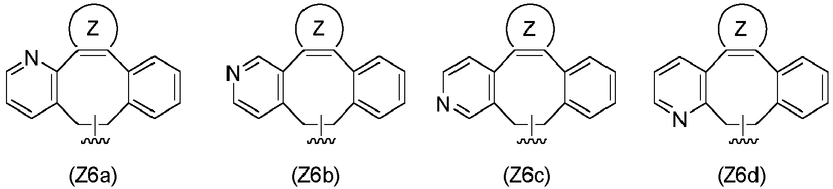

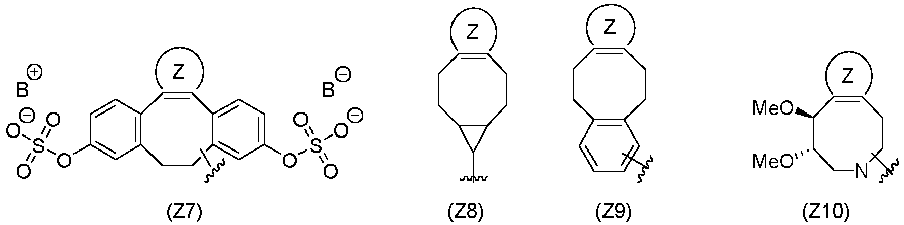

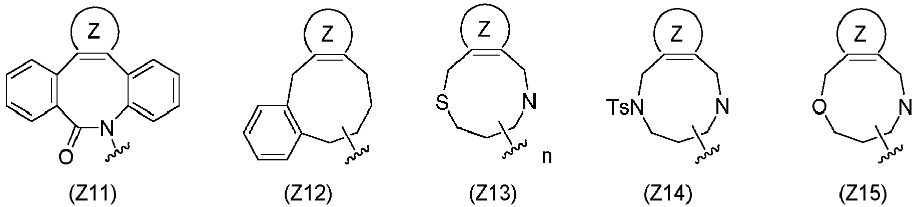

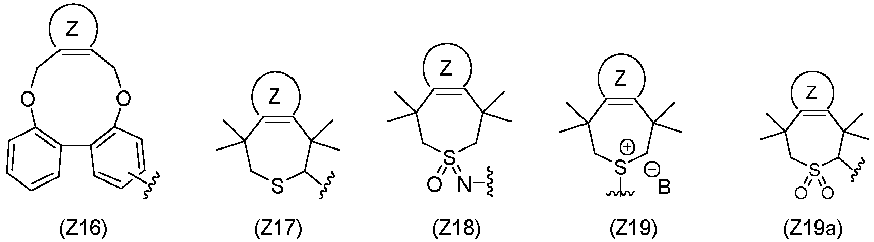

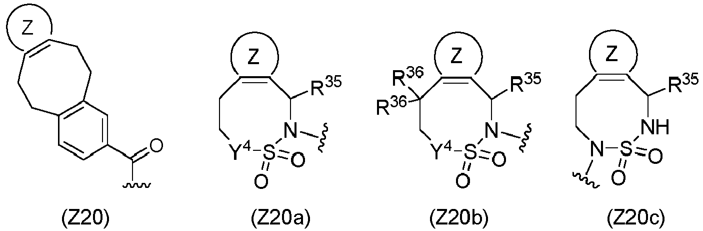

Herein, the connection to L is depicted with the wavy bond. B⁽⁻⁾ is an anion, preferably a pharmaceutically acceptable anion. B⁽⁺⁾ is a cation, preferably a pharmaceutically acceptable cation. R³⁶ is an halogen selected from fluor, chlorine, bromine and iodine, preferably R³⁶ is fluor. Y⁴ is a heteroatom, preferably Y⁴ is O or NH. R³⁵ is selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, Si, S and NR¹⁴ wherein R¹⁴ is independently selected from the group consisting of hydrogen and C₁ - C₄ alkyl groups, preferably R³⁵ is selected from H, C₅H₁₁, CH₃, CH₂CH₃, CH₂OH or CH₂OTBS.

Ring Z is formed by the cycloaddition reaction, and preferably is a triazole, a cyclohexene, a cyclohexadiene, a [2.2.2]-bicyclooctadiene, a [2.2.2]-bicyclooctene, an isoxazoline, an isoxazolidine, oxazolidine, a pyrazoline or a piperazine. Most preferably, ring Z is a triazole ring. Ring Z may have the structure selected from (Za) - (Zm) depicted below, wherein the carbon atoms labelled with ** correspond to the two carbon atoms of the (hetero)cycloalkane ring of (Z2) - (Z20), to which ring Z is fused. Since the connecting group Z is formed by reaction with a (hetero)cycloalkyne in the context of the present embodiment, the bond depicted above as --- is a double bond.

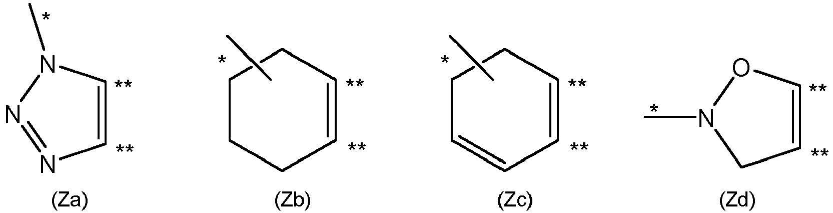

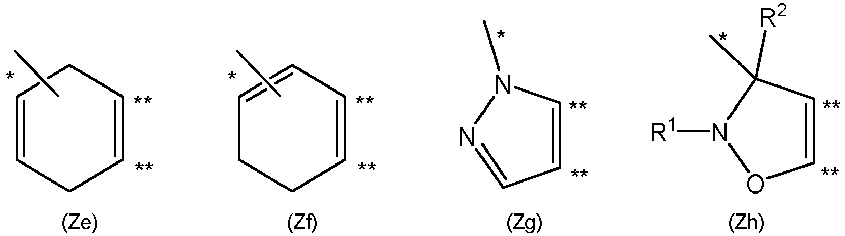

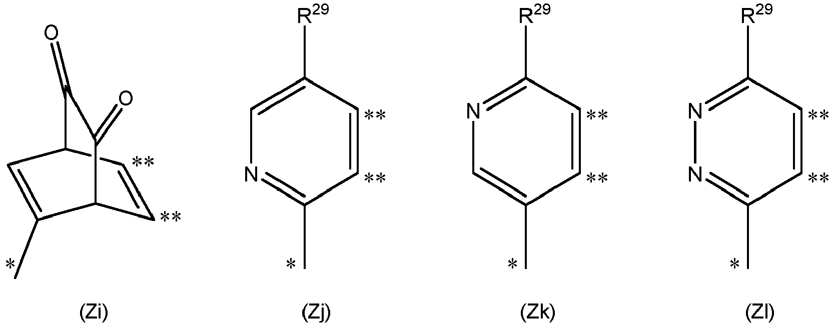

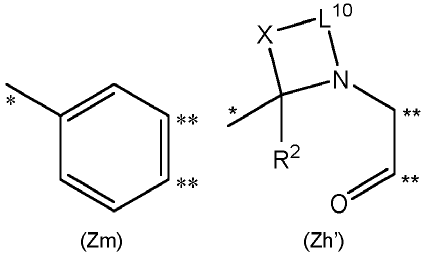

Herein, R²⁹ is selected from hydrogen, C₁₋₆ alkyl, aryl, C(O)-C₁₋₆ alkyl, C(O)-aryl, C(O)-O-C₁₋₆ alkyl, C(O)-O-aryl, C(O)-NR³³-C₁₋₆ alkyl and C(O)-NR³³-aryl, wherein R³³ is H or C₁₋₄ alkyl. Preferably, R²⁹ is selected from hydrogen, methyl, phenyl, pyridyl, pyridinyl and pyrimidinyl. It was found that R²⁹ is hydrogen gave optimal results in reactivity in the cycloaddition reaction, especially in case ring (Zl) is formed. Thus, in a preferred embodiment, ring Z is (Zl) wherein R²⁹ is selected from hydrogen, methyl, phenyl, pyridyl, pyridinyl and pyrimidinyl, more preferably R²⁹ is hydrogen.

For isoxazoline (Zh) and rearrangement product (Zh'), the following applies:
- R¹ is L¹⁰XR⁴;
- L¹⁰ is a linker of structure (C(R³)₂)_{z}, wherein z is 2 or 3, and each R³ is individually selected from H and C₁₋₄ alkyl, wherein two occurrences of R³ may be joined together to form a C₃₋₆ (hetero)cycloalkyl group;
- R⁴ is selected from H and C₁₋₄ alkyl;
- X is S, O or NH,
- R² is selected from H and C₁₋₄ alkyl.

Preferred embodiment are defined for nitrone reactive group (F3a) below, which equally apply to isoxazoline (Zh) and rearrangement product (Zh').

The inventors found that substituent R¹ improves the stability of the isoxazoline-based conjugates. Normally, isoxazolines may rearrange according to the scheme below:

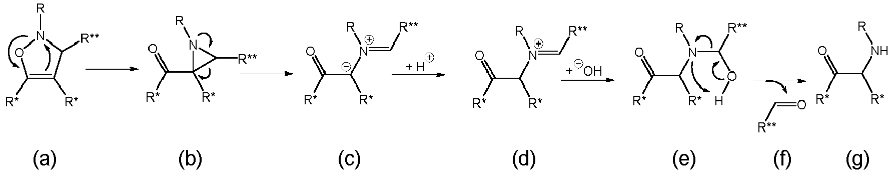

The a 4-isoxazoline ring may suffer from disintegration via this rearrangement. Isoxazoline ring (a) is rearranged into the aziridine ring (b), from which ring strain is released in zwitterionic species (c). Addition of water gives hydroxylamine (e), from which aldehyde (f) is split off to give ketonamine (g). This will not only destroy the isoxazoline ring, but also break the covalent linkage between the substituents at R* and the one at R**. Thus, in case the isoxazoline ring is part of a bioconjugate, the payload is removed from the biomolecule, therefore effectively eliminating its use and possibly creating severe side-effects.

The solution for the above problem developed by the present inventors involves a specific substituent R¹ on the nitrogen atom of the nitrone. Heteroatom X is able to capture the imine intermediate (d) of the rearrangement by reacting with the imine carbon atom. The inventors found that this leads not to splitting off of aldehyde (f), but instead leads to the rearrangement of the scheme below (exemplary scheme with R¹ = (CH₂)₂OH), wherein oxazolidine ring (h) is formed. This structure is stable and does not disintegrate. As such, the covalent connecting between the substituents at R* and at R** remains intact. The bioconjugate thus remains intact and effective, with the payload connected to the biomolecule. Thus, even though rearrangement of the 4-isoxazoline ring formed by the SPANC reaction is destroyed, the covalent connection is not, and therefore bioconjugates according to the present invention are a great improvement in terms of stability of the attachment of the payload to the biomolecule.

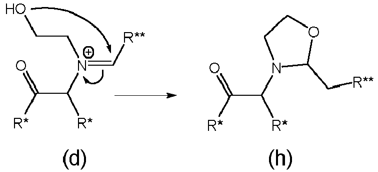

In case Z comprises a (hetero)cycloalkene moiety, it is preferred that ring Z is selected from (Za), (Zh), (Zj), (Zk) or (Zl), more preferably ring Z is according to structure (Za) or (Zl).

In a further preferred embodiment, Z is selected from the structures (Z21) - (Z38d), depicted here below:

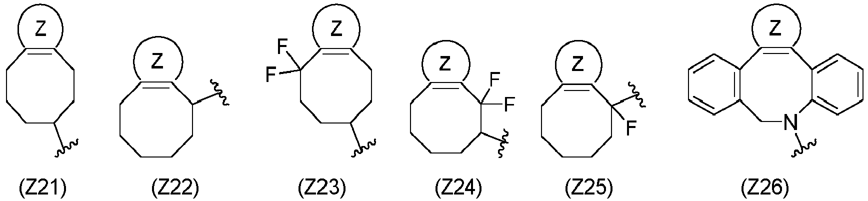

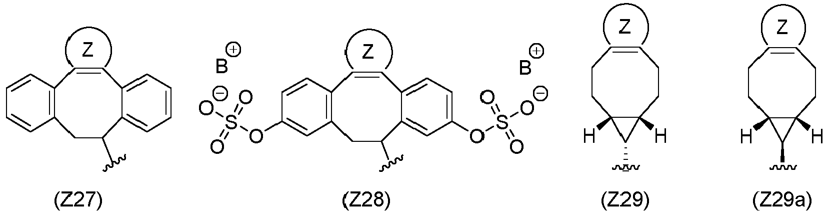

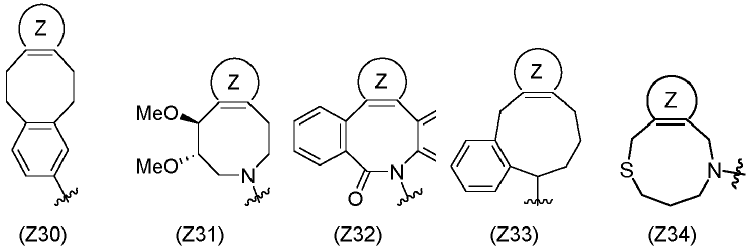

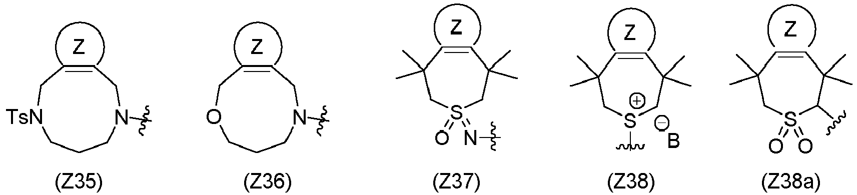

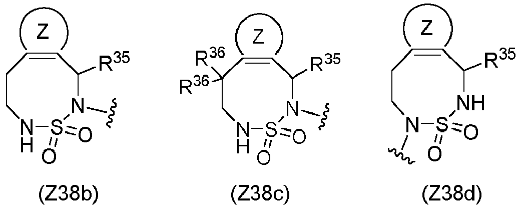

Herein, the connection to L is depicted with the wavy bond. Structure (Z29) can be in endo or exo configuration, preferably it is in endo configuration. In structure (Z38), B⁽⁻⁾ is an anion, preferably a pharmaceutically acceptable anion. B⁽⁺⁾ is a cation, preferably a pharmaceutically acceptable cation. Ring Z is selected from structures (Za) - (Zm), as defined above.

In a preferred embodiment, Z comprises a (hetero)cyclooctene moiety or a (hetero)cycloheptene moiety, preferably according to structure (Z8), (Z26), (Z27), (Z28), (Z37) or (Z38a), which are optionally substituted. Each of these preferred options for Z are further defined here below.

Thus, in a preferred embodiment, Z comprises a heterocycloheptene moiety according to structure (Z37) or (Z38a), which is optionally substituted. Preferably, the heterocycloheptene moiety according to structure (Z37) or (Z38a) is not substituted.

In a preferred embodiment, Z comprises a (hetero)cyclooctene moiety according to structure (Z8), more preferably according to (Z29), which is optionally substituted. Preferably, the cyclooctene moiety according to structure (Z8) or (Z29) is not substituted. In the context of the present embodiment, Z preferably comprises a (hetero)cyclooctene moiety according to structure (Z39) as shown below, wherein V is (CH₂)ₗ and I is an integer in the range of 0 to 10, preferably in the range of 0 to 6. More preferably, I is 0, 1, 2, 3 or 4, more preferably I is 0, 1 or 2 and most preferably I is 0 or 1. In the context of group (Z39), I is most preferably 1. Most preferably, Z is according to structure (Z42), defined further below.

In an alternative preferred embodiment, Z comprises a (hetero)cyclooctene moiety according to structure (Z26), (Z27) or (Z28), which is optionally substituted. In the context of the present embodiment, Z preferably comprises a (hetero)cyclooctene moiety according to structure (Z40) or (Z41) as shown below, wherein Y¹ is O or NR¹¹, wherein R¹¹ is independently selected from the group consisting of hydrogen, a linear or branched C₁ - C₁₂ alkyl group or a C₄- C₁₂ (hetero)aryl group. The aromatic rings in (Z40) are optionally O-sulfated at one or more positions, whereas the rings of (Z41) may be halogenated at one or more positions. Preferably, the (hetero)cyclooctene moiety according to structure (Z40) or (Z41) is not further substituted. Most preferably, Z is according to structure (Z43), defined further below.

In an alternative preferred embodiment, Z comprises a heterocycloheptenyl group and is according to structure (Z37).

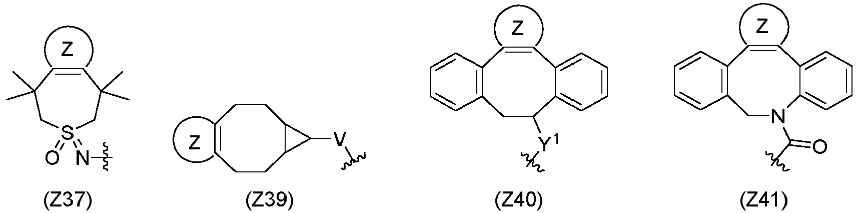

In an especially preferred embodiment, Z comprises a cyclooctenyl group and is according to structure (Z42): 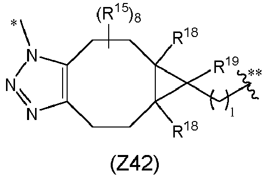 Herein:
- the bond labelled with * is connected to AB and the wavy bond labelled with ** is connected to L;
- R¹⁵ is independently selected from the group consisting of hydrogen, halogen, -OR¹⁶, -NO₂, -CN, -S(O)₂R¹⁶, -S(O)₃⁽⁻⁾,C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇- C₂₄ (hetero)arylalkyl groups;
- R¹⁸ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- R¹⁹ is selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted, or R¹⁹ is a second occurrence of Z (or Q) or D connected via a spacer moiety; and
- I is an integer in the range 0 to 10

In a preferred embodiment of the group according to structure (Z42), R¹⁵ is independently selected from the group consisting of hydrogen, halogen, -OR¹⁶, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and C₁ - C₆ alkyl, most preferably all R¹⁵ are H. In a preferred embodiment of the group according to structure (Z42), R¹⁸ is independently selected from the group consisting of hydrogen, C₁ - C₆ alkyl groups, most preferably both R¹⁸ are H. In a preferred embodiment of the group according to structure (Z42), R¹⁹ is H. In a preferred embodiment of the group according to structure (Z42), I is 0 or 1, more preferably I is 1

In an especially preferred embodiment, Z comprises a (hetero)cyclooctenyl group and is according to structure (Z43): 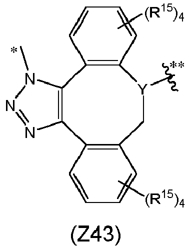 Herein:
- the bond labelled with * is connected to AB and the wavy bond labelled with ** is connected to L;
- R¹⁵ is independently selected from the group consisting of hydrogen, halogen, -OR¹⁶, -NO₂, -CN, -S(O)₂R¹⁶, -S(O)₃⁽⁻⁾, C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇- C₂₄ (hetero)arylalkyl groups;
- Y is N or CR¹⁵;
- a carbon atom in the fused aromatic rings may be replaced by a nitrogen atom, as in (Z6a) - (Z6d), preferably wherein Y is CR¹⁵.

In a preferred embodiment of the group according to structure (Z43), R¹⁵ is independently selected from the group consisting of hydrogen, halogen, -OR¹⁶, -S(O)₃⁽⁻⁾, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and -S(O)₃⁽⁻⁾. In a preferred embodiment of the group according to structure (Z43), Y is N or CH, more preferably Y = N.

In an especially preferred embodiment, Z comprises a heterocycloheptenyl group and is according to structure (Z37) or (Z38a), wherein ring Z is a triazole.

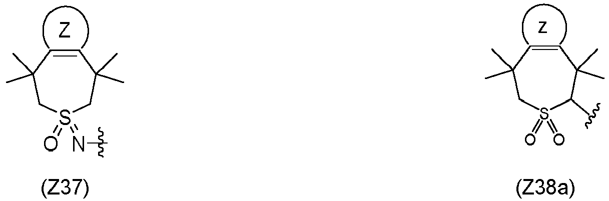

In an alternative preferred embodiment, connecting group Z comprises a (hetero)cycloalkane moiety, i.e. the bond depicted as --- is a single bond. The (hetero)cycloalkane group may also be referred to as a heterocycloalkyl group or a cycloalkyl group, preferably a cycloalkyl group, wherein the (hetero)cycloalkyl group is optionally substituted. Preferably, the (hetero)cycloalkyl group is a (hetero)cyclopropyl group, a (hetero)cyclobutyl group, a norbornyl group, a norbornenyl group, a (hetero)cycloheptyl group, a (hetero)cyclooctyl group, which may all optionally be substituted. Especially preferred are (hetero)cyclopropyl groups, (hetero)cycloheptyl group or (hetero)cyclooctyl groups, wherein the (hetero)cyclopropyl group, the (hetero)cycloheptyl group or the (hetero)cyclooctyl group is optionally substituted. Preferably, Z comprises a cyclopropyl moiety according to structure (Z44), a hetereocyclobutane moiety according to structure (Z45), a norbornane or norbornene group according to structure (Z46), a (hetero)cycloheptyl moiety according to structure (Z47) or a (hetero)cyclooctyl moiety according to structure (Z48). Herein, Y³ is selected from C(R²³)₂, NR²³ or O, wherein each R²³ is individually hydrogen, C₁ - C₆ alkyl or is connected to L, optionally via a spacer, and the bond labelled --- is a single or double bond. In a further preferred embodiment, the cyclopropyl group is according to structure (Z49). In another preferred embodiment, the (hetero)cycloheptane group is according to structure (Z50) or (Z51). In another preferred embodiment, the (hetero)cyclooctane group is according to structure (Z52), (Z53), (Z54), (Z55) or (Z56).

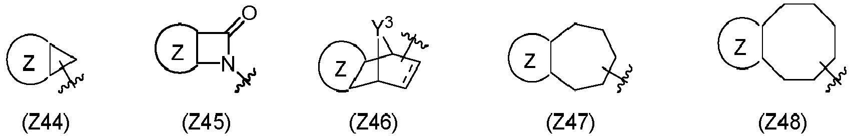

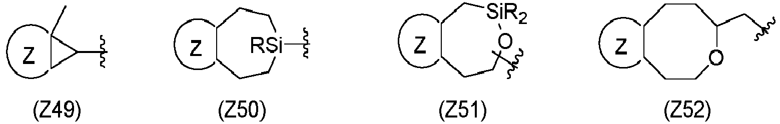

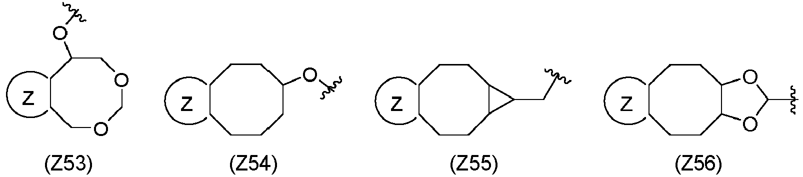

Herein, the R group(s) on Si in (Z50) and (Z51) are typically alkyl or aryl, preferably C₁-C₆ alkyl. Ring Z is formed during the cycloaddition reaction and is typically selected from structures (Zn) - (Zu), wherein the carbon atoms labelled with ** correspond to the two carbon atoms of the (hetero)cycloalkane ring of (Z44) - (Z56) to which ring Z is fused, and the carbon a carbon labelled with * is connected to AB. Since the connecting group Z is formed by reaction with a (hetero)cycloalkene in the context of the present embodiment, the bound depicted above as --- is a single bond.

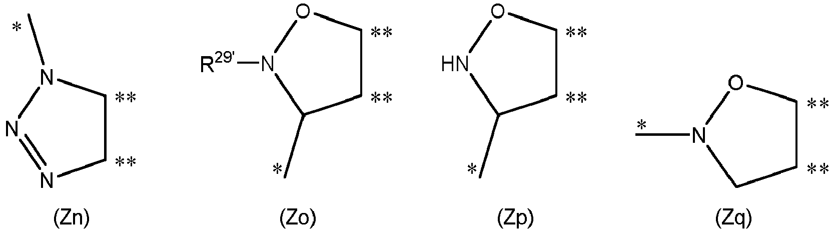

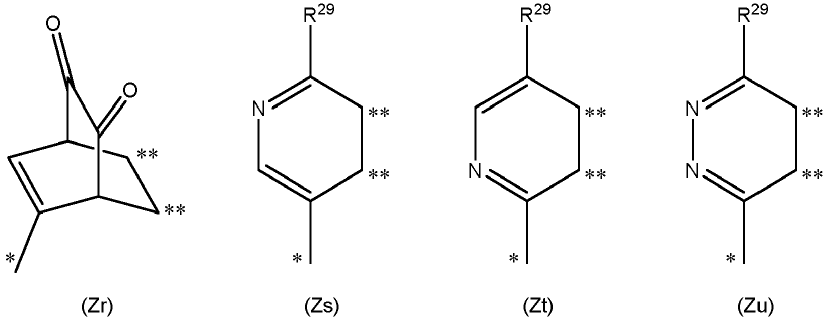

Herein, R²⁹ is selected from hydrogen, C₁₋₆ alkyl, aryl, C(O)-C₁₋₆ alkyl, C(O)-aryl, C(O)-O-C₁₋₆ alkyl, C(O)-O-aryl, C(O)-NR³³-C₁₋₆ alkyl and C(O)-NR³³-aryl, wherein R³³ is H or C₁₋₄ alkyl. Preferably, R²⁹ is selected from hydrogen, methyl, phenyl, pyridyl, pyridinyl and pyrimidinyl. It was found that R²⁹ is hydrogen gave optimal results in reactivity in the cycloaddition reaction, especially in case ring (Zu) is formed. Thus, in a preferred embodiment, ring Z is (Zu) wherein R²⁹ is selected from hydrogen, methyl, phenyl, pyridyl, pyridinyl and pyrimidinyl, more preferably R²⁹ is hydrogen.

In case Z comprises a (hetero)cycloalkane moiety, it is preferred that ring Z is selected from (Zn), (Zs), (Zt) or (Zu), most preferably ring Z is according to structure (Zu).

In a preferred embodiment, connection group Z comprise a moiety selected from (Z1) - (Z56), wherein ring Z is selected from (Za) - (Zu).

In the present invention, the exact structure of Z¹ and Z² typically differ, as Z¹ is formed by reaction of Q¹ and F¹, whereas Z² is formed by reaction of Q² and F². Preferably herein, F² is reactive towards Q² but not towards Q¹, such that Q¹ and Q² should differ.

In a preferred embodiment, F¹ is azide and Q¹ is a benzoannulated or tetramethylated (hetero)cycloalkyne, while F² is tetrazine and Q² is bicyclononyne. Herein, the reaction of F¹ and Q¹ will preferably form a connecting group Z¹ according to structure (Z5), (Z6), (Z7), (Z11), (Z17), (Z18), (Z19) or (Z19a), wherein ring Z is according to structure (Za), preferably according to structure (Z26), (Z27), (Z28), (Z32), (Z37), (Z38) or (Z38a), more preferably according to structure (Z40), (Z41) or (Z43). Herein, the reaction of F² and Q² will preferably form a connecting group Z² according to structure (Z8), wherein ring Z is according to structure (Zl), preferably according to structure (Z29), more preferably according to structure (Z42).

In yet another preferred embodiment, F¹ is azide and Q¹ is a cycloalkyne while Q² is a nitrone, ortho-quinone or tetrazine and F² is a cycloalkene, preferably trans-cyclooctene. Herein, the reaction of F¹ and Q¹ will preferably form a connecting group Z¹ according to structure (Z2) - (Z20), wherein ring Z is according to structure Za, preferably according to (Z8). Herein, the reaction of F² and Q² will preferably form a connecting group Z² according to (Z44) - (Z56), wherein ring Z is according to structure (Zo), (Zp), (Zq), (Zr), (Zq) preferably according to structure (Zo) or (Zu), more preferably according to structure (Zo).

In another preferred embodiment, Z¹ is obtainable by a click reaction between cycloalkyne and azide, whereas Z² is obtainable by a click reaction between cylcoalkyne and nitrone.

In a second preferred embodiment, Z, preferably Z¹, is formed by a nucleophilic reaction, preferably by a nucleophilic substitution or a Michael addition, preferably by a Michael addition. A preferred Michael reaction is the thiol-maleimide ligation, most preferably wherein Q is maleimide and F is a thiol group, wherein the thiol may be part of a disulphide bridge. Preferably, the thiol is present in the sidechain of a cysteine residue. Such a conjugation reaction with a thiol may also be referred to as thiol alkylation or thiol arylation. In a preferred embodiment, connection group Z¹ comprises a succinimidyl ring or its ring-opened succinic acid amide derivative, which may be formed by hydrolysis of the succinimidyl ring] In case a nucleophilic reaction is used for the conjugation, it is preferred that the structural moiety Q-(L¹)ₐ-BM-(L²)b-Q is selected from bromomaleimide, bis-bromomaleimide, bis(phenylthiol)maleimide, bis-bromopyridazinedione, bis(halomethyl)benzene, bis(halomethyl)pyridazine, bis(halomethyl)pyridine or bis(halomethyl)triazole.

Alternatively, Z¹ is formed by nucleophilic reaction at the amino group in the sidechain of a lysine residue (F), which may react with amino reactive groups Q. Such a conjugation reaction with a thiol may also be referred to as amide bond formation or carbamate bond formation. Typical amino reactive groups Q include N-hydroxysuccinimidyl (NHS) esters, *p*-nitrophenyl carbonates, pentafluorophenyl carbonates, isocyanates, isothiocyanates and benzoyl halides.

Preferred options for connection group Z¹ comprise a moiety selected from (Z57) - (Z71) depicted here below.

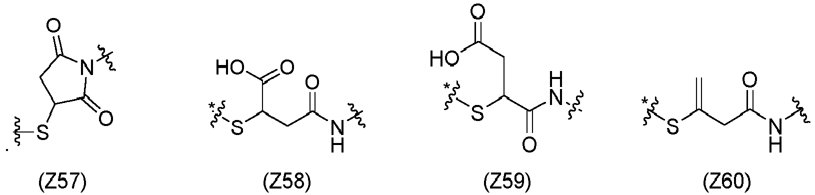

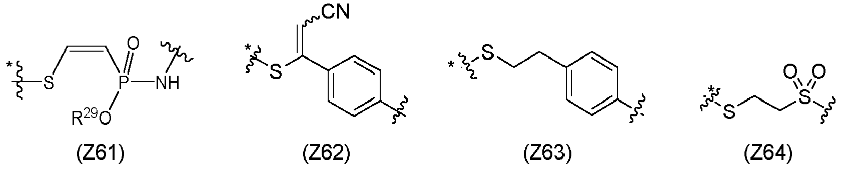

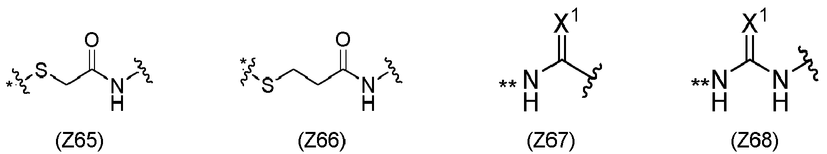

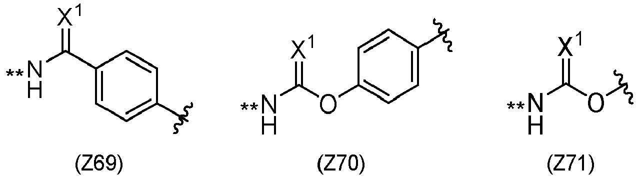

Herein, the wavy bond(s) labelled with an * in (Z57)-(Z66) is connected to AB, and the wavy bond without label to the payload via linker L. In addition, R²⁹ is C₁₋₁₂ alkyl, preferably C₁₋₄ alkyl, most preferably ethyl, and X¹ is O or S, preferably X¹ = O. The nitrogen atom labelled with ** in (Z67)-(Z71) corresponds to the nitrogen atom of the side chain of a lysine residue of the antibody, and the wavy bond without label to the payload via linker L. The carbon atoms of the phenyl group of (Z69) and (Z70) are optionally substituted, preferably optionally fluorinated.

### The linker L

Linker L connects payload D, via connecting group Z², with connecting group Z¹ (in the conjugates according to the invention) or connects payload D with reactive group Q (in the linker-cytokine constructs) or connects antibody with reactive group (in the linker-antibody-constructs). Linkers are known in the art and may be cleavable or non-cleavable.

In a preferred embodiment, linker L is represented by structure -(L¹)-(L²)ₒ-(L³)ₚ-(L⁴)_{q}-wherein:
- L¹ is connected to Z¹ and L⁴ if present is connected to Z²;
- L¹, L², L³ and L⁴ are each individually linkers that together link Z¹ to Z²;
- o, p and q are each individually 0 or 1, preferably o = p = q = 0.

### Linker L¹

L¹ may for example be selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups, C₉-C₂₀₀ arylalkynylene groups. Optionally the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups may be substituted, and optionally said groups may be interrupted by one or more heteroatoms, preferably 1 to 100 heteroatoms, said heteroatoms preferably being selected from the group consisting of O, S(O)y and NR²¹, wherein y' is 0, 1 or 2, preferably y' = 2, and R²¹ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups. In one embodiment, the optional substituents may be selected from polar groups, such as oxo groups, (poly)ethylene glycol diamines, (poly)ethylene glycol or (poly)ethylene oxide chains, (poly)propylene glycol or (poly)propylene oxide chains, carboxylic acid groups, carbonate groups, carbamate groups, cyclodextrins, crown ethers, saccharides (e.g. monosaccharides, oligosaccharides), phosphates or esters thereof, phosphonic acid or ester, phosphinic acid or ester, sulfoxides, sulfones, sulfonic acid or ester, sulfinic acid, or sulfenic acid.

In a preferred embodiment, linker L¹ contains a polar group, which may also be present in the chain of L¹. Such a polar group may be selected from (poly)ethylene glycol diamines (e.g. 1,8-diamino-3,6-dioxaoctane or equivalents comprising longer ethylene glycol chains), (poly)ethylene glycol or (poly)ethylene oxide chains, (poly)propylene glycol or (poly)propylene oxide chains and 1,x'-diaminoalkanes (wherein x' is the number of carbon atoms in the alkane, preferably x' = 1 - 10), -(O)ₐ-C(O)-NH-S(O)₂-NR¹³- (as further defined below, see structure (**23**)), -C(S(O)₃⁽⁻⁾)-,

-C(C(O)₂⁽⁻⁾)-, -S(O)₂-, -P(O)₂⁽⁻⁾-, -O(CH₂CH₂O)ₜ-, -NR³⁰(CH₂CH₂NR³⁰)ₜ-, and the following two structures:

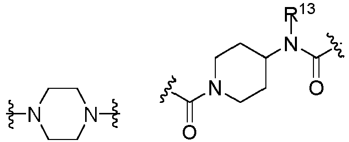

For the polar groups defined here above, it is irrelevant which end is connected to Z¹ and which end to (L²)ₒ.

The polar group may also contain an amino acid including an unnatural amino acid, preferably selected from Arg, Glu, Asp, Ser, Thr or cysteic acid. Herein, R¹³ is further defined below for structure (23). t is an integer in the range of integer in the range of 0 - 15, preferably 1 - 10, more preferably 2-5, most preferably t = 2 or 4. Each R³⁰ is individually H, C₁₋₁₂ alkyl, C₁₋₁₂ aryl, C₁₋₁₂ alkaryl or C₁₋₁₂ aralkyl. Linker L¹ may contain more than one such polar group, such as at least two polar groups. The polar group may also be present in a branch of linker L¹, which branches off a branching moiety as defined elsewhere. In the context of L¹, a nitrogen or carbon atom is preferably used as branching moiety. It is especially preferred to have a -O(CH₂CH₂O)ₜ- polar group present in a branch.

In a preferred embodiment, Linker L¹ is or comprises a sulfamide group, preferably a sulfamide group according to structure (**23**):

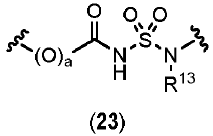

The wavy lines represent the connection to the remainder of the compound, typically to Q² or Z² and to L², L³, L⁴ or D. Preferably, the (O)ₐC(O) moiety is connected to Q² or Z² and the NR¹³ moiety to L², L³, L⁴ or D, preferably to L²

In structure (**23**), a = 0 or 1, preferably a = 1, and R¹³ is selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂-C₂₄ (hetero)aryl groups, C₃-C₂₄ alkyl(hetero)aryl groups and C₃-C₂₄ (hetero)arylalkyl groups optionally substituted and optionally interrupted by one or more heteroatoms selected from O, S and NR¹⁴ wherein R¹⁴ is independently selected from the group consisting of hydrogen and C₁ - C₄ alkyl groups. Alternatively, R¹³ is D connected to N optionally via a spacer moiety, preferably via Sp² as defined below, in one embodiment D is connected to N via -(B)ₑ-(A)_{f}-(B)_{g}-C(O)-. Alternatively, R¹³ is connected to elsewhere in the linker, optionally via a spacer moiety, to form a cyclic structure. For example, R¹³ may be connected to the linker via a CH₂CH₂ spacer moiety to form a piperazinyl ring, where the connection to D is via the second nitrogen of the piperazinyl ring.

In a preferred embodiment, R¹³ is hydrogen, a C₁ - C₂₀ alkyl group, preferably a C₁-- C₁₆ alkyl group, more preferably a C₁ - C₁₀ alkyl group, or connected to a further occurrence of D or to elsewhere in the linker optionally via a spacer moiety. Herein, the alkyl group is optionally substituted and optionally interrupted by one or more heteroatoms selected from O, S and NR¹⁴, preferably O, wherein R¹⁴ is independently selected from the group consisting of hydrogen and C₁ -C₄ alkyl groups. In another preferred embodiment, R¹³ is a C₁ - C₂₀ alkyl group, more preferably a C₁ -C₁₆ alkyl group, even more preferably a C₁- C₁₀ alkyl group, wherein the alkyl group is optionally interrupted by one or more O-atoms, and wherein the alkyl group is optionally substituted with an -OH group, preferably a terminal -OH group. In this embodiment it is further preferred that R¹³ is a (poly)ethylene glycol chain comprising a terminal -OH group. In another preferred embodiment, R¹³ is selected from the group consisting of hydrogen, methyl, ethyl, n-propyl, i-propyl, n-butyl, s-butyl and t-butyl or to elsewhere in the linker optionally via a spacer moiety, more preferably from the group consisting of hydrogen, methyl, ethyl, n-propyl and i-propyl, or connected to a further occurrence of D or to elsewhere in the linker optionally via a spacer moiety, and even more preferably from the group consisting of hydrogen, methyl and ethyl, or connected to a further occurrence of D or to elsewhere in the linker optionally via a spacer moiety. Yet even more preferably, R¹³ is hydrogen or connected to a further occurrence of D or to elsewhere in the linker optionally via a spacer moiety, and most preferably R¹³ is hydrogen.

In a preferred embodiment, L¹ is according to structure (**24**):

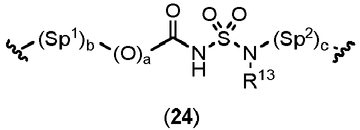

Herein, a and R¹³ are as defined above, Sp¹ and Sp² are independently spacer moieties and b and c are independently 0 or 1. Preferably, b = 0 or 1 and c = 1, more preferably b = 0 and c = 1. In one embodiment, spacers Sp¹ and Sp² are independently selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups and C₉-C₂₀₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂- C₂₄ alkenyl groups, C₂- C₂₄ alkynyl groups and C₃- C₂₄ cycloalkyl groups, the alkyl groups, alkenyl groups, alkynyl groups and cycloalkyl groups being optionally substituted. When the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups are interrupted by one or more heteroatoms as defined above, it is preferred that said groups are interrupted by one or more O-atoms, and/or by one or more S-S groups

More preferably, spacer moieties Sp¹ and Sp², if present, are independently selected from the group consisting of linear or branched C₁-C₁₀₀ alkylene groups, C₂-C₁₀₀ alkenylene groups, C₂-C₁₀₀ alkynylene groups, C₃-C₁₀₀ cycloalkylene groups, C₅-C₁₀₀ cycloalkenylene groups, C₈-C₁₀₀ cycloalkynylene groups, C₇-C₁₀₀ alkylarylene groups, C₇-C₁₀₀ arylalkylene groups, C₈-C₁₀₀ arylalkenylene groups and C₉-C₁₀₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂- C₂₄ alkenyl groups, C₂- C₂₄ alkynyl groups and C₃- C₂₄ cycloalkyl groups, the alkyl groups, alkenyl groups, alkynyl groups and cycloalkyl groups being optionally substituted.

Even more preferably, spacer moieties Sp¹ and Sp², if present, are independently selected from the group consisting of linear or branched C₁-C₅₀ alkylene groups, C₂-C₅₀ alkenylene groups, C₂-C₅₀ alkynylene groups, C₃-C₅₀ cycloalkylene groups, C₅-C₅₀ cycloalkenylene groups, C₈-C₅₀ cycloalkynylene groups, C₇-C₅₀ alkylarylene groups, C₇-C₅₀ arylalkylene groups, C₈-C₅₀ arylalkenylene groups and C₉-C₅₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂- C₂₄ alkenyl groups, C₂- C₂₄ alkynyl groups and C₃- C₂₄ cycloalkyl groups, the alkyl groups, alkenyl groups, alkynyl groups and cycloalkyl groups being optionally substituted.

Yet even more preferably, spacer moieties Sp¹ and Sp², if present, are independently selected from the group consisting of linear or branched C₁-C₂₀ alkylene groups, C₂-C₂₀ alkenylene groups, C₂-C₂₀ alkynylene groups, C₃-C₂₀ cycloalkylene groups, C₅-C₂₀ cycloalkenylene groups, C₈-C₂₀ cycloalkynylene groups, C₇-C₂₀ alkylarylene groups, C₇-C₂₀ arylalkylene groups, C₈-C₂₀ arylalkenylene groups and C₉-C₂₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂- C₂₄ alkenyl groups, C₂- C₂₄ alkynyl groups and C₃- C₂₄ cycloalkyl groups, the alkyl groups, alkenyl groups, alkynyl groups and cycloalkyl groups being optionally substituted.

In these preferred embodiments it is further preferred that the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups are unsubstituted and optionally interrupted by one or more heteroatoms selected from the group of O, S and NR¹⁶, preferably O, wherein R¹⁶ is independently selected from the group consisting of hydrogen and C₁ - C₄ alkyl groups, preferably hydrogen or methyl.

Most preferably, spacer moieties Sp¹ and Sp², if present, are independently selected from the group consisting of linear or branched C₁-C₂₀ alkylene groups, the alkylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂- C₂₄ alkenyl groups, C₂- C₂₄ alkynyl groups and C₃ - C₂₄ cycloalkyl groups, the alkyl groups, alkenyl groups, alkynyl groups and cycloalkyl groups being optionally substituted. In this embodiment, it is further preferred that the alkylene groups are unsubstituted and optionally interrupted by one or more heteroatoms selected from the group of O, S and NR¹⁶, preferably O and/or S-S, wherein R¹⁶ is independently selected from the group consisting of hydrogen and C₁ - C₄ alkyl groups, preferably hydrogen or methyl.

Preferred spacer moieties Sp¹ and Sp² thus include -(CH₂)ᵣ-, -(CH₂CH₂)ᵣ-, -(CH₂CH₂O)ᵣ-, -(OCH₂CH₂)ᵣ-, -(CH₂CH₂O)ᵣCH₂CH₂-, -CH₂CH₂(OCH₂CH₂)ᵣ-, -(CH₂CH₂CH₂O)ᵣ-, -(OCH₂CH₂CH₂)ᵣ-, -(CH₂CH₂CH₂O)ᵣCH₂CH₂CH₂- and -CH₂CH₂CH₂(OCH₂CH₂CH₂)ᵣ-, wherein r is an integer in the range of 1 to 50, preferably in the range of 1 to 40, more preferably in the range of 1 to 30, even more preferably in the range of 1 to 20 and yet even more preferably in the range of 1 to 15. More preferably n is 1, 2, 3, 4, 5, 6, 7, 8, 9 or 10, more preferably 1, 2, 3, 4, 5, 6, 7 or 8, even more preferably 1, 2, 3, 4, 5 or 6, yet even more preferably 1, 2, 3 or 4.

Alternatively, preferred linkers L¹ may be represented by -(W)ₖ-(A)_{d}-(B)ₑ(A)_{f}-(C(O))_{g}- or [-(W)ₖ-(A)_{d}-(B)ₑ-(A)_{f}-]₂BM-(C(O))_{g}- (A)_{d'}-(B)_{e'}-(A)_{f'}-(C(O))_{g'}-, wherein
- d and d' are individually 0 or 1,
- e and e' are individually an integer in the range 1-10,
- f and f are individually 0 or 1,
- g and g' are individually an integer in the range 0-10,
- k = 0 or 1 with the proviso that if k = 1 then d = 0,
- A is a sulfamide group according to structure (**23**) 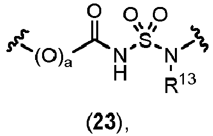
- B is a -CH₂-CH₂-O- or a -O-CH₂-CH₂- moiety, or (B)ₑ is a -(CH₂-CH₂-O)ₑ₁-CH₂-CH₂- or a -(CH₂-CH₂-O)ₑ₁-CH₂- moiety, wherein e1 is defined the same way as e;
- W is -OC(O)-, -C(O)O-, -C(O)NH-, -NHC(O)-, -OC(O)NH-, -NHC(O)O-, -C(O)(CH₂)ₘC(O)-, -C(O)(CH₂)ₘC(O)NH- or -(4-Ph)CH₂NHC(O)(CH₂)ₘC(O)NH-, preferably wherein W is -OC(O)NH-, -C(O)(CH₂)ₘC(O)NH- or -C(O)NH-, and wherein m is an integer in the range 0 - 10, preferably m = 0, 1, 2, 3, 4, 5 or 6, most preferably m = 2 or 3;
- preferably wherein L¹ is connected to Q via (W)ₖ and to L², L³ or D, preferably to L², via (C(O))_{g}, preferably via C(O);
- BM is a branching moiety, preferably selected from a carbon atom, a nitrogen atom, a phosphorus atom, a (hetero)aromatic ring, a (hetero)cycle or a polycyclic moiety, more preferably BM is a nitrogen atom.

In the context of the present embodiment, the wavy lines in structure (**23**) represent the connection to the adjacent groups such as (W)ₖ, (B)ₑ and (C(O))_{g}. It is preferred that A is according to structure (**23**), wherein a = 1 and R¹³ = H or a C₁- C₂₀ alkyl group, more preferably R¹³ = H or methyl, most preferably R¹³ = H.

Preferred linkers L¹ have structure -(W)ₖ-(A)_{d}-(B)ₑ-(A)_{f}-(C(O))_{g}-, wherein:
(a) k = 0; d = 1; g = 1; f= 0; B = -CH₂CH₂-O-; e = 1, 2, 3 or 4, preferably e = 2.
(b) k = 1; W = -C(O)(CH₂)ₘC(O)NH-; m = 2; d = 0; (B)ₑ = -(CH₂-CH₂-O)ₑ₁-CH₂-CH₂-; f = 0; g = 1; e1 = 1, 2, 3 or 4, preferably e = 1.
(c) k = 1; W = -OC(O)NH-; d = 0; B = -CH₂-CH₂-O-; g = 1; f = 0; e = 1, 2, 3 or 4, preferably e = 2.
(d) k = 1; W = -C(O)(CH₂)ₘC(O)NH-; m = 2; d = 0; (B)ₑ = -(CH₂-CH₂-O)ₑ₁-CH₂-CH₂-; f = 0; g = 1; e1 = 1, 2, 3 or 4, preferably e1 = 4.
(e) k = 1; W = -OC(O)NH-; d = 0; (B)ₑ = -(CH₂-CH₂-O)ₑ₁-CH₂-CH₂-; g = 1; f = 0; e1 = 1, 2, 3 or 4, preferably e1 = 4.
(f) k = 1; W = -(4-Ph)CH₂NHC(O)(CH₂)ₘC(O)NH-, m = 3; d = 0; (B)ₑ = -(CH₂CH₂-O)ₑ₁-CH₂-CH₂-; g = 1; f = 0; e1 = 1, 2, 3 or 4, preferably e1 = 4.
(g) k = 0; d = 0; g = 1; f = 0; B = -CH₂-CH₂-O-; e = 1, 2, 3 or 4, preferably e = 2.
(h) k = 1; W = -C(O)NH-; d = 0; g = 1; f= 0; B = -CH₂-CH₂-O-; e = 1, 2, 3 or 4, preferably e = 2.

Herein, it is preferred that when d and/or f = 1, than a = 1 and R¹³ = H. Most preferred is the linker is structure (a).

In a preferred embodiment, linker L¹ comprises a branching nitrogen atom, which is located in the backbone between Q¹ or Z¹ and (L²)ₒ and which contains a further moiety Z¹ or Q¹ as substituent, which is preferably linked to the branching nitrogen atom via a linker. An example of a branching nitrogen atom is the nitrogen atom NR¹³ in structure (**23**), wherein R¹³ is connected to a second occurrence of D via a spacer moiety. Alternatively, a branching nitrogen atoms may be located within L¹ according to structure -(W)ₖ-(A)_{d}-(B)ₑ-(A)_{f-}(C(O))_{g}-. In one embodiment, L¹ is represented by [-(W)ₖ-(A)_{d}-(B)ₑ-(A)_{f}-(C(O))_{g}-]₂BM-(A)_{d}-(B)ₑ-(A)_{f}-(C(O))_{g}-, wherein A, B, W, d, e, f, g and k are as defined above and individually selected for each occurrence, and BM is a branching moiety, preferably a branching nitrogen atom, to which two instances of -(A)_{d}-(B)ₑ-(A)_{f}-(C(O))_{g}- are connected. Herein, both (C(O))_{g} moieties are connected to - (L²)ₒ-(L³)ₚ-(L⁴)_{q}-D, wherein L², L³, L⁴, o, p, q and D are as defined above and are each selected individually. In a preferred embodiment, each of L², L³, L⁴, o, p, q and D are the same for both moieties connected to (C(O))_{g}.

Preferred linkers L¹ comprising a branching moiety atom have structure [-(W)ₖ-(A)_{d}-(B)ₑ-(A)_{f}-(C(O))_{g}-]₂N-(A')_{d}-(B')_{e'}-(A')_{f'}-(C(O))_{g'}- wherein:
(i) k = d = g = e' = 1; f = d' = g' = 0; W = -C(O)-; B = B' = -CH₂-CH₂-O-; A is according to structure (**23**) with a = 0 and R¹³ = H; e = 1, 2, 3 or 4, preferably e = 2.
(j) k = d = g = e' = g' = 1; f = d' = 0; W = -C(O)-; B = B' = -CH₂-CH₂-O-; A is according to structure (**23**) with a = 0 and R¹³ = H; e = 1, 2, 3 or 4, preferably e = 2.

### Linker L²

Linker L² is a peptide spacer. Linker L² is either absent (o = 0) or present (o = 1). Preferably, linker L² is present and o = 1. The combination of a peptide spacer L² and a cleavable linker L³ is well-known in the art. Linker L² functions as recognition and cleave site for cleaving-enzymes, more preferably the peptides are recognized by specific cleaving enzymes. This allows for cleavage at specific environments in which these cleaving enzymes are expressed such as specific tumors. Since different peptide sequences are cleaved by different enzymes, the L² group also allows customizing the conjugate for specific treatments.

The peptide spacer may also be defined by (NH-CR¹⁷-CO)ₙ, wherein R¹⁷ represents an amino acid side chain as known in the art. Also covered within this definition is proline, which has R¹⁷ joined with the nitrogen atom to form a cyclic moiety. Herein, the amino acid may be a natural or a synthetic amino acid. Preferably, the amino acid(s) are all in their L-configuration. n is an integer in the range of 1 - 5, preferably in the range of 2 - 4. Thus, the peptide spacer contains 1 - 5 amino acids. Preferably, the peptide is a dipeptide (n = 2), tripeptide (n = 3) or tetrapeptide (n = 4), most preferably the peptide spacer is a dipeptide. R¹⁷ represents the amino acid side chain, preferably selected from the side chains of alanine, cysteine, aspartic acid, glutamic acid, phenylalanine, glycine, histidine, isoleucine, lysine, acetyllysine, leucine, methionine, asparagine, pyrrolysine, proline, glutamine, arginine, serine, threonine, selenocysteine, valine, tryptophan, tyrosine and citrulline. Preferred amino acid side chains are those of Val, Cit, Ala, Lys, Arg, AcLys, Phe, Leu, Ile, Trp, Glu, Asp and Asn, more preferably from the side chains of Val, Cit, Ala, Glu and Lys. Alternatively worded, R¹⁷ is preferably selected from CH₃ (Ala), CH₂CH(CH₃)₂ (Leu), CH₂CH₂CH₂NHC(O)NH₂ (Cit), CH₂CH₂CH₂CH₂NH₂ (Lys), CH₂CH₂CH₂NHC(O)CH₃ (AcLys), CH₂CH₂CH₂NHC(=NH)NH₂ (Arg), CH₂Ph (Phe), CH(CH₃)₂ (Val), CH(CH₃)CH₂CH₃ (Ile), CH₂C(O)NH₂ (Asn), CH₂CH₂C(O)OH (Glu), CH₂C(O)OH (Asp) and CH₂(1*H*-indol-3-yl) (Trp). Especially preferred embodiments of R¹⁷ are CH₃ (Ala), CH₂CH₂CH₂NHC(O)NH₂ (Cit), CH₂CH₂CH₂CH₂NH₂ (Lys), CH₂CH₂C(O)OH (Glu) and CH(CH₃)₂ (Val). Most preferably, R¹⁷ is CH₃ (Ala), CH₂CH₂CH₂NHC(O)NH₂ (Cit), CH₂CH₂CH₂CH₂NH₂ (Lys), or CH(CH₃)₂ (Val).

Although any peptide spacer may be used, preferably the peptide spacer is selected from Val-Cit, Val-Ala, Val-Lys, Val-Arg, AcLys-Val-Cit, AcLys-Val-Ala, Glu-Val-Ala, Asp-Val-Ala, iGlu-Val-Ala, Glu-Val-Cit, Glu-Gly-Cit, Glu-Gly-Val, Asp-Val-Cit, iGlu-Val-Cit, Phe-Cit, Phe-Ala, Phe-Lys, Phe-Arg, Ala-Lys, Leu-Cit, Ile-Cit, Trp-Cit, Asn-Asn, Ala-Ala-Asn, Ala-Asn, Asn-Ala, Phe-Phe, Gly, Gly-Gly, Gly-Gly-Gly, Gly-Gly-Gly-Gly, Leu-Gly, Tyr-Gly, Ala-Gly, Pro-Gly, Phe-Gly, Phe-Gly, Ser-Gly, Gly-Phe-Gly, Gly-Gly-Phe-Gly, Gly-Phe-Gly-Gly, Phe-Gly-Gly-Gly, Gly-Gly-Gly-Phe, Phe-Phe-Gly-Gly, Gly-Gly-Phe-Phe, Gly-Gly-Gly-Phe-Gly and Lys, more preferably Val-Cit, Val-Ala, Glu-Val-Ala, Val-Lys, Phe-Cit, Phe-Ala, Phe-Lys, Ala-Ala-Asn, more preferably Glu-Gly-Cit, Val-Cit, Val-Ala, Asn-Asn, Ala-Ala-Asn, Asn-Ala most preferably Glu-Gly-Cit, Val-Cit, Val-Ala or Asn-Ala. Herein, AcLys is e-N-acetyllysine and iGlu is isoglutamate. In one embodiment, L² = Val-Cit. In another embodiment, L² = Val-Ala. In another embodiment, L² = Asn-Ala. In another embodiment, L² = Glu-Gly-Cit.

R¹⁷ represents the amino acid side chain, preferably selected from the side chains of alanine, cysteine, aspartic acid, glutamic acid, phenylalanine, glycine, histidine, isoleucine, lysine, acetyllysine, leucine, methionine, asparagine, pyrrolysine, proline, glutamine, arginine, serine, threonine, selenocysteine, valine, tryptophan, tyrosine and citrulline. Preferred amino acid side chains are those of Val, Cit, Ala, Lys, Arg, AcLys, Phe, Leu, Ile, Trp, Glu, Asp and Asn, more preferably from the side chains of Val, Cit, Ala, Glu and Lys. Alternatively worded, R¹⁷ is preferably selected from CH₃ (Ala), CH₂CH(CH₃)₂ (Leu), CH₂CH₂CH₂NHC(O)NH₂ (Cit), CH₂CH₂CH₂CH₂NH₂ (Lys), CH₂CH₂CH₂NHC(O)CH₃ (AcLys), CH₂CH₂CH₂NHC(=NH)NH₂ (Arg), CH₂Ph (Phe), CH(CH₃)₂ (Val), CH(CH₃)CH₂CH₃ (Ile), CH₂C(O)NH₂ (Asn), CH₂CH₂C(O)OH (Glu), CH₂C(O)OH (Asp) and CH₂(1*H*-indol-3-yl) (Trp). Especially preferred embodiments of R¹⁷ are CH₃ (Ala), CH₂CH₂CH₂NHC(O)NH₂ (Cit), CH₂CH₂CH₂CH₂NH₂ (Lys), CH₂CH₂C(O)OH (Glu) and CH(CH₃)₂ (Val). Most preferably, R¹⁷ is CH₃ (Ala), CH₂CH₂CH₂NHC(O)NH₂ (Cit), CH₂CH₂CH₂CH₂NH₂ (Lys), or CH(CH₃)₂ (Val).

In one embodiment, the amino acid side chain R¹⁷ is substituted with a polar group, preferably selected from oxo groups, (poly)ethylene glycol diamines, (poly)ethylene glycol or (poly)ethylene oxide chains, (poly)propylene glycol or (poly)propylene oxide chains, carboxylic acid groups, carbonate groups, carbamate groups, cyclodextrins, crown ethers, saccharides (e.g. monosaccharides, oligosaccharides), phosphates or esters thereof, phosphonic acid or ester, phosphinic acid or ester, sulfoxides, sulfones, sulfonic acid or ester, sulfinic acid, or sulfenic acid.

In an especially preferred embodiment, L² comprises the peptide spacer represented by general structure (**25**), preferably L² is represented by general structure (**25**):

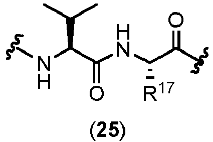

Herein, R¹⁷ is as defined above, preferably R¹⁷ is CH₃ (Ala) or CH₂CH₂CH₂NHC(O)NH₂ (Cit). The wavy lines indicate the connection to (L¹)ₙ and (L³)ₚ, preferably L² according to structure (**25**) is connected to (L¹)ₙ via NH and to (L³)ₚ via C(O).

L² may comprise a certain peptide sequence that is cleavable by specific enzymes. If those enzymes are exclusively expressed or overexpressed in the tumor microenvironment or in the endosomal/lysosomal compartment and leaked to the tumor microenvironment of cancer cells there is an increased probability of targeted release of the cytokine in the tumor and reduced release in healthy tissue.

### Linker L³

Linker L³ is a self-cleavable spacer, also referred to as self-immolative spacer. Linker L³ is either absent (p = 0) or present (p = 1). Preferably, linker L³ is present and p = 1. Cleavage of L² results in 1,6-β elimination in linker L³, resulting in decarboxylation and the release of the payload, D. This advantageously allows for increased probability of release of the payload in regions wherein enzymes that are able to cleave L² are overexpressed. In addition, release of a payload can induce bystander killing which is advantageous for tumours in which not all cancer cells have overexpression of the targeted receptor. Hence, in this embodiment, it is preferred that both linker L² and L³ are present (o = p = 1).

Preferably, L³ is para-aminobenzyloxycarbonyl (PABC) derivative, more preferably a PABC derivative according to structure (L3a):

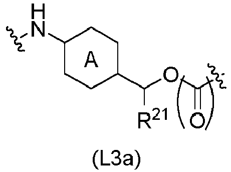

Herein, the wavy lines indicate the connection to L¹ or L², and to L⁴ or D. The brackets indicate an optional carbonyl group. Typically, the PABC derivative is connected via NH to L¹ or L², preferably to L², and via OC(O) to L⁴ or D.

Ring A is a 5- or 6-membered aromatic or heteroaromatic ring, preferably a 6-membered aromatic or heteroaromatic ring. Suitable 5-membered rings are oxazole, thiazole and furan. Suitable 6-membered rings are phenyl and pyridyl. Ring A may be substituted with a substituent selected from halogen, X²R⁴, N(R⁴)₂, C₁₋₄ alkyl and NO₂. Herein, X² and R⁴ are as defined above, including preferred embodiments thereof. In a preferred embodiment, the optional substituent is selected from F, Cl, Br, OH, OR⁴, SH, NH₂, Et, Me and NO₂. In an especially preferred embodiment, ring A comprises 0 - 2 substituents, more preferably 0 or 1 substituent, most preferably ring A is not substituted. In a preferred embodiment, ring A is 1,4-phenyl, 1,2-phenyl, 2,5-pyridyl or 3,6-pyridyl. Most preferably, A is 1,4-phenyl.

R²¹ is selected from H, R²⁶, C(O)OH and C(O)R²⁶, wherein R²⁶ is C₁ - C₂₄ (hetero)alkyl groups, C₃ -C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NR²⁸ wherein R²⁸ is independently selected from the group consisting of hydrogen and C₁ - C₄ alkyl groups. Preferably, R²⁶ is C₃ - C₁₀ (hetero)cycloalkyl or polyalkylene glycol. The polyalkylene glycol is preferably a polyethylene glycol or a polypropylene glycol, more preferably - (CH₂CH₂O)_{S}H or -(CH₂CH₂CH₂O)ₛH. The polyalkylene glycol is most preferably a polyethylene glycol, preferably -(CH₂CH₂O)ₛH, wherein s is an integer in the range 1 - 10, preferably 1 - 5, most preferably s = 1, 2, 3 or 4. More preferably, R²¹ is H or C(O)R²⁶, wherein R²⁶ = 4-methyl-piperazine or morpholine. Most preferably, R²¹ is H.

In an alternative embodiment, linker L³ is present (p = 1) and linker L² is not (o = 0) and L³ is a para-glucuronide-meta-amide-benzyloxycarbonyl derivative, preferably a glucuronide derivative according to structure (L3b):

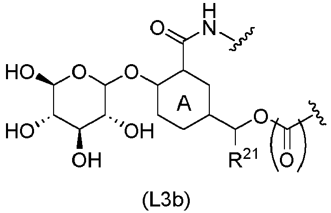

Herein, the wavy lines indicate the connection to L', and to L⁴ or D. The brackets indicate an optional carbonyl group. Typically, the glucuronide derivative is connected via NH to L', and via (O)CO to L⁴ or D. Ring A and R²¹ are defined as for the PABC derivative according to structure (L3a). Preferably, ring A a 6-membered aromatic or heteroaromatic ring, such as oxazole, thiazole, furan, phenyl and pyridyl. In a preferred embodiment, ring A is 1,3,4-phenyl, 2,4,5-pyridyl or 2,5,6-pyridyl. Most preferably, A is 1,3,4-phenyl. Preferably, R²¹ is H or C(O)R²⁶, wherein R²⁶ = 4-methyl-piperazine or morpholine. Most preferably, R²¹ is H.

Linker L³ according to structure (L3b) is cleavable by β-glucuronidase, similar to the mechanism in PABC, which results in self-immolation of the para-hydroxybenzyloxy group, decarboxylation and the release of the payload. An ADC comprising the glucuronide derivative according to structure (L3b) is especially useful for treating cancers having an overexpression of β-glucuronidase. β Glucuronidase concentrations in many solid tumours, including lung, breast, and gastrointestinal cancers, as well as in the tumour microenvironment, are reported to be higher than those in normal tissues, and the enzyme is not found in the general circulation. Thus, it is preferred that the conjugates according to the invention comprising the glucuronide derivative according to structure (L3b) are used to treat patients suffering from lung, breast, and gastrointestinal cancers

### Linker L⁴

Linker L⁴ is either absent (q=0) or present (q=1). Preferably, linker L⁴ is present and q = 1. Linker L⁴ is selected from:
- an aminoalkanoic acid spacer according to the structure - NR²²-(Cₓ-alkylene)-C(O)-, wherein x is an integer in the range 1 - 20 and R²² is H or C₁ - C₄ alkyl;
- an ethyleneglycol spacer according to the structure -NR²²-(CH₂-CH₂-O)ₑ₆-(CH₂)ₑ₇-C(O)-, wherein e6 is an integer in the range 1 - 10, e7 is an integer in the range 1 - 3 and R²² is H or C₁ - C₄ alky; and
- an amine spacer according to the structure NR²²-(SO₂NH-)ⱼ(C(O))ₕ-, wherein R²² is H or C₁ - C₄ alkyl, z is an integer in the range 1 - 10, j is 0 or 1, h is 0 or 1..

Linker L⁴ may be an aminoalkanoic acid spacer, i.e. -NR²²-(Cₓ-alkylene)-C(O)-, wherein x is an integer in the range 1 to 20, preferably 1 -10, most preferably 1 - 6. Herein, the aminoalkanoic acid spacer is typically connected to L³ via the nitrogen atom and to D via the carbonyl moiety. Preferred linkers L⁴ are selected from 6-aminohexanoic acid (Ahx, x = 5), β-alanine (x = 2) and glycine (Gly, x = 1), even more preferably 6-aminohexanoic acid or glycine. In one embodiment, L⁴ = 6-aminohexanoic acid. In one embodiment, L⁴ = glycine. Herein, R²² is H or C₁ - C₄ alkyl, preferably R²² is H or methyl, most preferably R²² is H.

Alternatively, linker L⁴ may be an ethyleneglycol spacer according to the structure -NR²²-(CH₂-CH₂-O)ₑ₆-(CH₂)ₑ₇-(C(O)-, wherein e6 is an integer in the range 1 -10, preferably e6 is in the range 2-6, and e7 is an integer in the range 1 - 3, preferably e7 is 2. Herein, R²² is H or C₁ - C₄ alkyl, preferably R²² is H or methyl, most preferably R²² is H.

Alternatively, linker L⁴ may be a diamine spacer according to the structure - NR²²-(Cₓ-alkylene)-NR²²-(C(O))ₕ-, wherein h is 0 or 1, x is an integer in the range 1 - 20, preferably an integer in the range 2
- 6, even more preferably x = 2 or 5, most preferably x = 2. R²² is H or C₁ - C₄ alkyl. Herein, R²² is H or C₁
- C₄ alkyl, preferably R²² is H or methyl, most preferably R²² is methyl. Herein, h is preferably 1, in which case linker L⁴ is especially suited for conjugation via a phenolic hydroxyl group present on payload D.

### Preferred Linkers

It is especially preferred that the linker L is selected from any one of the following structures:

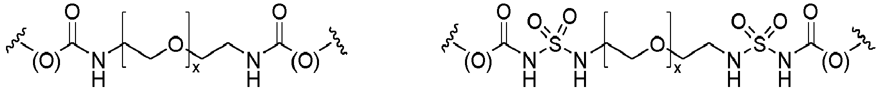

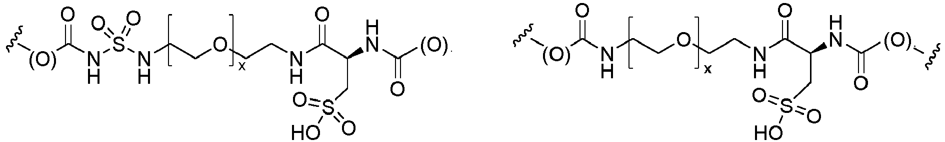

Herein it is preferred that x is in the range of 0-100, preferably x is in the range of 0-50, more preferably x is in the range of 1-30, most preferably x is in the range of 1-18.

In another preferred embodiment, α is preferably 0 and linker L is according to one of the structures below:

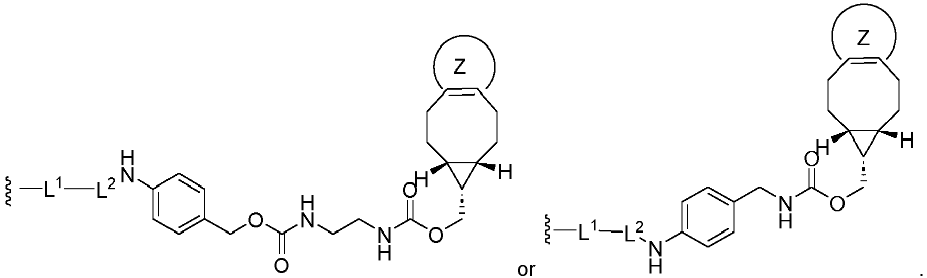

Herein L² is a peptide sequence that is a recognition site for a protease.

### Process for synthesising the conjugate according to general structure (1) or (2)

In a further aspect, the present invention relates a process for preparation of the conjugate according to the invention, the process comprising:
- reacting AB(F¹)ₓ, with Q¹-L-Q² or 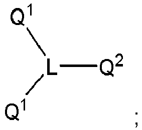
- reacting the resulting compound with F²-D; or the process comprises the following steps:
- reacting D-F² with Q¹-L-Q² or 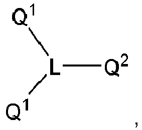
- reacting the resulting compound with AB(F¹)ₓ wherein AB(F¹)ₓ is a functionalized antibody containing x reactive moieties F¹ and x is an integer in the range of 1-10, wherein Q¹ and F¹ are mutually reactive towards each other and Q² and F² are mutually reactive towards each other, wherein the reactions between Q¹ and F¹ and between Q² and F² form a covalent linkage between the reactants.

In a preferred embodiment x = 2. The process according to this embodiment can be represented according schemes 1-4, preferably the process is according to scheme 2 or 4.

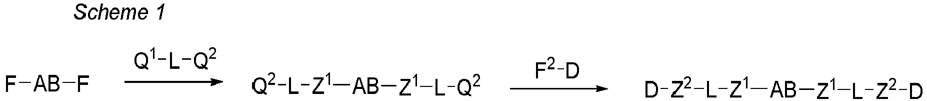

Herein, the immune cell-engaging polypeptide is represented by D. The conjugation reaction between reactive moieties Q¹ and F¹ afford the connecting group Z¹ and the conjugation reaction between reactive moieties Q² and F² afford the connecting group Z²

Alternatively, x=2 and the antibody-cytokine conjugate is obtained according to scheme 2.

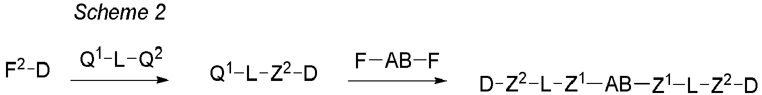

In yet another embodiment x=2, and the antibody-cytokine conjugate is obtained according to scheme 3.

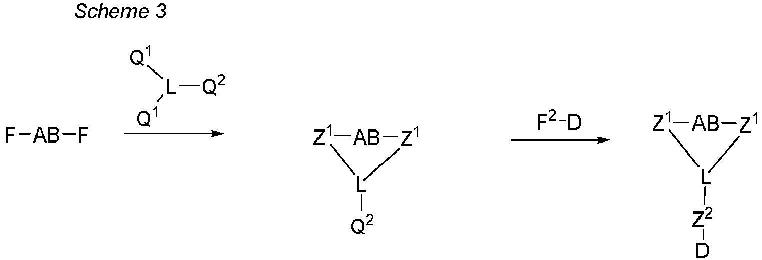

Alternatively, x=2 and the antibody-cytokine conjugate is obtained according to scheme 4.

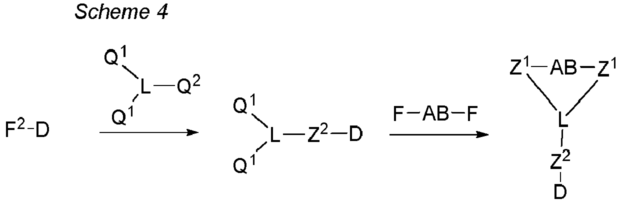

### Reactive moiety Q

Reactive moieties Q¹ and Q² are reactive to F¹ and F² respectively. In the context of the present invention, Q refers to Q¹ and Q². In the context of the present invention, the term "reactive moiety" may refer to a chemical moiety that comprises a reactive group, but also to a reactive group itself. For example, a cyclooctynyl group is a reactive group comprising a reactive group, namely a C-C triple bond. However, a reactive group, for example an azido reactive group, may herein also be referred to as a reactive moiety

Q is reactive towards and complementary to F. Herein, a reactive group is denoted as "complementary" to a reactive group when said reactive group reacts with said reactive group selectively, optionally in the presence of other functional groups. Complementary reactive moieties are known to a person skilled in the art, and are described in more detail below. The exact nature of Q, and F, depends on the type of reaction that is employed., the reaction is either a nucleophilic reaction, such as a Michael addition or nucleophilic substitution, or the reaction is a cycloaddition, such as a click reaction. The click probe is reactive in a cycloaddition (click reaction) and is preferably selected from an azide, a tetrazine, a triazine, a nitrone, a nitrile oxide, a nitrile imine, a diazo compound, an ortho-quinone, a dioxothiophene, a sydnone, an alkene moiety and an alkyne moiety. Preferably, click probe Q comprises or is an alkene moiety or an alkyne moiety, more preferably wherein the alkene is a (hetero)cycloalkene and/or the alkyne is a terminal alkyne or a (hetero)cycloalkyne.

In an especially preferred embodiment, Q comprises a cyclic (hetero)alkyne moiety. The alkynyl group may also be referred to as a (hetero)cycloalkynyl group, i.e. a heterocycloalkynyl group or a cycloalkynyl group, wherein the (hetero)cycloalkynyl group is optionally substituted. Preferably, the (hetero)cycloalkynyl group is a (hetero)cycloheptynyl group, a (hetero)cyclooctynyl group, a (hetero)cyclononynyl group or a (hetero)cyclodecynyl group. Herein, the (hetero)cycloalkynes may optionally be substituted. Preferably, the (hetero)cycloalkynyl group is an optionally substituted (hetero)cycloheptynyl group or an optionally substituted (hetero)cyclooctynyl group. Most preferably, the (hetero)cycloalkynyl group is a (hetero)cyclooctynyl group, wherein the (hetero)cyclooctynyl group is optionally substituted.

In an especially preferred embodiment, Q comprises a (hetero)cycloalkynyl or (hetero)cycloalkenyl group and is according to structure (Q1): 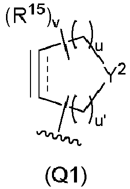 Herein:
- the bond depicted as - - - is a double bond or a triple bond;
- R¹⁵ is independently selected from the group consisting of hydrogen, halogen, -OR¹⁶, -NO₂, -CN, -S(O)₂R¹⁶, -S(O)₃⁽⁻⁾, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- Y² is C(R³¹)₂, O, S, S⁽⁺⁾R³¹, S(O)R³¹, S(O)=NR³¹ or NR³¹, wherein S⁽⁺⁾ is a cationic sulphur atom counterbalanced by B⁽⁻⁾, wherein B⁽⁻⁾ is an anion, and wherein each R³¹ individually is R¹⁵ or a connection with D, connected via L;
- u is 0, 1, 2, 3, 4 or 5;
- u' is 0, 1, 2, 3, 4 or 5, wherein u + u' = 0, 1, 2, 3, 4, 5, 6, 7 or 8;
v = an integer in the range 0-16;

Typically, v = (u + u') x 2 (when the connection to L, depicted by the wavy bond, is via Y²) or [(u + u') x 2] - 1 (when the connection to L, depicted by the wavy bond, is via one of the carbon atoms of u and u').

In a preferred embodiment of structure (Q1), reactive group Q comprises a (hetero)cycloalkynyl group and is according to structure (Q1 a): 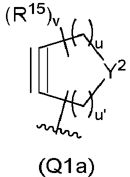 Herein,
- R¹⁵ and Y² are as defined above
- u is 0, 1, 2, 3, 4 or 5;
- u' is 0, 1, 2, 3, 4 or 5, wherein u + u' = 4, 5, 6, 7 or 8;
v = an integer in the range 8-16.

In a preferred embodiment, u + u' = 4, 5 or 6, more preferably u + u' = 5.

In a preferred embodiment, v = 8, 9 or 10, more preferably v = 9 or 10, most preferably v = 10.

In a preferred embodiment, Q is a (hetero)cycloalkynyl group selected from the group consisting of (Q2) - (Q20c) depicted here below.

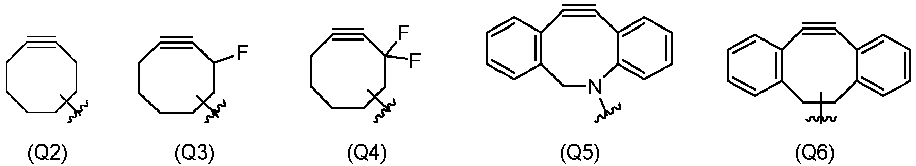

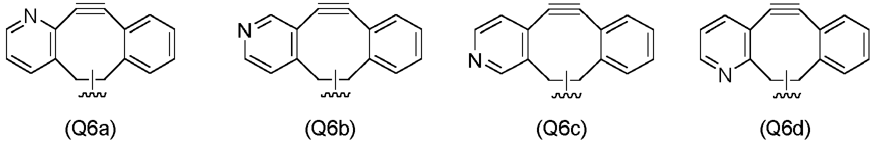

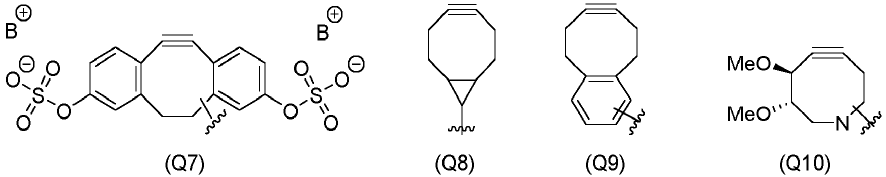

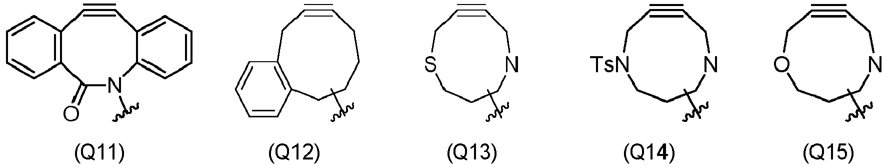

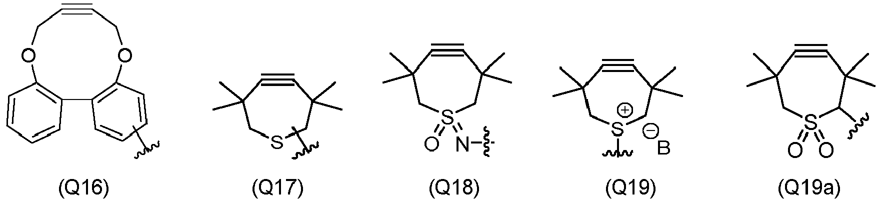

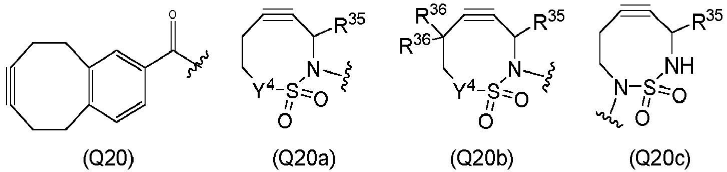

Herein, the connection to L, depicted with the wavy bond, may be to any available carbon or nitrogen atom of Q. The nitrogen atom of (Q10), (Q13), (Q14) and (Q15) may bear the connection to L, or may contain a hydrogen atom or be optionally functionalized. B⁽⁻⁾ is an anion, which is preferably selected from ⁽⁻⁾OTf, Cl⁽⁻⁾, Br⁽⁻⁾ or I⁽⁻⁾, most preferably B⁽⁻⁾ is ⁽⁻⁾OTf. B⁽⁺⁾ is a cation, preferably a pharmaceutically acceptable cation. In the conjugation reaction, B⁽⁻⁾ does not need to be a pharmaceutically acceptable anion, since B⁽⁻⁾ will exchange with the anions present in the reaction mixture anyway. In case (Q19) is used for Q, the negatively charged counter-ion is preferably pharmaceutically acceptable upon isolation of the conjugate according to the invention, such that the conjugate is readily useable as medicament. R³⁶ is an halogen selected from fluor, chlorine, bromine and iodine, preferably R³⁶ is fluor. Y⁴ is a heteroatom, preferably Y⁴ is O or NH. R³⁵ is selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, Si, S and NR¹⁴ wherein R¹⁴ is independently selected from the group consisting of hydrogen and C₁ - C₄ alkyl groups, preferably R³⁵ is selected from H, C₅H₁₁, CH₃, CH₂CH₃, CH₂OH or CH₂OTBS.

In a further preferred embodiment, Q is a (hetero)cycloalkynyl group selected from the group consisting of (Q21) - (Q38a) depicted here below.

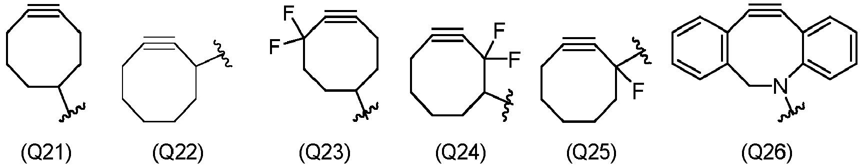

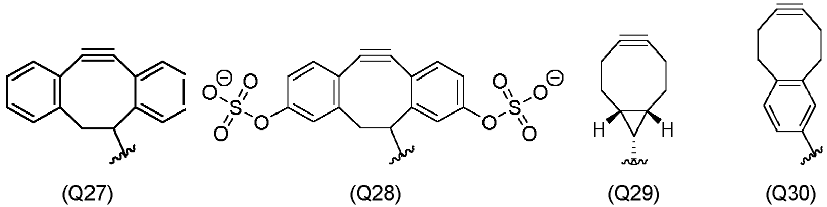

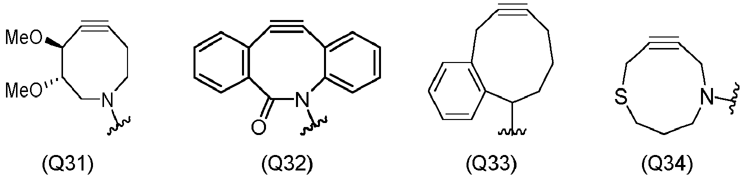

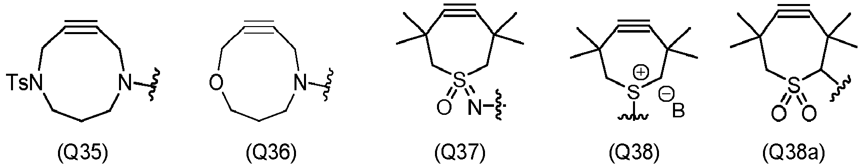

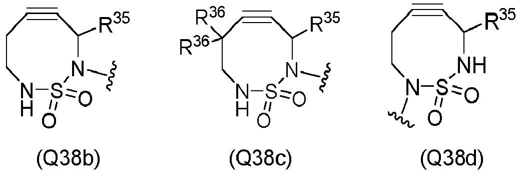

In structure (Q38), B⁽⁻⁾ is an anion, which is preferably selected from ⁽⁻⁾OTf, Cl⁽⁻⁾, Br⁽⁻⁾ or I⁽⁻⁾, most preferably B⁽⁻⁾ is ⁽⁻⁾OTf. In structure (Q28), B⁽⁺⁾ is a cation, preferably a pharmaceutically acceptable cation. Groups R³⁵ and R³⁶ on (Q38b), (Q38c) and (Q38d) are defined elsewhere and equally apply to the present embodiment.

In a preferred embodiment, Q comprises a (hetero)cyclooctyne moiety or a (hetero)cycloheptyne moiety, preferably according to structure (Q8), (Q26), (Q27), (Q28), (Q37) or (Q38a), which are optionally substituted. Each of these preferred options for Q are further defined here below.

Thus, in a preferred embodiment, Q comprises a heterocycloheptyne moiety according to structure (Q37), also referred to as a TMTHSI, which is optionally substituted. Preferably, the heterocycloheptyne moiety according to structure (Q37) is not substituted.

In an alternative preferred embodiment, Q comprises a cyclooctyne moiety according to structure (Q8), more preferably according to (Q29), also referred to as a bicyclo[6.1.0]non-4-yn-9-yl] group (BCN group), which is optionally substituted. Preferably, the cyclooctyne moiety according to structure (Q8) or (Q29) is not substituted. In the context of the present embodiment, Q preferably is a (hetero)cyclooctyne moiety according to structure (Q39) as shown below, wherein V is (CH₂)_{I} and I is an integer in the range of 0 to 10, preferably in the range of 0 to 6. More preferably, I is 0, 1, 2, 3 or 4, more preferably I is 0, 1 or 2 and most preferably I is 0 or 1. In the context of group (Q39), I is most preferably 1. Most preferably, Q is according to structure (Q42), defined further below.

In an alternative preferred embodiment, Q comprises a (hetero)cyclooctyne moiety according to structure (Q26), (Q27) or (Q28), also referred to as a DIBO, DIBAC, DBCO or ADIBO group, which are optionally substituted. In the context of the present embodiment, Q preferably is a (hetero)cyclooctyne moiety according to structure (Q40) or (Q41) as shown below, wherein Y¹ is O or NR¹¹, wherein R¹¹ is independently selected from the group consisting of hydrogen, a linear or branched C₁ - C₁₂ alkyl group or a C₄ - C₁₂ (hetero)aryl group. The aromatic rings in (Q40) are optionally O-sulfonylated at one or more positions, whereas the rings of (Q41) may be halogenated at one or more positions. Preferably, the (hetero)cyclooctyne moiety according to structure (Q40) or (Q41) is not further substituted. Most preferably, Q is according to structure (Q43), defined further below.

In an alternative preferred embodiment, Q comprises a heterocycloheptynylgroup and is according to structure (Q37).

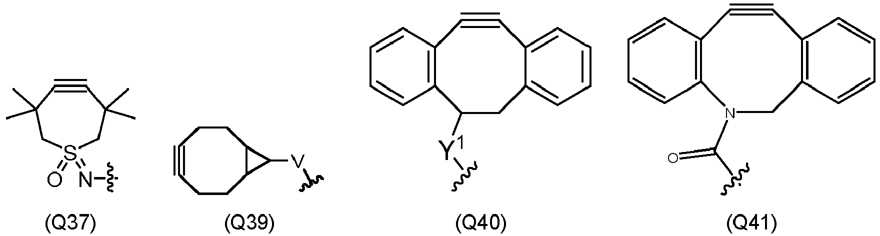

In an especially preferred embodiment, Q comprises a cyclooctynyl group and is according to structure (Q42): 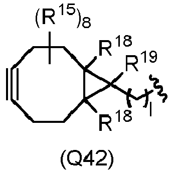 Herein:
- R¹⁵ is independently selected from the group consisting of hydrogen, halogen, -OR¹⁶, -NO₂, -CN, -S(O)₂R¹⁶, -S(O)₃⁽⁻⁾,C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;

- R¹⁸ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- R¹⁹ is selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted, or R¹⁹ is a second occurrence of Q or D connected via a spacer moiety; and
- I is an integer in the range 0 to 10.

In a preferred embodiment of the reactive group according to structure (Q42), R¹⁵ is independently selected from the group consisting of hydrogen, halogen, -OR¹⁶, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and C₁ - C₆ alkyl, most preferably all R¹⁵ are H. In a preferred embodiment of the reactive group according to structure (Q42), R¹⁸ is independently selected from the group consisting of hydrogen, C₁ - C₆ alkyl groups, most preferably both R¹⁸ are H. In a preferred embodiment of the reactive group according to structure (Q42), R¹⁹ is H. In a preferred embodiment of the reactive group according to structure (Q42), I is 0 or 1, more preferably I is 1.

In an especially preferred embodiment, Q comprises a (hetero)cyclooctynyl group and is according to structure (Q43): 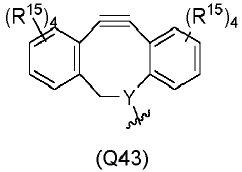 Herein:
- R¹⁵ is independently selected from the group consisting of hydrogen, halogen, -OR¹⁶, -NO₂, -CN, - S(O)₂R¹⁶, -S(O)₃⁽⁻⁾, C₁ - C₂₄alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- Y is N or CR¹⁵;
- a carbon atom in the fused aromatic rings may be replaced by a nitrogen atom, as in (Q6a) - (Q6d), preferably wherein Y is CR¹⁵.

In a preferred embodiment of the reactive group according to structure (Q43), R¹⁵ is independently selected from the group consisting of hydrogen, halogen, -OR¹⁶, -S(O)₃⁽⁻⁾, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and -S(O)₃⁽⁻⁾. In a preferred embodiment of the reactive group according to structure (Q43), Y is N or CH, more preferably Y = N.

In an alternative preferred embodiment, Q comprises a cyclic alkene moiety. The alkenyl group Q may also be referred to as a (hetero)cycloalkenyl group, i.e. a heterocycloalkenyl group or a cycloalkenyl group, preferably a cycloalkenyl group, wherein the (hetero)cycloalkenyl group is optionally substituted. Preferably, the (hetero)cycloalkenyl group is a (hetero)cyclopropenyl group, a (hetero)cyclobutenyl group, a norbornene group, a norbornadiene group, a *trans*-(hetero)cycloheptenyl group, a *trans-*(hetero)cyclooctenyl group, a *trans*-(hetero)cyclononenyl group or a *trans*-(hetero)cyclodecenyl group, which may all optionally be substituted. Especially preferred are (hetero)cyclopropenyl groups, *trans-*(hetero)cycloheptenyl group or *trans*-(hetero)cyclooctenyl groups, wherein the (hetero)cyclopropenyl group, the *trans*-(hetero)cycloheptenyl group or the *trans*-(hetero)cyclooctenyl group is optionally substituted. Preferably, Q comprises a cyclopropenyl moiety according to structure (Q44), a hetereocyclobutene moiety according to structure (Q45), a norbornene or norbornadiene group according to structure (Q46), a *trans*-(hetero)cycloheptenyl moiety according to structure (Q47) or a *trans-*(hetero)cyclooctenyl moiety according to structure (Q48). Herein, Y³ is selected from C(R²³)₂, NR²³ or O, wherein each R²³ is individually hydrogen, C₁ - C₆ alkyl or is connected to L, optionally via a spacer, and the bond labelled - - - is a single or double bond. In a further preferred embodiment, the cyclopropenyl group is according to structure (Q49). In another preferred embodiment, the *trans*-(hetero)cycloheptene group is according to structure (Q50) or (Q51). In another preferred embodiment, the *trans-*(hetero)cyclooctene group is according to structure (Q52), (Q53), (Q54), (Q55) or (Q56).

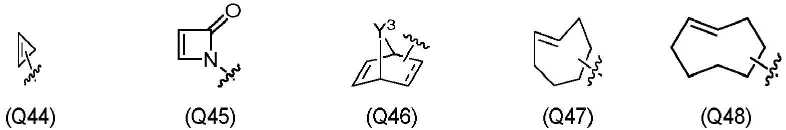

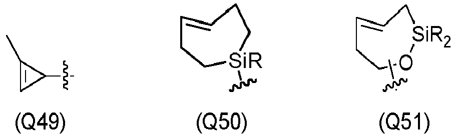

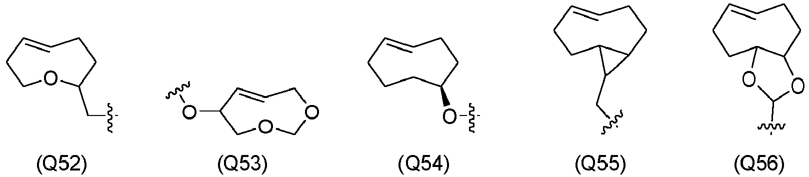

Herein, the R group(s) on Si in (Q50) and (Q51) are typically alkyl or aryl, preferably C₁-C₆ alkyl.

In an alternative preferred embodiment, Q is a thiol-reactive probe. In this embodiment, Q is a reactive group compatible with cysteine conjugation. Such probes are known in the art and may be selected from the group consisting of a maleimide moiety, a haloacetamide moiety, an allenamide moiety, a phosphonamidite moiety, a cyanoethynyl moiety, a vinylsulfone, a vinylpyridine moiety or a methylsulfonylphenyloxadiazole moiety. Most preferably, Q comprises a maleimide moiety. Reagents may be monoalkylation type or may be a cross-linker for reaction with two cysteine side-chains.

In a preferred embodiment, click probe QZ comprise a moiety selected from (Q1) - (Q56), more preferably is a moiety selected from (Q1) - (Q56).

### Reactive moiety F

Reactive moieties F¹ and F² are reactive towards Q¹ and Q² resspectively. In the context of the present invention, F refers to F¹ and F². F is reactive towards and complementary to Q. Herein, a reactive group is denoted as "complementary" to a reactive group when said reactive group reacts with said reactive group selectively, optionally in the presence of other functional groups. Complementary reactive click probes are known to a person skilled in the art, and are described in more detail below. The exact nature of Q, and F, depends on the type of click reaction that is employed. The click probe is reactive in a cycloaddition (click reaction) and is preferably selected from an azide, a tetrazine, a triazine, a nitrone, a nitrile oxide, a nitrile imine, a diazo compound, an *ortho*-quinone, a dioxothiophene, a sydnone, an alkene moiety and an alkyne moiety. Preferably, click probe F comprises or is an azide moiety or a tetrazine moiety.

F is reactive towards Q in the conjugation reaction defined below, preferably wherein the conjugation reaction is a cycloaddition or a nucleophilic reaction. As the skilled person will understand, the options for F are the same as those for Q, provided that F and Q are reactive towards each other. Thus, F preferably comprises a click probe, a thiol, a thiol-reactive moiety, an amine or an amine-reactive moiety, more preferably F is a click probe, a thiol or an amine, most preferably F is a click probe. The click probe is reactive in a cycloaddition (click reaction) and is preferably selected from an azide, a tetrazine, a triazine, a nitrone, a nitrile oxide, a nitrile imine, a diazo compound, an *ortho*-quinone, a dioxothiophene, a sydnone, an alkene moiety and an alkyne moiety. Preferably, the click probe comprises or is an azide, a tetrazine, a triazine, a nitrone, a nitrile oxide, a nitrile imine, a diazo compound, an *ortho*-quinone, a dioxothiophene or a sydnone, most preferably an azide. Typical thiol-reactive moieties are selected from maleimide moiety, a haloacetamide moiety, an allenamide moiety, a phosphonamidite moiety, a cyanoethynyl moiety, an *ortho-*quinone moiety, a vinylsulfone, a vinylpyridine moiety or a methylsulfonylphenyloxadiazole moiety. Most preferably, the thiol-reactive moiety comprises or is a maleimide moiety. Typical amine-reactive moieties are selected from N-hydroxysuccinimidyl esters, p-nitrophenyl carbonates, pentafluorophenyl carbonates, isocyanates, isothiocyanates and benzoyl halides. In a preferred embodiment, F is a click probe or a thiol, more preferably F is an azide or a thiol, most preferably F is an azide.

The reactive group F on the antibody are typically introduced by a specific technique, for example a (bio)chemical or a genetic technique. The reactive group that is placed in the antibody is prepared by chemical synthesis, for example an azide or a terminal alkyne. Methods of preparing modified antibodies are known in the art, e.g. from WO 2014/065661, WO 2016/170186 and WO 2016/053107, which are incorporated herein by reference. From the same documents, the conjugation reaction between the modified antibody and a linker-toxin-construct is known to the skilled person.

Preferably, F is a click probe reactive towards a (hetero)cycloalkene and/or a (hetero)cycloalkyne, and is typically selected from the group consisting of azide, tetrazine, triazine, nitrone, nitrile oxide, nitrile imine, diazo compound, ortho-quinone, dioxothiophene and sydnone. Preferred structures for the reactive group are structures (F1) - (F10) depicted here below.

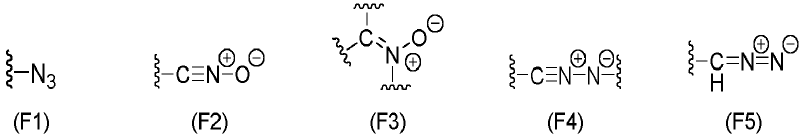

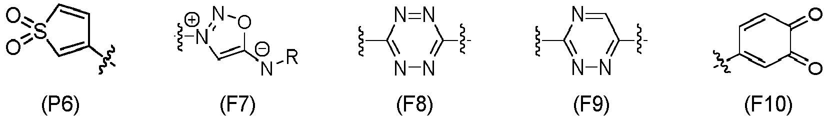

Herein, the wavy bond represents the connection to AB or D. For (F3), (F4), (F8) and (F9), the payload can be connected to any one of the wavy bonds. The other wavy bond may then be connected to an R group selected from hydrogen, C₁ - C₂₄ alkyl groups, C₂ - C₂₄ acyl groups, C₃ - C₂₄ cycloalkyl groups, C₂ - C₂₄ (hetero)aryl groups, C₃ - C₂₄ alkyl(hetero)aryl groups, C₃ - C₂₄ (hetero)arylalkyl groups and C₁ - C₂₄ sulfonyl groups, each of which (except hydrogen) may optionally be substituted and optionally interrupted by one or more heteroatoms selected from O, S and NR³² wherein R³² is independently selected from the group consisting of hydrogen and C₁ - C₄ alkyl groups. The skilled person understands which R groups may be applied for each of the groups F. For example, the R group connected to the nitrogen atom of (F3) may be selected from alkyl and aryl, and the R group connected to the carbon atom of (F3) may be selected from hydrogen, alkyl, aryl, acyl and sulfonyl. Preferably, the reactive moiety F is selected from azides, nitrones or tetrazines.

In an especially preferred embodiment, F is a tetrazine according to structure (F8a):

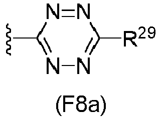

Herein, R²⁹ is selected from hydrogen, C₁₋₆ alkyl, aryl, C(O)-C₁₋₆ alkyl, C(O)-aryl, C(O)-O-C₁₋₆ alkyl, C(O)-O-aryl, C(O)-NR³³-C₁₋₆ alkyl and C(O)-NR³³-aryl, wherein R³³ is H or C₁₋₄ alkyl. Preferably, R²⁹ is selected from hydrogen, methyl, phenyl, pyridyl, pyridinyl and pyrimidinyl. It was found that R²⁹ is hydrogen gave optimal results in reactivity in the cycloaddition reaction. Thus, in a preferred embodiment, ring F, in particular F², is (F8a) wherein R²⁹ is selected from hydrogen, methyl, phenyl, pyridyl, pyridinyl and pyrimidinyl, more preferably R²⁹ is hydrogen.

In another especially preferred embodiment, F is a nitrone according to structure (F3a).

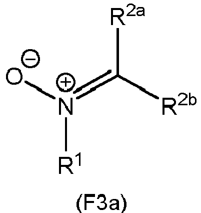

R¹ is L¹⁰XR⁴. Herein, X is a heteroatom having a lone pair which is capable of capturing the imine intermediate (d) by reacting with the imine carbon atom (see Scheme 3). In case this reaction forms a 5- or 6-membered ring, this capture of imine intermediate (d) is efficient and will stop the rearrangement reaction of Scheme 2. Hence, L¹⁰ should be a linker of two or three carbon atoms.

More specifically, L¹⁰ is a linker of structure (C(R³)₂)_{z}, wherein z is 2 or 3. Each R³ is individually selected from H and C₁₋₄ alkyl. Alternatively, two occurrences of R³ may be joined together to form an oxo group or a C₃₋₆ (hetero)cycloalkyl group. Preferably, L¹⁰ is a linker of structure CH₂-C(R³)₂ or CH₂-CH₂-C(R³)₂, more preferably L¹⁰ = CH₂-C(R³)₂. In case two occurrences of R³ are joined together to form a spiro-connected ring, it is preferred that such a ring is a C₃ - C₆ ring, preferably a C₄ or C₅ ring.

Preferred embodiments of L¹⁰ are (L¹⁰A) - (L¹⁰P):

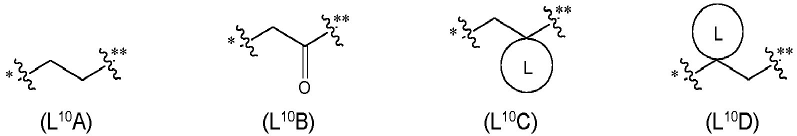

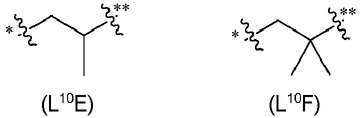

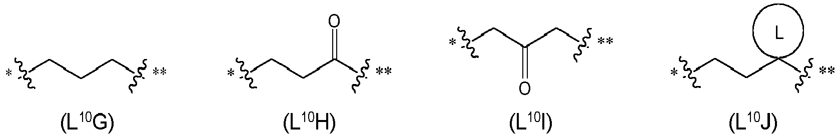

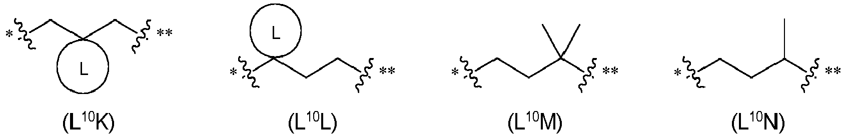

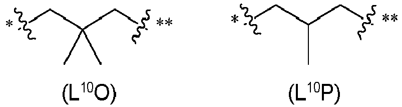

Herein, the wavy bond labelled with * is connected to the nitrogen atom of the nitrone group and the wavy bond labelled with * is connected to XR⁴. Ring (L) is spiro connected to the backbone atoms of L¹⁰. Ring (L) is preferably a cyclobutyl ring or a cyclopentyl ring, most preferably a cyclobutyl ring. Especially preferred are (L¹⁰A), (L¹⁰B), (L¹⁰C) and (L¹⁰G), wherein ring L is a cyclobutyl ring.

X is S, O or NH. Most preferably, X is O. R⁴ is selected from H and C₁₋₄ alkyl. Typically, R⁴ is H when X is O or NH, and R⁴ is H or C₁₋₄ alkyl when X is S. Thus, XR⁴ is typically selected from OH, NH₂, SH and S-C₁₋₄ alkyl. In a preferred embodiment, XR⁴ is SH, OH, NH₂, most preferably XR⁴ is OH.

The nature of R^{2a} and R^{2b} is not crucial for the present invention, and any suitable substituent for a nitrone compound can be used. R^{2a} and R^{2b} may be the same or different, typically they are different. In case R^{2a} and R^{2b} are different, the configuration of the double bound between the nitrogen atom and carbon atom of the nitrone group may either be in E-configuration of in Z-configuration. The exact configuration has no influence on the working of the present invention. Herein, the connection to AB or D is typically via R^{2a} or R^{2b}.

Typically, R^{2a} is selected from Hand C₁ - C₆ (cyclo)alkyl. In a preferred embodiment, R^{2a} is selected from H and C₁ **-** C₅ (cyclo)alkyl, more preferably R^{2a} is H, Me or Et, most preferably R^{2a} is H.

Typically, R^{2b} is selected from the group consisting of C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, which may optionally be substituted and which may optionally be interrupted by one or more heteroatoms selected from O, S and NR¹⁴, wherein R¹⁴ is independently selected from the group consisting of hydrogen and C₁ - C₄ alkyl groups. Alternatively, R^{2a} and R^{2b} are joined to form a (hetero)cyclic moiety. In an alternative embodiment, R^{2b} is L(D)ᵣ, wherein r is an integer in the range of 1 - 10, and L is a linker covalently connecting D with the nitrone group. In a further alternative embodiment, R^{2b} is L⁶AB, wherein L⁶ is a linker covalently connecting AB with the nitrone group. Preferred embodiments of antibody AB, payload D, linkers Land L⁶ and integer r are defined elsewhere.

In a preferred embodiment, R^{2b} is hydrogen, a C₁ - C₂₀ alkyl group, preferably a C₁-- C₁₆ alkyl group, more preferably a C₁ - C₁₀ alkyl group, L(D)ᵣ or L⁶AB. Herein, the alkyl group is optionally substituted and optionally interrupted by one or more heteroatoms selected from O, S and NR¹⁴, preferably O, wherein R¹⁴ is independently selected from the group consisting of hydrogen and C₁ - C₄ alkyl groups. In another preferred embodiment, R^{2b} is selected from the group consisting of hydrogen, methyl, ethyl, n-propyl, i-propyl, n-butyl, s-butyl, t-butyl, L(D)ᵣ or L⁶AB, more preferably from the group consisting of hydrogen, methyl, ethyl, n-propyl, i-propyl, L(D)ᵣ or L⁶AB, and even more preferably from the group consisting of hydrogen, methyl, ethyl, L(D)ᵣ or L⁶AB.

It is especially preferred that the nitrone compound according to the invention is used in the preparation of a bioconjugate, wherein antibody AB is covalently connected to a payload D. Hence, it is especially preferred that R^{2b} is L(D)ᵣ or L⁶AB. In one embodiment, R^{2b} is L(D)ᵣ and the nitrone compound is to be coupled with a (hetero)cycloalkyne compound comprising a antibody AB. In one embodiment, R^{2b} is L⁶B and the nitrone compound is to be coupled with a (hetero)cycloalkyne compound comprising a payload D. It is especially preferred that the nitrone is coupled to the payload and thus that R^{2b} is L(D)ᵣ.

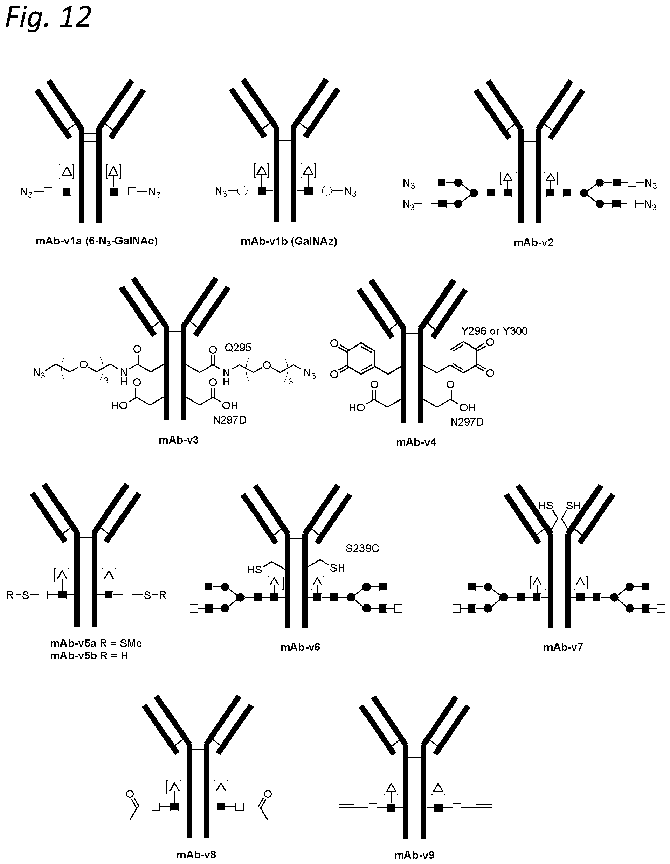

In a preferred embodiment, click probes F¹ and F² are selected from the group consisting of azide, tetrazine, triazine, nitrone, nitrile oxide, nitrile imine, diazo compound, dioxothiophene, sydnone, iminosydnone and catechol. Note that catechol in situ oxidized to an *ortho*-quinone group, which is reactive as click probe. Likewise, the term "tetrazine" also encompasses "hydrotetrazine", a known precursor that forms tetrazine upon in situ oxidation. Such precursors of click probes, which in situ form reactive groups, are also covered in the present invention. Figure 12 gives some known examples of in situ formed click probes by oxidation. In a preferred embodiment, F¹ is an azide and F² is iminosydnone, catechol, which forms a *ortho*-quinone group in situ, ortetrazine. More preferably, F² is iminosydnone according to structure (F7), catechol, which forms structure (F10) in situ, or tetrazine according to structure (F8). Most preferably, F¹ is an azide according to structure (F1) and F² is a tetrazine according to structure (F8a).

In the present invention, it is preferred that the exact structure of Q¹ and Q² differ, so that Q¹ is not reactive towards F², whereas Q² is reactive towards F².

In a preferred embodiment, F¹ is azide and Q¹ is an benzoannulated or tetramethylated (hetero)cycloalkyne, while F² is tetrazine and Q² is bicyclononyne. Herein, Q¹ is preferably according to structure (Q5), (Q6), (Q7), (Q11), (Q17), (Q18), (Q19) or (Q19a), more preferably according to structure (Q26), (Q27), (Q28), (Q32), (Q37), (Q38) or (Q38a), most preferably according to structure (Q40), (Q41) or (Q43). Herein, Q² is preferably according to structure (Q8), more preferably according to structure (Q29), most preferably according to structure (Q42).

In another preferred embodiment, F¹ is azide and Q² is a cycloalkyn, while Q² is trans-cyclooctene and F² is a nitrone or tetrazine.

In a preferred embodiment, D-F² is obtained by a ligase-mediated step, preferably the ligase is selected from tubulin tyrosine ligase, transglutaminase, lipoic acid ligase, farnesyl transferase, glycosyl transferase, formyl-glycine generating enzyme (FGE), trypsiligase/subtiligase, more preferably the ligase is sortase. Preferably, the sortase mediated step is as follows:

*D-LPX¹TG·X²ₙ ⁺ (G)_{n'}-F² → *D-LPX²T (G)_{n'}-F² ,

or

F²-LPX¹TG·X²ₙ ⁺ (G)_{n'}-D* → F²-LPX²T (G)_{n'}-D

Herein, D* is the remaining part of D, L is leucine, P is proline, T is threonine, G is Glycine. X¹ is any amino acid, X² is any hydrophobic amino acid and n is an integer in the range of 1 -20, preferably n is 3-15. F² may comprise a PEG chain. More preferably, LPX¹TGX²ₙ is a LPETGGH₁₀ and n' is 3. In an especially preferred embodiment, F² is an azide and the conjugation proceeds via a SPAAC reaction or F² is a tetrazine and the conjugation proceeds via an inverse-demand Diels-Alder reaction.

In another preferred embodiment, F is a nitrone and D-F² is obtained by converting a terminal serine or threonine into a nitrone. The conversion into a nitrone can be realised by oxidizing the serine or threonine side chain and then converting into a nitrone as shown below:

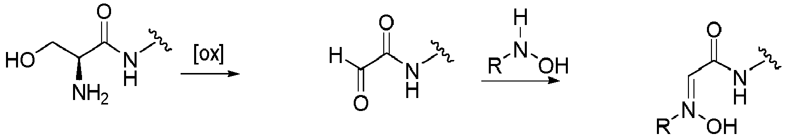

Such conversion of serine or threonine into a nitrone can be performed by conventional chemical conversions. Herein, [ox] is an oxidizing agent, such as NalO₄. R corresponds to R¹ as defined above for (F3a). Its exact nature is not relevant in the context of the present invention, although it is preferably defined as R¹ above. The thus obtained conjugates have improved stability, since they may rearrange into stable conjugates as explained above.

It is especially preferred that the oxidation of the terminal amino acid, conversion into a nitrone and the conjugation to the linker are performed in one pot synthesis. Preferably, the Q² is trans-cyclooctene, or Q² is a cyclo-alkyne and the conjugation reaction is a strain-promoted alkyne-nitrone cycloaddition (SPANC).

### Application

The conjugates of the present invention are suitable for medical treatment, the conjugates are especially suitable in the treatment of cancer or an autoimmune disease. Alternatively, the conjugates of the present invention are used for the treatment of blood disorders, such as neutropenia, anemia and thrombocytopenia. Thus, in a preferred embodiment, the invention further concerns a method for the treatment of cancer comprising administering to a subject in need thereof the conjugate according to the invention. The subject in need thereof is typically a cancer patient. The use of conjugates, such as antibody-drug conjugates, is well-known in the field of cancer treatment, and the conjugates according to the invention are especially suited in this respect. The method as described is typically suited for the treatment of cancer. In the method according to this aspect, the antibody-conjugate is typically administered in a therapeutically effective dose. A preferred dose for administration of the conjugates according to the invention is in the range of 3 - 20 mg per kg bodyweight, every three weeks, or every two weeks, or every week, preferably every three weeks. The present aspect of the invention can also be worded as a conjugate according to the invention for use in the treatment of cancer. In other words, this aspect concerns the use of a conjugate according to the invention for the preparation of a medicament or pharmaceutical composition for use in the treatment of cancer. In the present context, treatment of cancer is envisioned to encompass treating, imaging, diagnosing, preventing the proliferation of, containing and reducing tumours.

This aspect of the present invention may also be worded as a method for targeting a tumour cell expressing a specific extracellular receptor, comprising contacting the conjugate according to the invention with cells that may possibly express the extracellular receptor, and wherein the antibody specifically targets the extracellular receptor. These tumour cells expressing the extracellular receptor may be present in a subject, in which case the method comprises administering to a subject in need thereof the conjugate according to the invention. Alternatively, the method occurs ex *vivo* or *in vitro.* In a preferred embodiment, the cells that may possibly express the extracellular receptor are cells that express the extracellular receptor. The targeting of tumour cells preferably includes one or more of treating, preventing the proliferation of, containing and reducing the tumour cells.

In the context of diagnosis, it is typically unknown whether the cells that are being contacted in fact express the specific extracellular receptorthat is being investigated. For example, in the diagnosis of HER2-positive breast cancer, a conjugate containing an antibody that targets HER2, such as trastuzumab, may be contacted with the cells. In case the tumour cells are in fact HER2-expressing, the conjugate will target the cells, while in case the tumour cells are not HER2-expressing, the conjugate will not target the cells. Likewise, in the treatment of a cancer cells specifically expressing an extracellular receptor, the skilled person will understand that a cell-binding agent, such as an antibody, is to be used that targets that specific extracellular receptor.

In the methods of the present invention, it is preferred that the extracellular receptor is selected from the group consisting of 5T4 (TPBG), ADAM9, ALPP, ALPPL2, AMHRII, ASCT2 (SLC1A5), ASLG659, ASPHD1, av-integrin, avb3-integrin/ITGAV/CD51, Axl, B7-H3 (CD276), B7-H4 (VTCN1), BAFF-R, BCMA (CD269), BMPR1B, Brevican, c-KIT (CD117), c-Met, C4.4a (LYPD3), CA-IX (CA9)/MN, Cadherin-6, CanAg, CCR7, CD117 (c-KIT), CD123 (IL-3Rα), CD13, CD133, CD138/syndecan-1, CD166 (ALCAM), CD19, CD20, CD203C, CD205, Ly75, CD21, CD22, CD228 (P79, SEMF), CD25 (IL-2R-α), CD30 (TNFRSF8), CD324 (CDH1/E-cadherin), CD33, CD352 (SLAMF6, NTB-A), CD37, CD38, CD44v6, CD45, CD46, CD47, CD48a (SLAMF2), CD56 (NCAM), CD70, CD71 (TF-R), CD72, CD74, CD79a, CD79b, CDH6, Cadherin-6, CEACAM5 (CD66e), claudin, CLDN18.2, CLDN6 (claudin-6, Skullin), CLEC12A, CLL-1/CLEC12A, Cripto, CS1 (SLAMF7, CD319), CXCR5, DKL-1, DLL3 (delta-like 3), DPEP3, E16, EGFR, EGFRvlll, ENPP3, CD203c (AGS-16), EpCAM, EphA2, EphB2R, Ephrin-A4 (EFNA4), ETBR, FAP, FGFR2, FGFR3, fibronectin EDB , FLT3, FOLR1 (FR-a), Gal-3BP, GD3, GDNF-Ra1, GEDA, GFRA1, Globo H, GPC3 (glypican-3), gpNMB, GPR172A, GPR19, GPR54, GRP20, guanyl cyclase C (GCC), HER2, HER3, HLA-DOB, IGF-1R, IL13R, IL20Ra, Integrin avb6, KAAG-1, LAMP-1 (CD107a), Lewis Y (CD174), LGR5, LIV-1 (SLC39A6, ZIP6), LRRC15, LY64, Ly6E (lymph. antig 6), Ly6G6D, LY6K, mesothelin (MSLN), MFI2 (TAA), MICA/B, MOSPD2, MPF, MUC1 (CA6), MUC16/CA-125 , MUC1c, NaPi2b (SLC34A2), NCA, Nectin-4 (PVRL4), Notch3, P-cadherin (pCAD, CDH3), P2X5, PD-L1 (CD274, B7-H1), PMEL17, PRLR (prolactin), PSCA, PSMA, PTK7 (CCK4), RET, RNF43, RON, ROR1, ROR2, Serna 5b, SEZ6, SLITRK6 (SLC44A4), STEAP1, STEAP2, STn, TAG72, TENB2, TF (CD142, thromoplastin), TIM-1, TM4SF, TMEFF, TMEM118, TMEM46, TNF-alpha, TROP-2 (TACSTD2), TWEAKR, receptor tyrosine kinases (RTK), tenascin, or antibody is specific for an extracellular protein resulting from a viral infection and/or for a tumour-associated carbohydrate antigen (TACA). Likewise, it is preferred that the tumour cells express an extracellular receptor selected from the same group. The skilled person is capable of matching the desired extracellular receptor with a suitable cell-binding agent capable of targeting that extracellular receptor.

The conjugates of the present invention are also especially suitable as antibiotic, antiviral, antiinflammatory and anti-autoimmune agent. Thus, in an alternative embodiment, the invention concerns a method for the treatment of infection, inflammation of an autoimmune disorder, comprising administering to a subject in need thereof the conjugate according to the invention. In the method according to this aspect, the antibody-conjugate is typically administered in a therapeutically effective dose. The present aspect of the invention can also be worded as a conjugate according to the invention for use in the treatment of infection, inflammation of an autoimmune disorder. In other words, this aspect concerns the use of a conjugate according to the invention for the preparation of a medicament or pharmaceutical composition for use in the treatment of infection, inflammation of an autoimmune disorder. Herein, the infection may be bacterial or viral.

In the present embodiment, the antibody is preferably specific to an extracellular protein resulting from a viral infection and/or tumour-associated carbohydrate antigen. Herein, the extracellular protein resulting from a viral infection may be human polio virus (HPV), human cytomegalovirus (HCMV) or human papillomavirus (HPV). The tumour-associated carbohydrate antigen (TACA) may be selected from the group of Tn, STn, T-antigen, LDN, Lewis^{c} (Le^{c}), Sialyl-Lewis^{c} (SLe^{c}), 6-Sialyl-Lewis^{c} (6SLe^{c}), LN, alpha-Gal, 3SLN, 6SLN, H-antigen, A-antigen, B-antigen, Lewis^{a} (Le"), Sialyl-Lewis^{a} (SLe^{a}), 6-Sialyl-Lewis^{a} (6SLe^{a}), Lewis^{b} (Le^{b}), Sialyl-Lewis^{b} (SLe^{b}), 6-Sialyl-Lewis^{b} (6SLe^{b}), Lewis^{x} (Le^{x}), Sialyl-Lewis^{x} (SLe^{x}), 6-Sialyl-Lewis^{x} (6SLe^{x}), Lewis^{y} (Le^{y}), Sialyl-Lewis^{y} (SLe^{y}), 6-Sialyl-Lewis^{y} (6SLe^{y}) and or combinations thereof. The antibody may also be specific to both an extracellular protein and a TACA at the same time.

In a preferred embodiment, the present invention concerns a conjugate for use in the treatment of an autoimmune disorder. The skilled person understands which combination of target and payload is most suitable for a certain autoimmune disease. Cytokines may have inflammatory activities, such as IL-17 and interferon-γ or have a regulatory effect in inflammation such as IL-10 and TGF-β. It is preferred in the treatment of autoimmune diseases that the conjugate of the present invention comprises a regulatory cytokine, more preferably the cytokine is selected from IL-4, IL-6, IL-9, IL-10, IL-11, and IL-13, IFN-α and TGF-β. Alternatively the conjugate comprises an antibody that specifically targets the abnormal cells that cause the auto-immune disease, herein it is preferred that the cytokine promotes inflammation, herein the payload is preferably selected from IL-1, IL-2, IL-6, IL-12, IL-15 IL-17, IL-18, IFN-y, TNF-α and GM-CFS

In this light, the invention also concerns a pharmaceutical composition comprising the conjugate according to the invention and a pharmaceutically acceptable carrier. The pharmaceutical composition typically contains the conjugate according to the invention in a pharmaceutically effective dose.

### Description of the figures

Figure 1 shows a representative (but not comprehensive) set of functional groups (F) in a biomolecule, either naturally present or introduced by engineering, which upon reaction with a reactive group lead to connecting group Z. Functional group F may be artificially introduced (engineered) into a biomolecule at any position of choice. The pyridazine connecting group (bottom line) is the product of the rearrangement of the tetrazabicyclo[2.2.2]octane connecting group, formed upon reaction of tetrazine with alkyne, with loss of N₂. Connecting groups Z of structure **(10a)** - **(10j)** are preferred connecting groups to be used in the present invention.

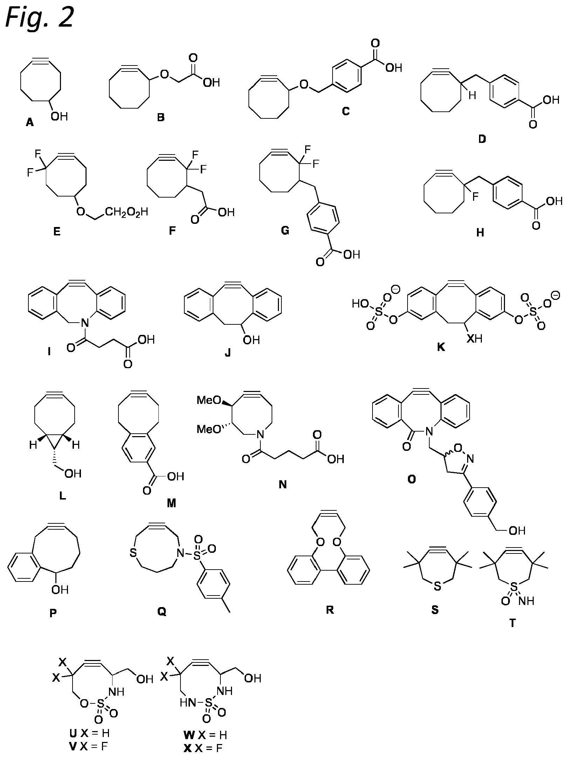

Figure 2 shows cyclooctynes suitable for metal-free click chemistry, and preferred embodiments for reactive moiety Q. The list is not comprehensive, for example alkynes can be further activated by fluorination, by substitution of the aromatic rings or by introduction of heteroatoms in the aromatic ring.
Figure 3 shows several structures of derivatives of UDP sugars of galactosamine, which may be modified with e.g. a 3-mercaptopropionyl group (**11a**)**,** an azidoacetylgroup (**11b**), or an azidodifluoroacetyl group (**11c**) at the 2-position, or with an azido group at the 6-position of N-acetyl galactosamine (**11d**) or with a thiol group at the 6-position of N-acetyl galactosamine (**11e**). The monosaccharide (i.e. with UDP removed) are preferred moieties Su to be used in the present invention.
Figure 4 shows the general process for non-genetic conversion of a monoclonal antibody into an antibody containing probes for click conjugation (F). The click probe may be on various positions in the antibody, depending on the technology employed. For example, the antibody may be converted into an antibody containing two click probes (structure on the left) or four click probes (bottom structure) or eight probes (structure on the right) for click conjugation.

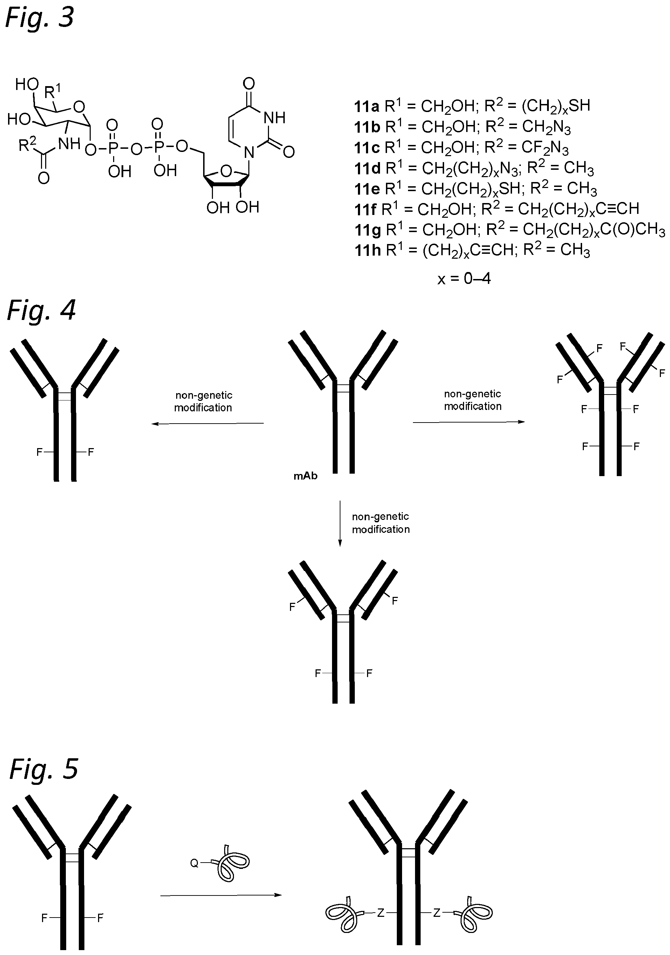

Figure 5 depicts how an IgG antibody modified with two click probes (F) can react with a polypeptide modified with the complementary click probe (Q) to form a stable bond (Q) upon reaction, where the polypeptide is elected from any polypeptide that is able to bind to an immune cell, thereby forming a bispecific antibody. Modification of the polypeptide with a single click probe Q may be achieved by any selective genetic or non-genetic method. Probes for click conjugation may be elected from any suitable combination depicted in Figure 1. Stoichiometry of the resulting bispecific antibody depends on the number of click probes F installed in the first modification of the antibody. A symmetrical, bivalent IgG may be employed (CDR1 = CDR2), thus leading to a bispecific antibody with a 2:2 molecular format (2x attachment of polypeptide). A non-symmetrical antibody may also be employed (CDR1 # CDR2), thus leading to a trispecific antibody with a 1:1:2 molecular format. If more than 2 click probes F are installed, the molecular format may be further varied, leading to for example a 2:4 molecular format (4x F installed on a symmetrical antibody) or 1:1:8 molecular format (8x F installed on a non-symmetrical antibody).

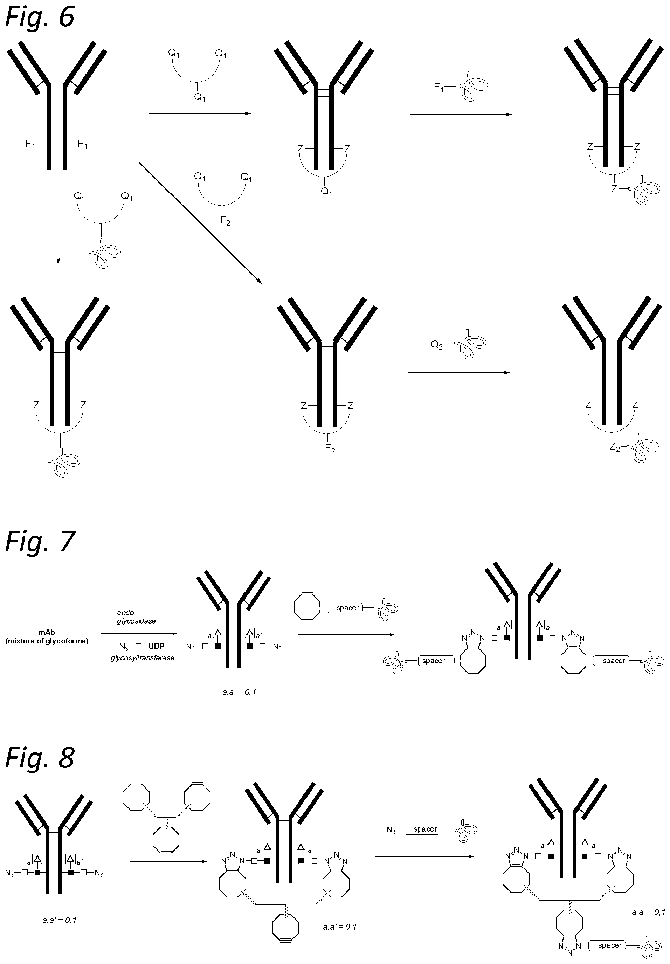

Figure 6 shows three alternative methods to install a single immune cell-engaging polypeptide onto a full-length antibody (2:1 molecular format). The full-length antibody therefore has has first been modified with two click probes F. In one approach (arrow down), the IgG(F₂) is subjected to a polypeptide that has been modified with two complementary click probes Q, connected via a suitable spacer, both of which will react with one occurrence of F on the antibody. In the second approach (arrow right), the IgG(F₂) is subjected to a trivalent construct containing three complementary probes Q of which two will react with IgG(F₂), leaving one unit of Q free for subsequent reaction with F-modified polypeptide. In the third approach (diagonal arrow), the IgG(F₂) is subjected to a trivalent construct containing two complementary probes Q and one non-reactive click probe F² (which is also different from F). The two click probes Q will react with IgG(F₂), leaving F² for subsequent reaction with Q₂-modified polypeptide.
Figure 7 depicts a specific example of forming a bispecific antibody of 2:2 molecular format based on glycan remodeling of a full-length IgG and azide-cyclooctyne click chemistry. The IgG is first enzymatically remodeled by endoglycosidase-mediated trimming of all different glycoforms, followed by glycosyltransferase-mediated transfer of azido-sugar onto the core GlcNAc liberated by endoglycosidase. In the next step, the azido-remodeled IgG is subjected to an immune cell-engaging polypeptide, which has been modified with a single cyclooctyne for metal-free click chemistry (SPAAC), leading to a bispecific antibody of 2:2 molecular format. It is also depicted that the cyclooctyne-polypeptide construct will have a specific spacer between cyclooctyne and polypeptide, which enables tailoring of IgG-polypeptide distance or impart other properties onto the resulting bispecific antibody.
Figure 8 is an illustration of how a azido-sugar remodeled antibody can be converted into a bispecific with a 2:1 molecular format by subjecting first to trivalent cyclooctyne construct suitable for clipping onto bis-azido antibody, leaving one cyclooctyne free for subsequent SPAAC with azido-modified polypeptide, effectively installing only one polypeptide onto the IgG. The latter polypeptide may also be modified with other complement click probes for reaction with cyclooctyne, e.g. a tetrazine moiety for inverse electron-demand Diels-Alder cycloaddition. Any combinations of F and Q (Figure 1) can be envisaged here.

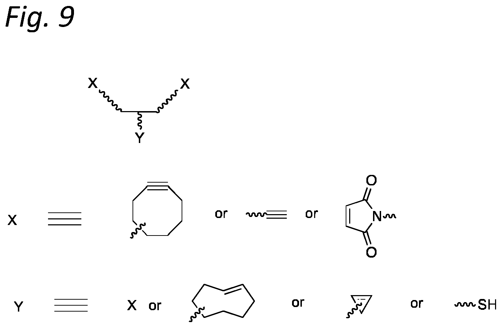

Figure 9 shows various options for trivalent constructs for reaction with a bis-azidosugar modified mAb. The trivalent construct may be homotrivalent or heterotrivalent (2+1 format). A homotrivalent contract (X = Y) may consist of 3x cyclooctyne or 3x acetylene or 3x maleimide or 3x other thiol-reactive group. A heterotrivalent construct (X # Y) may for example consist of two cyclooctyne groups and one maleimide group or two maleimides groups and one trans-cyclooctene group. The heterotrivalent construct may exist of any combination of X and Y unless X and Y and reactive with each other (e.g. maleimide + thiol).

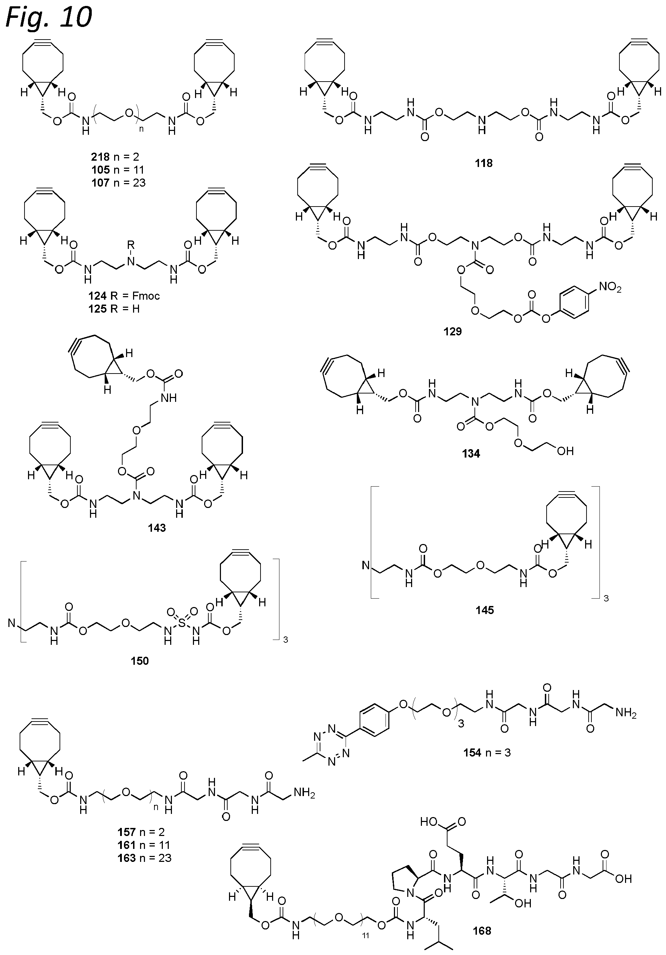

Figure 10 shows a range of bivalent BCN reagents **(105, 107, 118, 125, 129, 134),** trivalent BCN reagents **(143, 145, 150),** and monovalent BCN reagents for sortagging **(154, 157, 161, 163, 168).**

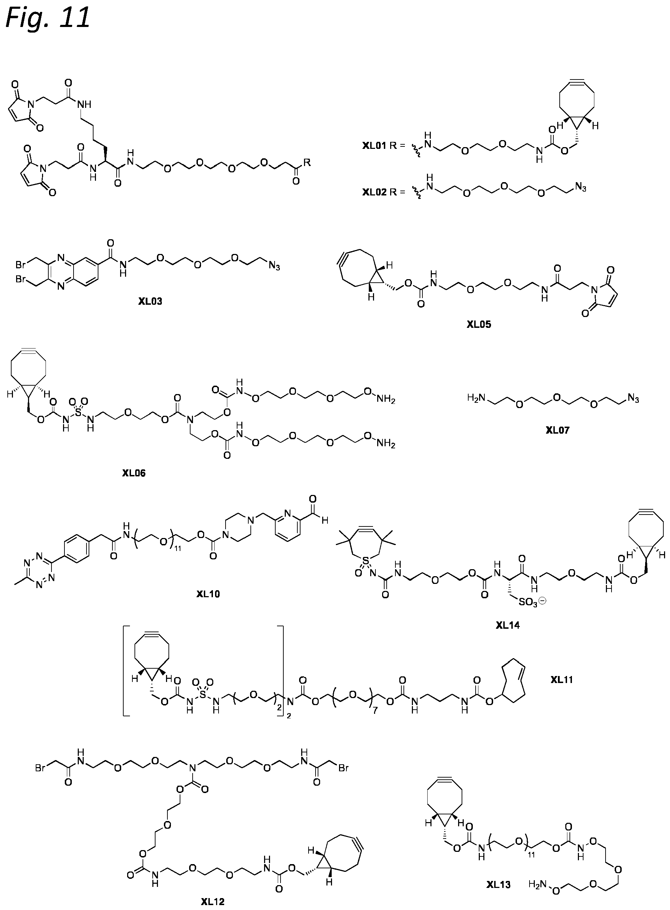

Figure 11 shows a range of bivalent or trivalent cross-linkers **(XL01-XL13).**
Figure 12 shows a range of antibody variants as starting materials for subsequent conversion to antibody conjugates

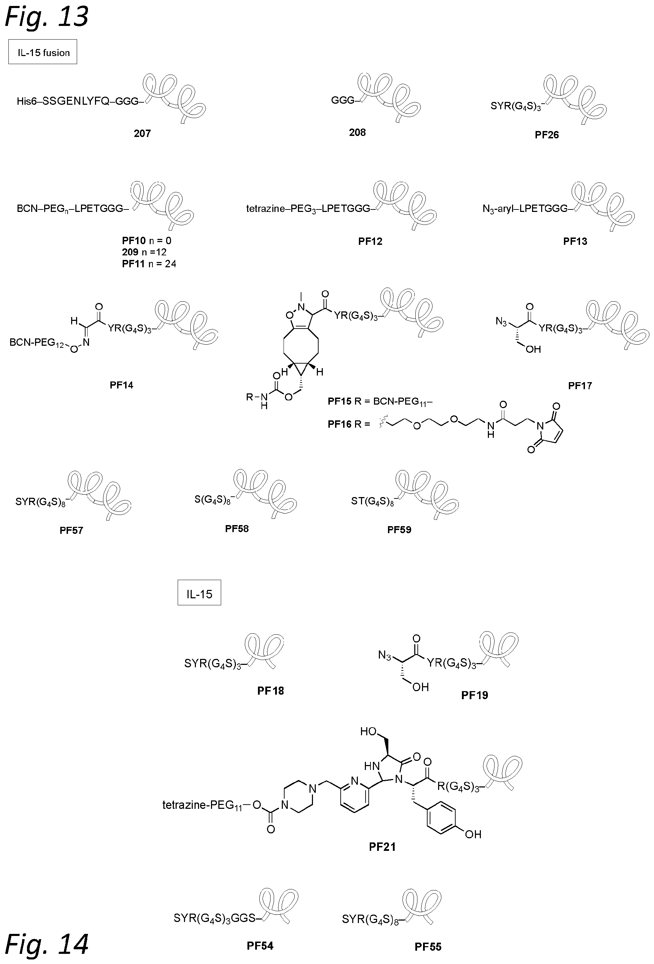

Figure 13 shows structures of various mutants of IL-15 **(PF18)** or IL-15R-IL-15 fusion protein **(207, 208, PF26 and PF57-PF59,** IL-15R = Sushi domain of IL-15 receptor) and derivatives thereof equipped with a suitable click probe (BCN, tetrazine or azide) or maleimide, in each case modified at its N-terminus to enable site-specific modification.
Figure 14 shows structures of IL-15 variants with non-cleavable spacers **(PF54** and **PF55).**

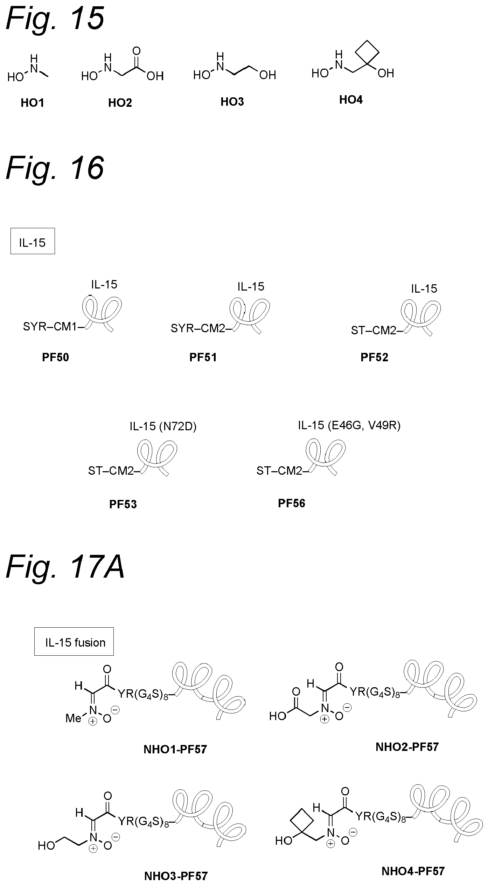

Figure 15 shows structures of hydroxylamines **HO1-HO4.**
Figure 16 shows structures of IL-15 variants with different N-terminal sequence tags **(PF50-52)** and IL-15 mutants **(PF53** and **PF56)**
Figure 17 shows structures of various nitrone-variants of IL-15R-IL-15 fusion protein **(PF57)** obtained by incorporation of hydroxylamine **HO1-HO4** and various nitrone-variants of various IL-15 mutants **(PF18, PF50, PF51, PF53** and **PF56)** obtained by incorporation of hydroxylamine **HO1** and **HO2.**
Figure 18 shows the SDS-page analysis under reducing conditions for various antibody-cytokine conjugates based on mPD-L1 and IL-15 variants **PF51-PF54** and **FPF56.**
Figure 19 shows RP-UPLC analysis under nonreducing conditions showing formation of the 2:1 format for trastuzumab and IL-15 variants with different N-terminal sequences.
Figure 20 shows RP-UPLC analysis under nonreducing conditions showing formation of the 2:1 format for trastuzumab and IL-15 variants modified with hydroxylamines **HO2-HO4**.
Figure 21 shows aggregation of antibody-cytokine conjugates after 0-5 freeze-thaw cycles measured by SE-HPLC.
Figure 22 shows cleavage of **mPD-L1-v1a-218-NHO2-PF52 (2:2)** by matriptase.
Figure 23 shows cleavage of various antibody-cytokine conjugates by matriptase.
Figure 24 shows in vitro IL-15 activity assay of various intact antibody-cytokine conjugates. **mPD-L1-v1a-218-NHO2-PF52 (2:2)** treated with matriptase was included as a reference.
Figure 25 shows in vitro IL-15 activity assay of various matriptase-treated antibody-cytokine conjugates.
Figure 26 shows in vitro IL-15 activity of **mPD-L1-v1a-218-NHO2-PF52 (2:2)** and mPD-L1-v1a-**218-NHO2-PF56 (2:2)** with and without pre-treatmetn with matriptase MT-SP1. Free PF52 was included as a reference.
Figure 27 shows in vitro IL-15 activity of **tras-v1a-145-NHO1-PF50 (2:1)** with and without pretreatment with urokinase. Free **PF50** and trastuzumab were included as a reference.
Figure 28 shows the in vivo tolerability of **mPD-L1-v1a-218-NHO1-PF51, mPD-L1-v1a-218-NHO2-PF52, mPD-L1-v1a-218-NHO2-PF54** and **IC1** in female BALB/c mice.). Mice were given a single dose s.c. of 6 mg/kg (top graph), 12 mg/kg (middle graph) and 18 mg/kg (bottom graph).
Figure 29 shows the in vivo tolerability of antibody-cytokine conjugates in female BALB/c mice. ). Conjugates consist of different IL-15 mutants and different molecular formats (2:2 and 2:1). Mice were given a single dose s.c. at the indicated dose levels.
Figure 30 shows the in vivo efficacy of in a mouse CT26 syngeneic colon cancer model. Antibody-cytokine conjugates **mPD-L1-v1a-218-NHO1-PF51, mPD-L1-v1a-218-NHO2-PF52, mPD-L1-v1a-218-NHO2-PF54** and **IC1** were dosed at 6 mg/kg on day 0 and day 7 and mice were monitored for tumor volume. Data are plotted as mean values +/- standard error of the mean (top graph) and as median tumor volume (bottom graph).
Figure 31 shows the in vivo efficacy of in a mouse CT26 syngeneic colon cancer model comparing antibody-cytokine conjugates based on different IL-15 mutants and different molecular formats (2:2 and 2:1). Immunocytokines were dosed in all cases on day 0 and day 7 with a dose level of either 3 or 6 mg/kg as indicated. Mice were monitored for tumor volume. Data are plotted as mean values +/- standard error of the mean (top graph) and as median tumor volume (bottom graph).

### Examples

The invention is illustrated by the following examples.

### Synthesis of hydroxylamine compounds

### Example 1. Synthesis of compound HO2

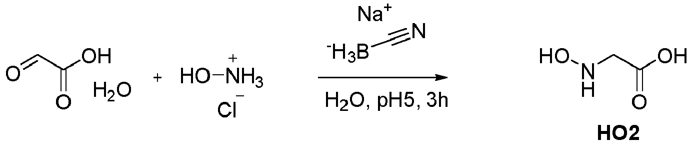

2-oxoacetic acid hydrate (1.24 g, 1 Eq, 13.5 mmol) and hydroxylamine hydrochloride (936 mg, 1 Eq, 13.5 mmol) were stirred in water (60 mL) at room temperature. 1M NaOH was added to raise the pH to 5 before addition of sodium cyanoborohydride (2.12 g, 2.5 Eq, 33.7 mmol). The reaction was left to stir for 3h.The mixture was concentrated in vacuo and the resulting crude was purified by crystallization from a minimum of water to afford **HO2** (754 mg, 8.28 mmol, 62 %) as a white solid. 1H NMR (400 MHz, D2O) δ 3.77 (s, 1H). 13C NMR (101 MHz, DMSO) δ 172.40, 55.10.

### Example 2. Synthesis of compound HO3

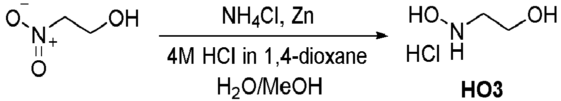

To a solution of ammonium chloride (793 mg, 2.7 Eq, 14.8 mmol) in water (3 mL) was added 2-nitroethan-1-ol (500 mg, 394 µL, 1 Eq, 5.49 mmol) and methanol (12 mL). The resulting solution was stirred and zinc (729 mg, 2.03 Eq, 11.1 mmol) was added in small portions keeping the temperature below 65 °C. The mixture was stirred at RT for 30 min and filtered. The precipitate was washed with methanol and H2O. Hydrogen chloride in 1,4-dioxane (400 mg, 2.75 mL, 4 molar, 2 Eq, 11.0 mmol) was added to the filtrate and the solution was concentrated in vacuo. The resulting crude was triturated with IPA to remove insoluble NH4Cl crystals. The filtrate was concentrated in vacuo and the resulting crude was taken up in H2O. Aqueous phase was extracted with Et2O and EtOAc to remove residual 2-nitroethan-1-ol. The aqueous phase was concentrated in vacuo to afford as **HO3** (444 mg, 3.91 mmol, 71 %) as a yellow oil. 1H NMR (400 MHz, D2O) δ 3.82 - 3.78 (m, 2H), 3.32 - 3.28 (m, 2H). 13C NMR (101 MHz, D2O) δ 54.26, 52.38.

### Example 3. Synthesis of compound HO4

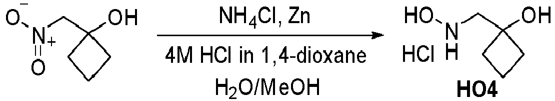

To a solution of ammonium chloride (551 mg, 2.7 Eq, 10.3 mmol) in water (3 mL) was added 1-(nitromethyl)cyclobutan-1-ol (500 mg, 1 Eq, 3.81 mmol) and methanol (12 mL). The resulting solution was stirred and zinc (506 mg, 2.03 Eq, 7.74 mmol) was added in small portions keeping the temperature below 65 °C. The mixture was stirred at RT for 15 min and filtered. The precipitate was washed with methanol and H2O. hydrogen chloride in Dioxane (278 mg, 1.91 mL, 4 molar, 2 Eq, 7.63 mmol) was added to the filtrate and the solution was concentrated in vacuo. The resulting crude was triturated with IPA to remove insoluble NH4Cl crystals. The filtrate was concentrated in vacuo and the resulting crude was taken up in H2O. Aqueous phase was extracted with Et2O and EtOAc to remove residual 1-(nitromethyl)cyclobutan-1-ol. The aqueous phase was concentrated in vacuo to afford as **HO4** (495 mg, 3.22 mmol, 85 %) as a yellow oil. 1H NMR (400 MHz, D2O) δ 3.49 (s, 2H), 2.24-2.09 (m, 4H), 1.89-1.50 (m, 2H). 13C NMR (101 MHz, D2O) δ 70.80, 57.41, 33.44, 11.41.

### Synthesis of BCN-compounds

### Example 4. Synthesis of compound 218

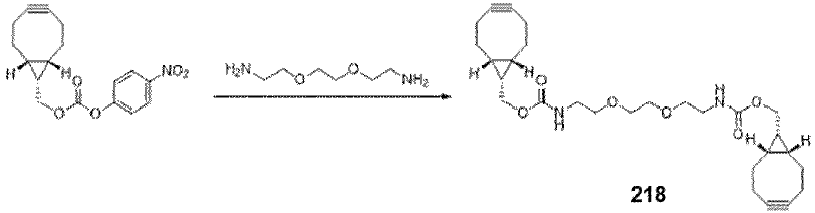

To a solution of 2-[2-(2-aminoethoxy)ethoxy]ethan-1-amine (71 mg, 0.48 mmol, 1 eq) in anhydrous DCM (1 mL) was added BCN-OPNP (339.2 mg, 1.07 mmol, 2.2 eq) and triethylamine (100 µL, 0.71 mmol, 1.5 eq). After stirring for 3 at room temperature, the reaction mixture was purified by flash column chromatography over silicagel ((0% → 30% EtOAc in DCM (to remove p-nitrophenol) followed by 0% → 20% MeOH in DCM ) to give **218** as a colorless oil (191.5 mg, 0.36 mmol, 74%). LCMS (ESI+) calculated for C48H41N2O6S+ (M+H+) 501.63 found 501.56.1H NMR (400 MHz, CDCl3) δ (ppm) 4.16 (d, J = 8.0 Hz, 4H), 3.62 (s, 4H), 3.57 (dd, J = 5.6, 4.7 Hz, 4H), 3.40 (q, J = 5.4 Hz, 4H), 2.36 - 2.17 (m, 12H), 1.61 (m, 4H), 1.37 (p, J = 8.6 Hz, 2H), 1.01 - 0.90 (m, 4H).

### Example 5. Synthesis of compound 127 and 128

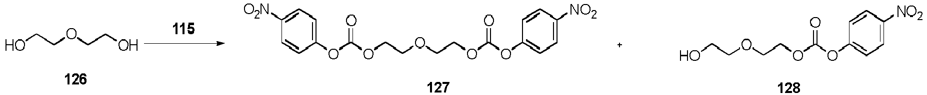

To a solution of diethyleneglycol **126** (446 µL, 0.50 g, 4.71 mmol) in DCM (20 mL) were added 4-nitrophenol chloroformate (1.4 g, 7.07 mmol) and Et3N (3.3. mL, 2.4 g, 23.6 mmol). The mixture was stirred, filtered and concentrated in vacuo (at 55°C). The residue was purified by silica gel chromatography (15% → 75% EtOAc in heptane) and two products were isolated. Product **127** was obtained as a white solid (511 mg, 1.17 mmol, 25%).1H NMR (400 MHz, CDCl3) δ (ppm) 8.31-8.23 (m, 4H), 7.43-7.34 (m, 4H), 4.54-4.44 (m, 4H), 3.91-3.83 (m,4H). Product **128** was obtained as a colorless oil (321 mg, 1.18 mmol, 25%).1H NMR (400 MHz, CDCl3) δ (ppm) 8.32-8.24 (m, 2H), 7.43-7.36 (m, 2H), 4.50-4.44 (m, 2H), 3.86-3.80 (m, 2H), 3.81-3.74 (m, 2H), 3.69-3.64 (m, 2H).

### Example 6. Synthesis of compound 145

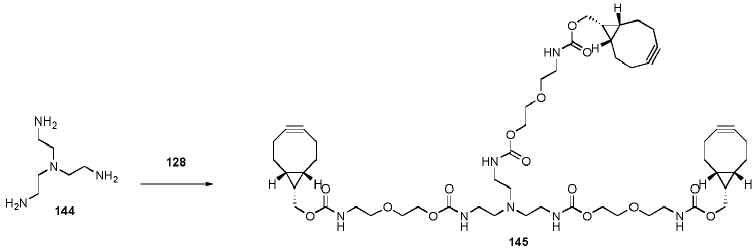

To a solution of 128 (200 mg, 0.45 mmol) in DCM (1 mL) were added triethylamine (41.6 uL, 0.30 mmol) and tris(2-aminoethyl)amine **144** (14.9 uL, 0.10 mmol). After stirring the mixture for 150 minutes, it was concentrated in vacuo. The residue was purified by silica gel column chromatography (25% ----> 100% EtOAc in DCM then 0% → 10% MeOH in DCM) and gave **145** in 43% yield (45.4 mg, 42.5 umol) as a yellow oil. 1H NMR (400 MHz, CDCl3): δ (ppm) 5.68-5.18 (m, 6H), 4.32-4.18 (m, 6H), 4.18-4.11 (d, J = 7.9 Hz, 6H), 3.74-3.61 (m, 6H), 3.61-3.51 (m, 6H), 3.43-3.29 (m, 6H), 3.29-3.15 (m, 6H), 2.65-2.47 (m, 6H), 2.37-2.16 (m, 18H), 1.69-1.49 (m, 6H), 1.35 (quintet, J = 8.9 Hz, 3H), 1.03-0.87 (m, 6H).

### Example 7-10: Synthesis of compound XL14

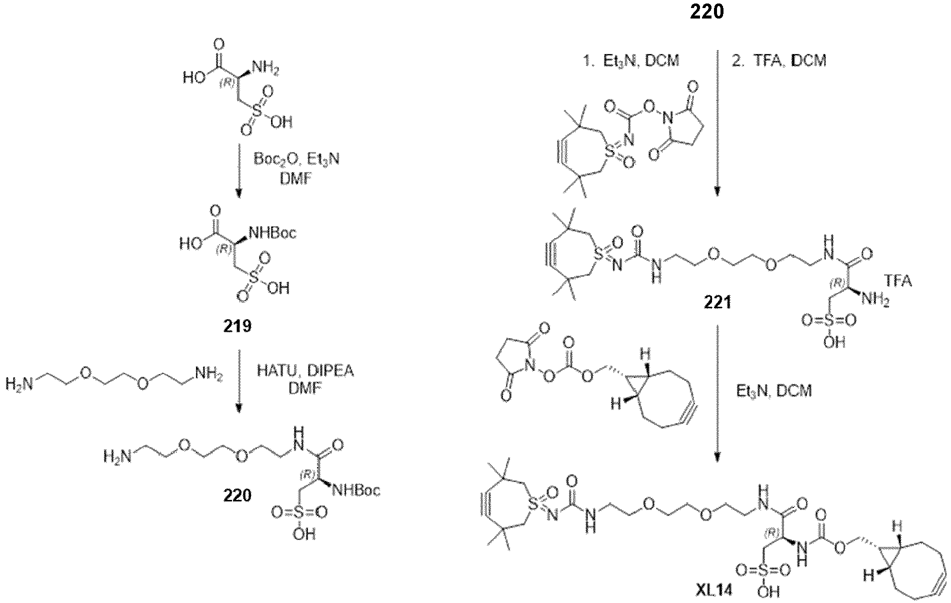

### Example 7. Synthesis of compound 219

To a solution of sulfo-D-alanine hydrate (554 mg, 1 Eq, 2.96 mmol) and triethylamine (599 mg, 825 µL, 2 Eq, 5.92 mmol) in DMF (15 mL) was added di-tert-butyl dicarbonate (775 mg, 1.2 Eq, 3.55 mmol).The reaction mixture was stirred at room temperature for 3 h. The solvent was evaporated in vacuo. The residue was redissolved in CH2Cl2 (10 mL) and treated with Et2O (30 mL) under vigorous stirring. The organic solvents were decanted and discarded. The oily residue was washed with Et2O (20 mL) and dried in vacuo to give **219** (1134 mg, 2.9 mmol, 100 %, 70% Purity) as a colorless oil. LCMS (ESI-) calculated for C8H14NO7S- (M-H-) 268.1, found 268.3.

### Example 8. Synthesis of compound 220

To a solution of compound **219** (100 mg, 1 Eq, 371 µmol) in DMF (1 mL) was added 2,2'-(ethane-1,2-diylbis(oxy))bis(ethan-1-amine) (550 mg, 542 µL, 10 Eq, 3.71 mmol), HATU (155 mg, 1.1 Eq, 409 µmol) and DIPEA (144 mg, 194 µL, 3 Eq, 1.11 mmol). The reaction mixture was stirred for 18 hours and subsequently purified by reverse phase chromatography (10% --> 100% MeCN in H2O + 1% AcOH) to afford compound **220** (42 mg, 0.11 mmol, 28 %) as a colorless oil. LCMS (ESI+) calculated for C14H30N3O8S+ (M+H+) 400.2, found 400.5.

### Example 9. Synthesis of compound 221

To a solution of compound **220** (50 mg, 1 Eq, 0.13 mmol) in DMF (1000 µL) was added TMTHSI-OSu (64 mg, 1.5 Eq, 0.19 mmol), triethylamine (51 mg, 70 µL, 4 Eq, 0.5 mmol) and the mixture was stirred at room temperature for 18 hours. The reaction mixture was purified by Reverse phase chromatography (10% --> 100% MeCN in H2O + 1% AcOH) to afford Boc-intermediate (41 mg, 66 µmol, 52 %) as a colorless oil. LCMS (ESI+) calculated for C25H45N4O10S2+ (M+H+) 625.3, found 625.5.

To a solution of Boc-intermediate (41.0 mg, 1 Eq, 65.6 µmol) in DCM (1000 µL) was added TFA (150 mg, 101 µL, 20 Eq, 1.31 mmol) and the mixture was stirred at room temperature for 1 hour. The reaction mixture was concentrated in vacuo to afford compound **221** (41.9 mg, 65.6 µmol, 100 %) as a colorless oil. LCMS (ESI+) calculated for C20H37N4O8S2+ (M+H+) 525.2, found 525.5.

### Example 10. Synthesis of compound XL14

To a solution of compound **221** (41.9 mg, 1 Eq, 65.6 µmol) in DMF (1000 µL) was added ((1R,8S,9s)-bicyclo[6.1.0]non-4-yn-9-yl)methyl (2,5-dioxopyrrolidin-1-yl) carbonate (28.7 mg, 1.5 Eq, 98.4 µmol), triethylamine (26.6 mg, 36.6 µL, 4 Eq, 262 µmol) and the mixture was stirred at room temperature for 18 hours. The reaction mixture was purified by flash column chromatography over silicagel (0-30% MeOH in DCM) to afford **XL14** (11.9 mg, 17.0 µmol, 25.9 %) as a white solid. LCMS (ESI+) calculated for C31H49N4O10S2+ (M+H+) 701.3, found 701.5.

### Expression and isolation of cytokines

### General procedure for mass spectral analysis of cytokines, antibodies and antibody-cytokine fusions

Prior to mass spectral analysis, IgG was treated with IdeS, which allows analysis of the Fc/2 fragment. For analysis of the Fc/2 fragment, a solution of 20 µg (modified) IgG was incubated for 1 hour at 37 °C with IdeS/Fabricator^{™} (1.25 U/µL) in PBS Ph 7.4 in a total volume of 10 µL. Samples were diluted to 80 µL using PBS pH 7.4. For analysis of cytokines, a solution of 4 µg (modified) cytokine was diluted to 80 µL using PBS pH 7.4. MS analysis is performed on JEOL AccuTOF LC-plus JMS-T100LP system (ESI-TOF) combined with a HPLC system (Agilent 1100 series, Hewlett Packard). On the HPLC system a MassPREP^{™} On-line Desalting Cartridge (Waters PIN 186002785) is installed. For analysis of cytokines, a solution of 4 µg (modified) cytokine was diluted to 80 µL using PBS followed by analysis electrospray ionization time-of-flight (ESI-TOF) on a JEOL AccuTOF. Deconvoluted spectra were obtained using Magtran software.

### General procedure for expression of cytokines and inclusion body isolation

Expression of cytokines starts with transformation of the corresponding into BL21(DE3) cells (Novagen). Transformed cells were plated on LB-agar with ampicillin and incubated overnight at 37 °C. A single colony was picked and used to inoculate a small culture of TB medium + ampicillin followed by incubated overnight at 37 °C. Next, the overnight culture was used to inoculate a larger volume by performing a dilution of 20 fold in TB medium + ampicillin. The culture was incubated at 37 °C at 160 RPM and, when OD600 reached 2.5-4.0, induced with 1 mM IPTG (addition of 1 mL of 1 M stock solution). After >16 hour induction at 37 °C at 160 RPM, the culture was pelleted by centrifugation (5000 xg for 5 min). The culture cell pellet was lysed in BugBuster^{™} containing Benzonase and incubated on roller bank for 30 min at room temperature. After lysis the insoluble fraction was separated from the soluble fraction by centrifugation (15 minutes, 15000 x g). Half of the insoluble fraction was dissolved in BugBuster^{™} with lysozyme (final concentration of 200 pg/mL) and incubated on the roller bank for 10 min. Next the solution was diluted with 10 volumes of miliQ and centrifuged 15 min, 15000 x g . The pellet was washed in a solution of 1 :10 diluted BugBuster^{™} and centrifuged at 10 min, 12000 x g. This washing round was performed 1-3 times until a very white inclusion body pellet was obtained.

### General procedure for refolding of cytokines

The purified inclusion bodies were dissolved and denatured in 5 M guanidine with 40 mM Cysteamine and 20 mM Tris pH 8.0, to a final concentration of 1 mg/mL. Afterwards, the solution was incubated for 2 hours at RT on a rollerbank. Followingly, the 1 mg/mL solution is added dropwise to 10 volumes of refolding buffer (50 mM Tris, 10.53 mM NaCl, 0.44 mM KCI, 2.2 mM MgCl2 , 2.2 mM CaCl2, 0.055% PEG- 4000, 0.55 M L-arginine, 4 mM cysteamine, 4 mM cystamine, at pH 8.0) in a cold room at 4°C, stirring required. Leave solution at least 24 hours at 4°C. Dialyze the solution to 10 mM NaCl and 20 mM Tris pH 8.0, 1x overnight and 2x 4 hours, using a Spectrum^{™} Spectra/Por^{™} 2 RC Dialysis Membrane Tubing 12-14 kDa MWCO. The refolded product was loaded onto a equilibrated Q-trap anion exchange column (GE health care) on an AKTA Purifier-10 (GE Healthcare). The column was first washed with buffer A (20 mM Tris, 10 mM NaCl, pH 8.0). Retained protein was eluted with buffer B (20 mM Tris buffer, 1 M NaCl, pH 8.0) on a gradient of 60 mL from buffer A to buffer B. Mass spectrometry was performed for the analysis of the product.

### Example 11. Design of IL-15 variants

Cleavable IL-15 variants were designed consisting of an N-terminal sequence suitable for chemical modification, consisting of either (M)SYR-, (M)ST- or (M)S-, fused to a protease-cleavable linker (amino acid sequence being identified by SEQ ID NO: 1 and SEQ ID NO: 2) and either wild-type or mutated human IL-15 (amino acid sequence of wild-type IL-15 being identified by SEQ ID NO: 3). The N-terminal methionine will be cleaved after expression leaving an N-terminal serine needed for chemical modification. Both cleavable linkers have been designed for recognition by several tumor-specific proteases including matriptase (MT-SP1), legumain and urokinase (UpA). Non-cleavable variants have been designed by replacing the cleavable linker by either -(G₄S)₈-, -(G₄S)₃- or a -(G₄S)₃GGS- linker. In some cases the sushi domain of IL-15Ra is fused to IL-15 via a flexible linker (amino acid sequence of IL-15Rα-linker-IL-15 being identified by SEQ ID NO: 4). Mutated IL-15 include the N72D mutation with enhanced IL-15Rβ-binding (amino acid sequence being identified by SEQ ID NO: 8), and the E46G,V49R mutation lacking IL-15Rα-binding (amino acid sequence being identified by SEQ ID NO: 11).

### Example 12. Gene synthesis and cloning into expression vector

Codon-optimized DNA encoding **PF18** (amino acid sequence being identified by SEQ ID NO: 5), **PF50** (amino acid sequence being identified by SEQ ID NO: 6), **PF51** (amino acid sequence being identified by SEQ ID NO: 7), **PF52** (amino acid sequence being identified by SEQ ID NO: 8), **PF53** (amino acid sequence being identified by SEQ ID NO: 9), **PF54** (amino acid sequence being identified by SEQ ID NO: 10), **PF55** (amino acid sequence being identified by SEQ ID NO: 11), **PF56** (amino acid sequence being identified by SEQ ID NO: 12), **PF57** (amino acid sequence being identified by SEQ ID NO: 13), **PF58** (amino acid sequence being identified by SEQ ID NO: 14), **PF59** (amino acid sequence being identified by SEQ ID NO: 15) were obtained from Genscript, Piscataway, USA. All codon-optimized DNA sequences were cloned into a pET15b-vector for tag-free expression of the proteins in E. coli.

### Example 13. Expression and refolding of cytokines PF18 and PF50-PF59

Expression and inclusion body isolation for each cytokine mentioned in table 1. was performed according to *General procedure for expression of cytokines and inclusion body isolation.* The refolding of the cytokines was performed according to *General procedure for refolding of cytokines* with small adaptations. For cytokine **PF51, PF52, PF53, PF54 and PF56,** the refolding was performed as normal. For cytokine **PF18, PF50 and PF55,** the cytokine was dissolved and denatured in 5 M guanidine with 40 mM Cysteamine and 20 mM Tris pH 8.0, to a final concentration of 2 mg/mL instead of 1 mg/mL. For cytokine **PF57 and PF59,** the cytokine was dissolved and denatured in 5 M guanidine with 40 mM Cysteamine and 20 mM Tris pH 8.0, to a final concentration of 3 mg/mL instead of 1 mg/mL. For cytokine **PF58,** the cytokine was dissolved and denatured in 5 M guanidine with 40 mM Cysteamine and 20 mM Tris pH 8.0, to an unknown concentration instead of 1 mg/mL.

**Table 1. Expression and refolding conditions for the purified cytokines.**

| Cytokine | Expression scale (L) | Inclusion body yield (mg) | Refolding scale (mg) | Final yield | Observed mass (Da) | Calculated mass (Da |
|---|---|---|---|---|---|---|
| **PF18** | 1 | 600 | 120 | 44.0 mg (36.7%) | 14122 | 14121 |
| **PF50** | 0.5 | 194 | 97 | 4.1 mg (4.2%) | 15308 | 15310 |
| **PF51** | 1.5 | 407 | 203 | 63.3 mg (31.3%) | 14606 | 14606 |
| **PF52** | 1 | 313 | 313 | 166.9 mg (53.4%) | 14388 | 14392 |
| **PF53** | 0.5 | 108 | 108 | 39.5 mg (36.6%) | 14388 | 14393 |
| **PF54** | 1 | 302 | 302 | 128.3 mg (42.3%) | 14104 | 14108 |
| **PF55** | 1.5 | 218 | 109 | 28.0 mg (25.6%) | 15696 | 15695 |
| **PF56** | 1 | 188 | 188 | 92.4 mg (48.9%) | 14372 | 14377 |
| **PF57** | 1.5 | 143 | 143 | 16.7 mg (11.7 %) | 25719 | 25721 |
| **PF58** | 1 | N.D. | N.D. | 2.4 mg | 25401 | 25402 |
| **PF59** | 0.5 | 172 | 172 | 1.3 mg (0.7%) | 25506 | 25503 |

### Preparation of modified cytokines

### Example 14. N-terminalnitrone functionalization of PF18 to obtain NHO1-PF18

To IL15 **PF18** (8070 µL, 50 µM in PBS) was added 2 eq NalO4 (16.4 µL of 50 mM stock in PBS). The reaction was incubated for 5 minutes at 4 °C. Mass spectral analysis showed oxidation of the serine into the corresponding hydrate (observed masses 14109 Da). The reaction mixture was purified using PD-10 desalting columns packed with Sephadex G-25 resin (Cytiva) and eluted using PBS. To the elute (11500 µL, 32 µM in PBS) was added 160 eq **NHO1** (558 µL of 100 mM stock in PBS) and 160 eq p-Anisidine (558 µL of 100 mM stock in PBS). The reaction mixture was incubated for 3 hours at 25 °C. Mass spectral analysis showed one single peak (observed mass 14119 Da) corresponding to **NHO1-PF18.** The reaction mixture was purified using PD-10 desalting columns packed with Sephadex G-25 resin (Cytiva) and eluted using PBS.

### Example 15. N-terminalnitrone functionalization of PF50 to obtain NHO1-PF50

To **PF50** (2500 µL, 80 µM in PBS) was added 4 eq NalO₄ (7.8 µL of 100 mM stock in PBS). The reaction was incubated for 5 minutes at 4 °C. Mass spectral analysis showed oxidation of the serine into the corresponding hydrate (observed mass 15294 Da). The reaction mixture was purified using PD-10 desalting columns packed with Sephadex G-25 resin (Cytiva) and eluted using PBS. To the elute (3500 µL, 50 µM in PBS) was added 160 eq **NHO1** (308 µL of 100 mM stock in PBS) and 160 eq p-Anisidine (308 µL of 100 mM stock in PBS). The reaction mixture was incubated overnight at 25 °C. Mass spectral analysis showed one single peak (observed mass 15306 Da) corresponding to **NHO1-PF50.** The reaction mixture was purified using PD-10 desalting columns packed with Sephadex G-25 resin (Cytiva) and eluted using PBS.

### Example 16. N-terminal nitrone functionalization of PF51 to obtain NHO1-PF51

To **PF51** (56100 µL, 61 µM in PBS) was added 2 eq NalO₄ (68.5 µL of 100 mM stock in PBS). The reaction was incubated for 5 minutes at RT. Mass spectral analysis showed oxidation of the serine into the corresponding hydrate (observed masses 14594 Da). To quench the remaining NalO₄ in the reaction, 6.6 eq of p-methoxybenzenethiol were added (2260 µL, 10 mM in DMF). To the quenched reaction was added 160 eq **NHO1** (5500 µL of 100 mM stock in PBS) and 160 eq p-Anisidine (5500 µL of 100 mM stock in PBS). The reaction mixture was incubated overnight at RT. Mass spectral analysis showed one single peak (observed mass 14604 Da) corresponding to **NHO1-PF51.** The reaction mixture was dialyzed to PBS, 1x overnight and 2x 3 hours, using a Spectrum^{™} Spectra/Por^{™} 2 RC Dialysis Membrane Tubing 12-14 kDa MWCO.

### Example 17. N-terminalnitrone functionalization of PF55 to obtain NHO1-PF55

To **PF55** (12807 µL, 61 µM in PBS) was added 2 eq NalO₄ (16.5 µL of 100 mM stock in PBS). The reaction was incubated for 5 minutes at RT. Mass spectral analysis showed oxidation of the serine into the corresponding hydrate (observed mass 15683 Da). To quench the remaining NalO₄ in the reaction, 6.6 eq of p-methoxybenzenethiol were added (546 µL, 10 mM in DMF). To the quenched reaction was added 160 eq **NHO1** (1366 µL of 100 mM stock in PBS) and 160 eq p-Anisidine (1366 µL of 100 mM stock in PBS). The reaction mixture was incubated overnight at RT. Mass spectral analysis showed one single peak (observed mass 15694 Da) corresponding to **NHO1-PF55.** The reaction mixture was directly loaded onto a desalting column (Cytiva, HiPrep^{™} 26/10 desalting column) using PBS pH 7.5 as a mobile phase on an AKTA explorer (GE Healthcare).

### Example 18. N-terminal nitrone functionalization of PF57 to obtain NHO1-PF57

To **PF57** (8000 µL, 69 µM in PBS) was added 2 eq NalO₄ (11 µL of 100 mM stock in PBS). The reaction was incubated for 5 minutes at RT. Mass spectral analysis showed oxidation of the serine into the corresponding hydrate (observed mass 25707 Da). To quench the remaining NalO₄ in the reaction, 6.6 eq of p-methoxybenzenethiol were added (364 µL, 10 mM in DMF). To the quenched reaction was added 160 eq **NHO1** (870 µL of 100 mM stock in PBS) and 160 eq p-Anisidine (870 µL of 100 mM stock in PBS). The reaction mixture was incubated overnight at RT. Mass spectral analysis showed one single peak (observed mass 25720 Da) corresponding to **NHO1-PF57.** The reaction mixture was directly loaded onto a desalting column (Cytiva, HiPrep^{™} 26/10 desalting column) using PBS pH 7.5 as a mobile phase on an AKTA explorer (GE Healthcare).

### Example 19. N-terminalnitrone functionalization of PF58 to obtain NHO1-PF58

To **PF58** (1443 µL, 65 µM in PBS) was added 2 eq NalO₄ (18.8 µL of 10 mM stock in PBS). The reaction was incubated for 5 minutes at RT. Mass spectral analysis showed oxidation of the serine into the corresponding hydrate (observed mass 25388 Da). To quench the remaining NalO₄ in the reaction, 6.6 eq of p-methoxybenzenethiol were added (62 µL, 10 mM in DMF). To the quenched reaction was added 160 eq **NHO1** (150 µL of 100 mM stock in PBS) and 160 eq p-Anisidine (150 µL of 100 mM stock in PBS). The reaction mixture was incubated overnight at RT. Mass spectral analysis showed one single peak (observed mass 25401 Da) corresponding to **NHO1-PF58.** The reaction mixture was directly loaded on a Superdex^{™}75 Increase (Cytiva) using PBS pH 7.5 as a mobile phase on an AKTA explorer (GE Healthcare).

### Example 20. N-terminalnitrone functionalization of PF59 to obtain NHO1-PF59

To **PF59** (1000 µL, 30 µM in PBS) was added 2 eq NalO₄ (6 µL of 10 mM stock in PBS). The reaction was incubated for 5 minutes at RT. Mass spectral analysis showed oxidation of the serine into the corresponding hydrate (observed mass 25494 Da). To quench the remaining NalO₄ in the reaction, 6.6 eq of p-methoxybenzenethiol were added (19.8 µL, 10 mM in DMF). To the quenched reaction was added 160 eq **NHO1** (49 µL of 100 mM stock in PBS) and 160 eq p-Anisidine (49 µL of 100 mM stock in PBS). The reaction mixture was incubated overnight at RT. Mass spectral analysis showed one single peak (observed mass 25503 Da) corresponding to **NHO1-PF59.** The reaction mixture was purified using Amicon Ultra-0.5 Centrifugal Filter Units (10 kDa MWCO) by performing 5 rounds of buffer exchange to PBS pH 7.5.

### Example 21. N-terminalnitrone functionalization of PF52 to obtain NHO2-PF52

To **PF52** (88679 µL, 61 µM in PBS) was added 2 eq NalO₄ (1113 µL of 10 mM stock in PBS). The reaction was incubated for 5 minutes at RT. Mass spectral analysis showed oxidation of the serine into the corresponding hydrate (observed mass 14375 Da). To quench the remaining NalO₄ in the reaction, 6.6 eq of p-methoxybenzenethiol were added (3672 µL, 10 mM in DMF). To the quenched reaction was added 160 eq **NHO2** (8901 µL of 100 mM stock in PBS) and 160 eq p-Anisidine (8901 µL of 100 mM stock in PBS). The reaction mixture was incubated overnight at RT. Mass spectral analysis showed one single peak (observed mass 14429 Da) corresponding to **NHO2-PF52.** Dialyze the solution to 10 mM NaCl and 20 mM Tris pH 7.5, 2x 1 hour, using a Spectrum^{™} Spectra/Por^{™} 2 RC Dialysis Membrane Tubing 12-14 kDa MWCO. For purification, the dialyzed product was directly loaded onto an AEX column (Cytiva 2x5 mL, HiTrap^{™} Q HP) on an AKTA explorer (GE Healthcare). After loading the product, the column was first washed with buffer A (20 mM Tris, 10 mM NaCl, pH 7.5). Retained protein was eluted with a linear gradient to buffer B (20 mM Tris buffer, 1 M NaCl, pH 7.5).The elute was directly loaded onto a desalting column (Cytiva, HiPrep^{™} 26/10 desalting column) using PBS pH 7.5 as a mobile phase on an AKTA explorer (GE Healthcare).

### Example 22. N-terminalnitrone functionalization of PF53 to obtain NHO2-PF53

To **PF53** (22170 µL, 61 µM in PBS) was added 2 eq NalO₄ (278 µL of 10 mM stock in PBS). The reaction was incubated for 5 minutes at RT. Mass spectral analysis showed oxidation of the serine into the corresponding hydrate (observed mass 14376 Da). To quench the remaining NalO₄ in the reaction, 6.6 eq of p-methoxybenzenethiol were added (918 µL, 10 mM in DMF). To the quenched reaction was added 160 eq **NHO2** (2225 µL of 100 mM stock in PBS) and 160 eq p-Anisidine (2225 µL of 100 mM stock in PBS). The reaction mixture was incubated overnight at RT. Mass spectral analysis showed one single peak (observed mass 14430 Da) corresponding to **NHO2-PF53.** Dialyze the solution to 10 mM NaCl and 20 mM Tris pH 7.5, 2x 1 hour, using a Spectrum^{™} Spectra/Por^{™} 2 RC Dialysis Membrane Tubing 12-14 kDa MWCO. For purification, the dialyzed product was directly loaded onto an AEX column (Cytiva 2x5 mL, HiTrap^{™} Q HP) on an AKTA explorer (GE Healthcare). After loading the product, the column was first washed with buffer A (20 mM Tris, 10 mM NaCl, pH 7.5). Retained protein was eluted with a linear gradient to buffer B (20 mM Tris buffer, 1 M NaCl, pH 7.5).The elute was directly loaded onto a desalting column (Cytiva, HiPrep^{™} 26/10 desalting column) using PBS pH 7.5 as a mobile phase on an AKTA explorer (GE Healthcare).

### Example 23. N-terminalnitrone functionalization of PF54 to obtain NHO2-PF54

To **PF54** (69660 µL, 61 µM in PBS) was added 2 eq NalO₄ (85 µL of 100 mM stock in PBS). The reaction was incubated for 5 minutes at RT. Mass spectral analysis showed oxidation of the serine into the corresponding hydrate (observed mass 14090 Da). To quench the remaining NalO₄ in the reaction, 6.6 eq of p-methoxybenzenethiol were added (2807 µL, 10 mM in DMF). To the quenched reaction was added 160 eq **NHO2** (6800 µL of 100 mM stock in PBS) and 160 eq p-Anisidine (6800 µL of 100 mM stock in PBS). The reaction mixture was incubated overnight at RT. Mass spectral analysis showed one single peak (observed mass 14146 Da) corresponding to **NHO2-PF54.** Dialyze the solution to 10 mM NaCl and 20 mM Tris pH 7.5, 2x 1 hour, using a Spectrum^{™} Spectra/Por^{™} 2 RC Dialysis Membrane Tubing 12-14 kDa MWCO. For purification, the dialyzed product was directly loaded onto an AEX column (Cytiva 2x5 mL, HiTrap^{™} Q HP) on an AKTA explorer (GE Healthcare). After loading the product, the column was first washed with buffer A (20 mM Tris, 10 mM NaCl, pH 7.5). Retained protein was eluted with a linear gradient to buffer B (20 mM Tris buffer, 1 M NaCl, pH 7.5).The elute was directly loaded onto a desalting column (Cytiva, HiPrep^{™} 26/10 desalting column) using PBS pH 7.5 as a mobile phase on an AKTA explorer (GE Healthcare).

### Example 24. N-terminalnitrone functionalization of PF56 to obtain NHO2-PF56

To **PF56** (45629 µL, 61 µM in PBS) was added 2 eq NalO₄ (55.7 µL of 100 mM stock in PBS). The reaction was incubated for 5 minutes at RT. Mass spectral analysis showed oxidation of the serine into the corresponding hydrate (observed mass 14359 Da). To quench the remaining NalO₄ in the reaction, 6.6 eq of p-methoxybenzenethiol were added (1837 µL, 10 mM in DMF). To the quenched reaction was added 160 eq **NHO2** (4453 µL of 100 mM stock in PBS) and 160 eq p-Anisidine (4453 µL of 100 mM stock in PBS). The reaction mixture was incubated overnight at RT. Mass spectral analysis showed one single peak (observed mass 143414 Da) corresponding to **NHO2-PF56.** Dialyze the solution to 10 mM NaCl and 20 mM Tris pH 7.5, 2x 1 hour, using a Spectrum^{™} Spectra/Por^{™} 2 RC Dialysis Membrane Tubing 12-14 kDa MWCO. For purification, the dialyzed product was directly loaded onto an AEX column (Cytiva 2x5 mL, HiTrap^{™} Q HP) on an AKTA explorer (GE Healthcare). After loading the product, the column was first washed with buffer A (20 mM Tris, 10 mM NaCl, pH 7.5). Retained protein was eluted with a linear gradient to buffer B (20 mM Tris buffer, 1 M NaCl, pH 7.5).The elute was directly loaded onto a desalting column (Cytiva, HiPrep^{™} 26/10 desalting column) using PBS pH 7.5 as a mobile phase on an AKTA explorer (GE Healthcare).

### Example 25. N-terminal nitrone functionalization of PF57 to obtain NHO2-PF57

To **PF57** (1350 µL, 61 µM in PBS) was added 2 eq NalO₄ (16.3 µL of 10 mM stock in PBS). The reaction was incubated for 5 minutes at RT. Mass spectral analysis showed oxidation of the serine into the corresponding hydrate (observed mass 25708 Da). To quench the remaining NalO₄ in the reaction, 6.6 eq of p-methoxybenzenethiol were added (54 µL, 10 mM in DMF). To the quenched reaction was added 160 eq **NHO2** (133 µL of 100 mM stock in PBS) and 160 eq p-Anisidine (133 µL of 100 mM stock in PBS). The reaction mixture was incubated overnight at RT. Mass spectral analysis showed one single peak (observed mass 25763 Da) corresponding to **NHO2-PF57.** The reaction mixture was purified using Amicon Ultra-0.5 Centrifugal Filter Units (10 kDa MWCO) by performing 5 rounds of buffer exchange to PBS pH 7.5.

### Example 26. N-terminal nitrone functionalization of PF57 to obtain NHO3-PF57

To **PF57** (1350 µL, 61 µM in PBS) was added 2 eq NalO₄ (16.3 µL of 10 mM stock in PBS). The reaction was incubated for 5 minutes at RT. Mass spectral analysis showed oxidation of the serine into the corresponding hydrate (observed mass 25708 Da). To quench the remaining NalO₄ in the reaction, 6.6 eq of p-methoxybenzenethiol were added (54 µL, 10 mM in DMF). To the quenched reaction was added 160 eq **NHO3** (133 µL of 100 mM stock in PBS) and 160 eq p-Anisidine (133 µL of 100 mM stock in PBS). The reaction mixture was incubated overnight at RT. Mass spectral analysis showed one single peak (observed mass 25749 Da) corresponding to **NHO3-PF57.** The reaction mixture was purified using Amicon Ultra-0.5 Centrifugal Filter Units (10 kDa MWCO) by performing 5 rounds of buffer exchange to PBS pH 7.5.

### Example 27. N-terminal nitrone functionalization of PF57 to obtain NHO4-PF57

To **PF57** (1350 µL, 61 µM in PBS) was added 2 eq NalO₄ (16.3 µL of 10 mM stock in PBS). The reaction was incubated for 5 minutes at RT. Mass spectral analysis showed oxidation of the serine into the corresponding hydrate (observed mass 25708 Da). To quench the remaining NalO₄ in the reaction, 6.6 eq of p-methoxybenzenethiol were added (54 µL, 10 mM in DMF). To the quenched reaction was added 160 eq **NHO4** (133 µL of 100 mM stock in PBS) and 160 eq p-Anisidine (133 µL of 100 mM stock in PBS). The reaction mixture was incubated overnight at RT. Mass spectral analysis showed one single peak (observed mass 25789 Da) corresponding to **NHO4-PF57.** The reaction mixture was purified using Amicon Ultra-0.5 Centrifugal Filter Units (10 kDa MWCO) by performing 5 rounds of buffer exchange to PBS pH 7.5.

### Example 28. N-terminal BCN functionalization of NHO1-PF51by SPANC to obtain 218-NHO1-PF51

To **NHO1-PF51** (77055 µL, 44 µM in PBS) was added 10 eq BCN-PEG₂-BCN **218** (8562 µL, 4 mM in DMF). The reaction was incubated overnight at room temperature. Dialyze the solution to 10 mM NaCl and 20 mM Tris pH 8.0, 2x 1 hour, using a Spectrum^{™} Spectra/Por^{™} 2 RC Dialysis Membrane Tubing 12-14 kDa MWCO. For purification, the dialyzed product was directly loaded onto an AEX column (Cytiva 2x5 mL, HiTrap^{™} Q HP) on an AKTA explorer (GE Healthcare). After loading the product, the column was first washed with buffer A (20 mM Tris, 10 mM NaCl, pH 8.0). Retained protein was eluted with a linear gradient to buffer B (20 mM Tris buffer, 1 M NaCl, pH 8.0). The elute was dialyzed to PBS, 1x overnight and 2x 3 hours, using a Spectrum^{™} Spectra/Por^{™} 2 RC Dialysis Membrane Tubing 12-14 kDa MWCO. Mass spectrometry showed the correct product (observed mass 15105 Da) corresponding to **218-NHO1-PF51**

### Example 29. N-terminal BCN functionalization of NHO2-PF52 by SPANC to obtain 218-NHO2-PF52

To **NHO2-PF52** (173349 µL, 22 µM in PBS) was added 10 eq BCN-PEG₂-BCN **218** (17335 µL, 2 mM in DMF). The reaction was incubated overnight at room temperature while stirring. Dialyze the solution to 100 mM NaCl and 20 mM Tris pH 7.5, 2x 1 hour, using a Spectrum^{™} Spectra/Por^{™} 2 RC Dialysis Membrane Tubing 12-14 kDa MWCO. For purification, the dialyzed product was directly loaded onto an AEX column (Cytiva 2x5 mL, HiTrap^{™} Q HP) on an AKTA explorer (GE Healthcare). After loading the product, the column was first washed with buffer A (20 mM Tris, 100 mM NaCl, pH 7.5). Retained protein was eluted with a linear gradient to buffer B (20 mM Tris buffer, 1 M NaCl, pH 7.5). The elute was directly loaded onto a desalting column (Cytiva, HiPrep^{™} 26/10 desalting column) using PBS pH 7.5 as a mobile phase on an AKTA explorer (GE Healthcare). Mass spectrometry showed the correct product (observed mass 14929 Da) corresponding to **218-NHO2-PF52.**

### Example 30. N-terminal BCN functionalization of NHO2-PF53 by SPANC to obtain 218-NHO2-PF53

To **NHO2-PF3** (62408 µL, 22 µM in PBS) was added 10 eq bisBCN-PEG₂ **218** (6934 µL, 2 mM in DMF). The reaction was incubated overnight at room temperature while stirring. Dialyze the solution to 100 mM NaCl and 20 mM Tris pH 7.5, 2x 1 hour, using a Spectrum^{™} Spectra/Por^{™} 2 RC Dialysis Membrane Tubing 12-14 kDa MWCO. For purification, the dialyzed product was directly loaded onto an AEX column (Cytiva 2x5 mL, HiTrap^{™} Q HP) on an AKTA explorer (GE Healthcare). After loading the product, the column was first washed with buffer A (20 mM Tris, 100 mM NaCl, pH 7.5). Retained protein was eluted with a linear gradient to buffer B (20 mM Tris buffer, 1 M NaCl, pH 7.5). The elute was directly loaded onto a desalting column (Cytiva, HiPrep^{™} 26/10 desalting column) using PBS pH 7.5 as a mobile phase on an AKTA explorer (GE Healthcare). Mass spectrometry showed the correct product (observed mass 14930 Da) corresponding to **218-NHO2-PF53.**

### Example 31. N-terminal BCN functionalization of NHO2-PF54 by SPANC to obtain 218-NHO2-PF54

To **NHO2-PF54** (160712 µL, 22 µM in PBS) was added 10 eq bisBCN-PEG₂ **218** (17857 µL, 2 mM in DMF). The reaction was incubated overnight at room temperature while stirring. Dialyze the solution to 100 mM NaCl and 20 mM Tris pH 7.5, 2x 1 hour, using a Spectrum^{™} Spectra/Por^{™} 2 RC Dialysis Membrane Tubing 12-14 kDa MWCO. For purification, the dialyzed product was directly loaded onto an AEX column (Cytiva 2x5 mL, HiTrap^{™} Q HP) on an AKTA explorer (GE Healthcare). After loading the product, the column was first washed with buffer A (20 mM Tris, 100 mM NaCl, pH 7.5). Retained protein was eluted with a linear gradient to buffer B (20 mM Tris buffer, 1 M NaCl, pH 7.5). The elute was directly loaded onto a desalting column (Cytiva, HiPrep^{™} 26/10 desalting column) using PBS pH 7.5 as a mobile phase on an AKTA explorer (GE Healthcare). Mass spectrometry showed the correct product (observed mass 14646 Da) corresponding to **218-NHO2-PF54.**

### Example 32. N-terminal BCN functionalization of NHO1-PF55 by SPANC to obtain 218-NHO1-PF55

To **NHO1-PF55** (22000 µL, 45 µM in PBS) was added 10 eq bisBCN-PEG₂ **218** (2000 µL, 5 mM in DMF). The reaction was incubated overnight at room temperature. The reaction mixture was directly loaded onto a desalting column (Cytiva, HiPrep^{™} 26/10 desalting column) using PBS pH 7.5 as a mobile phase on an AKTA explorer (GE Healthcare). Mass spectrometry showed the correct product (observed mass 16196 Da) corresponding to **218-NHO1-PF55.**

### Example 33. N-terminal BCN functionalization of NHO2-PF56 by SPANC to obtain 218-NHO2-PF56

To **NHO2-PF56** (93103 µL, 22 µM in PBS) was added 10 eq bisBCN-PEG₂ **218** (10345 µL, 2 mM in DMF). The reaction was incubated overnight at room temperature while stirring. Dialyze the solution to 100 mM NaCl and 20 mM Tris pH 7.5, 2x 1 hour, using a Spectrum^{™} Spectra/Por^{™} 2 RC Dialysis Membrane Tubing 12-14 kDa MWCO. For purification, the dialyzed product was directly loaded onto an AEX column (Cytiva 2x5 mL, HiTrap^{™} Q HP) on an AKTA explorer (GE Healthcare). After loading the product, the column was first washed with buffer A (20 mM Tris, 100 mM NaCl, pH 7.5). Retained protein was eluted with a linear gradient to buffer B (20 mM Tris buffer, 1 M NaCl, pH 7.5). The elute was directly loaded onto a desalting column (Cytiva, HiPrep^{™} 26/10 desalting column) using PBS pH 7.5 as a mobile phase on an AKTA explorer (GE Healthcare). Mass spectrometry showed the correct product (observed mass 14914 Da) corresponding to **218-NHO2-PF56.**

### Preparation of modified antibodies

### General procedure for analytical RP-UPLC of cytokines, monoclonal antibodies and antibody-cytokine conjugates

Prior to RP-UPLC analysis, protein samples were diluted to approximately 0.1 mg/mL using PBS pH 7.4. RP-UPLC analysis was performed on a Waters Acquity UPLC-SQD. The sample (5 µL) was injected with 0.4 mL/min onto Bioresolve RP mAb 2.1×150 mm 2.7 µm (Waters) with a column temperature of 70 °C. A linear gradient was applied in 9 minutes from 30 to 54% acetonitrile in 0.1% TFA and water.

### General procedure for analytical SE-HPLC of cytokines, monoclonal antibodies and antibody-cytokine conjugates

HPLC-SEC analysis was performed on an Agilent 1100 series (Hewlett Packard) using an Xbridge BEH200A (3.5 µM, 7.8x300 mm, PN 186007640 Waters) column. The sample was diluted to 1 mg/mL in PBS and measured with 0.86 mL/min isocratic method (0.1 M sodium phosphate buffer pH 6.9 (NaHPO4/Na2PO4) containing 10% isopropanol) for 16 minutes.

### Example 34. Transient expression of mPD-L1 in CHO

mPD-L1 is an antibody targeting murine PD-L1 as described in US2016/0340429 A1, consisting of a LC sequence being identified by SEQ ID NO: 16, and a HC sequence being identified by SEQ ID NO: 17. mPD-L1 was transiently expressed in CHO KI cells by Evitria (Zurich, Switzerland) at 500 mL scale. The antibody was purified using two HiTrap MabSelect Sure 5 mL columns connected in series. After loading of the supernatant the column was washed with TBS pH 7.5 for 10 CV. The IgG was eluted with 0.1 M sodium acetate pH 3.0 and neutralized with 2.5 M Tris-HCl pH 7.2. After three times dialysis to 20 mM Histidine-HCl, 150 mM NaCl, pH 7.5 the IgG was concentrated to 19.3 mg/mL using a Vivaspin Turbo 15 ultrafiltration unit (Sartorius) and obtained in a final yield of 87 mg.

### Example 35. Enzymatic remodeling of trastuzumab to tras(6-N₃-GalNAc)₂ (tras-v1a)

Trastuzumab (Herceptin), obtained from the pharmacy, was reconstituted and buffer exchanged to 20 mM Histidine-HCl, 150 mM NaCl, pH 7.5 using a HiPrep^{™} 26/10 Desalting Column (Cytiva) on an AKTA Purifier-10 (Cytiva). Trastuzumab (31.3 mL, 1128 mg, 36.0 mg/mL in 20 mM Histidine-HCl, 150 mM NaCl, pH 7.5) was incubated with EndoSH (1% w/w), as described in PCT/EP2017/052792, His-TnGaINAcT, as described in PCT/EP2016/059194 (3% w/w), alkaline phosphatase (commercially available from Roche, 0.01% w/w) and UDP-6-N3-GalNAc (10 eq compared to IgG), prepared according to PCT/EP2016/059194, in 20 mM Histidine-HCl pH 7.5 150 mM NaCl pH 7.5 and 6 mM MnCl₂ for 16 hours at 30 °C. Next, the functionalized IgG was purified using a protein A column (Captiva PriMab, CV = 50 mL). After loading of the reaction mixture the column was washed with TBS + 0.2% Triton for 10 CV followed by TBS pH 7.5 for 10 CV. The IgG was eluted with 0.1 M sodium acetate pH 3.0 and neutralized with 2.5 M Tris-HCl pH 7.2. After three times dialysis to TBS pH 7.5, the IgG was concentrated to approximately 25 mg/mL using a Vivaspin Turbo 15 ultrafiltration unit (Sartorius). Mass spectral analysis of a sample after IdeS treatment showed one major Fc/2 products (observed mass 24366 Da, approximately 90% of total Fc/2) corresponding to the 6-N₃-GalNAc-GlcNAc(Fuc)-substituted Fc/2 fragment, and one minor Fc/2 products (observed mass 24220 Da, approximately 10% of total Fc/2) corresponding to the 6-N₃-GalNAc-GlcNAc-substituted Fc/2 fragment.

### Example 36. Intramolecular cross-linking of trast-v1a with trivalent linker 145 to give trast-v1a-145

To a solution of ***trast-v1a*** (1698 µL, 35 mg, 20.61 mg/mL in TBS pH 7.5) was added TBS pH 7.5 (1802 µL), propylene glycol (3427 µL) and trivalent linker **145** (23.3 µL, 40 mM solution in DMF, 4 equiv. compared to IgG). The reaction was incubated overnight at RT followed by purification on a Superdex200 Increase 16/600 column (Cytiva) on an AKTA Purifier-10 (Cytiva) using PBS pH 7.4 as mobile phase. Mass spectral analysis of the IdeS-digested sample showed one major product (calculated mass 49799 Da, observed mass 49799Da), corresponding to intramolecularly cross-linked trastuzumab derivative ***trast-v1a-145*.**

### Example 37. Enzymatic remodeling ofmPD-L1 to mPD-L1(6-N₃-GalNAc)₂ (mPD-L1-v1a)

mPD-L1 (4528 µL, 87 mg, 19.3 mg/mL in 20 mM Histidine-HCl, 150 mM NaCl, pH 7.5) was incubated with EndoSH (1% w/w), as described in PCT/EP2017/052792, His-TnGaINAcT, as described in PCT/EP2016/059194 (4% w/w), alkaline phosphatase (commercially available from Roche, 0.01% w/w) and UDP-6-N3-GalNAc (25 eq compared to IgG), prepared according to PCT/EP2016/059194, in 20 mM Histidine-HCl pH 7.5 150 mM NaCl pH 7.5 and 6 mM MnCl₂ for 16 hours at 30 °C. Next, the functionalized IgG was purified using two HiTrap MabSelect Sure 5 mL columns connected in series. After loading of the reaction mixture the column was washed with TBS + 0.2% Triton for 10 CV followed by TBS pH 7.5 for 10 CV. The IgG was eluted with 0.1 M sodium acetate pH 3.0 and neutralized with 2.5 M Tris-HCl pH 7.2. After three times dialysis to PBS pH 7.4, the IgG was concentrated to approximately 25 mg/mL using a Vivaspin Turbo 15 ultrafiltration unit (Sartorius). Mass spectral analysis of a sample after IdeS treatment showed one major Fc/2 products (observed mass 24368 Da, approximately 90% of total Fc/2) corresponding to the 6-N₃-GalNAc-GlcNAc(Fuc)-substituted Fc/2 fragment.

### Example 38. Intramolecular cross-linking ofmPD-L1-v1a with trivalent linker 145 to give mPD-L1-v1a-145

To a solution of ***mPD-L1-v1a*** (1569 µL, 50 mg, 31.86 mg/mL in PBS pH 7.4), was added TBS pH 7.5 (3431 µL), propylene glycol (4967 µL) and trivalent linker **145** (33.3 µL, 40 mM solution in DMF, 4 equiv. compared to IgG). The reaction was incubated overnight at RT followed by purification on a Superdex200 Increase 16/600 column (Cytiva) on an AKTA Purifier-10 (Cytiva) using PBS pH 7.4 as mobile phase. Mass spectral analysis of the IdeS-digested sample showed one major product (calculated mass 49799 Da, observed mass 49799Da), corresponding to intramolecularly cross-linked mPD-L1 derivative ***mPD-L1-v1a-145.***

### Example 39. Conjugation of mPD-L1-v1a with bivalent linker XL14 to give mPD-L1-v1a-XL14

To a solution of ***mPD-L1-v1a*** (1883 µL, 60 mg, 31.86 mg/mL in PBS pH 7.4), was added TBS pH 7.5 (1617 µL) and bivalent linker **XL14** (500 µL, 20 mM solution in DMF, 25 equiv. compared to IgG). The reaction was incubated overnight at RT followed by purification on a Superdex200 Increase 16/600 column (Cytiva) on an AKTA Purifier-10 (Cytiva) using PBS pH 7.4 as mobile phase. Mass spectral analysis of the IdeS-digested sample showed one major product (observed mass 25063 Da, approximately 90% of total Fc/2), corresponding to desired product with **XL14** conjugated to only a single Fc/2-fragment leaving the second reactive handle available for further conjugation. One minor product was observed (observed mass 49427 Da, approximately 10% of total Fc/2), corresponding to the undesired intramolecularly cross-linked derivative.

### Preparation of antibody-cytokine conjugates

### General procedure for purification of antibody-cytokine conjugates

Unless stated otherwise, antibody-cytokine conjugates were purified according to the following procedure. The reaction was diluted with 2 volumes of 20 mM Tris-HCl pH 8.0 to reduce the salt concentration to 50 mM NaCl. Next, the sample was loaded onto a 5 mL HiTrap Q HP column (Cytiva) using 20 mM Tris-HCl pH 8.0, 50 mM NaCl as mobile phase. After loading the column was washed with ≥20 column volumes (CV) of 20 mM Tris-HCl pH 8.0, 50 mM NaCl, 0.2% Triton-X100 followed by ≥20CV of 20 mM Tris-HCl pH 8.0, 50 mM NaCl. Next the product was eluted using a linear gradient of 20CV to 20 mM Tris-HCl pH 8.0, 500 mM NaCl. Fractions containing the desired product were collected, pooled and concentrated to ∼5 mL using Vivaspin Turbo 15 ultrafiltration units (Sartorius). Next, the sample was purified on a Superdex200 Increase 16/600 column (Cytiva) using PBS pH 7.4 as mobile phase. The final antibody-cytokine conjugate was snap-frozen and stored at -80 °C until further use.

### Example 40. Transient expression of genetic fusion IC1 in CHO

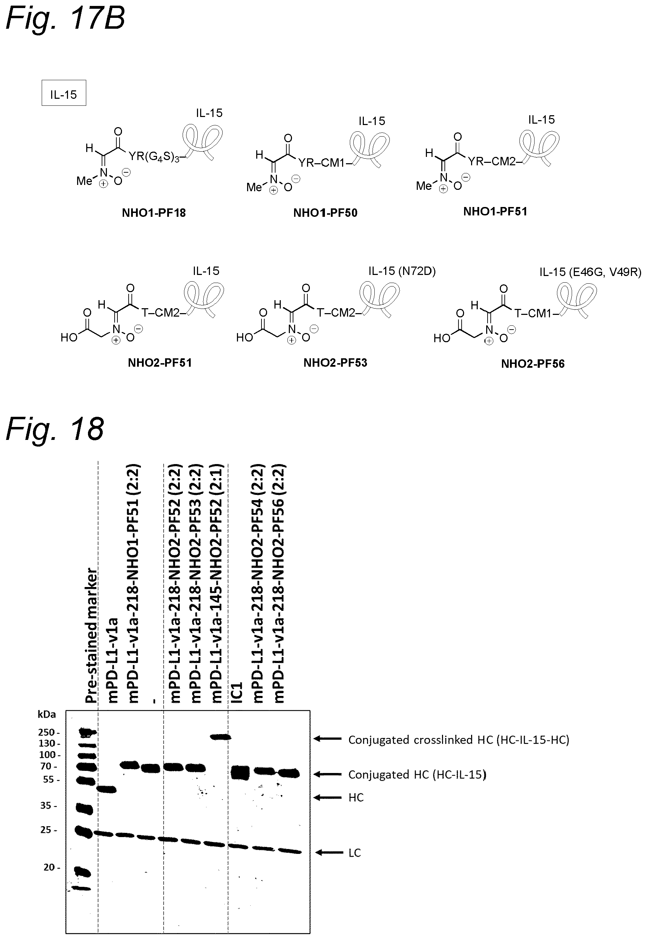

The genetic fusion **IC1** consists of the identical mPD-L1 antibody targeting murine PD-L1, C-terminally fused to an identical cleavable linker and IL-15 sequence. The LC sequence being identified by SEQ ID NO: 16, and a HC sequence being identified by SEQ ID NO: 18. **IC1** was transiently expressed in CHO KI cells by Evitria (Zurich, Switzerland) at 1100 mL scale. The antibody was purified using two HiTrap MabSelect Sure 5 mL columns connected in series. After loading of the supernatant the column was washed with TBS pH 7.5 for 10 CV. The genetic fusion protein was eluted with 0.1 M sodium acetate pH 3.0 and neutralized with 2.5 M Tris-HCl pH 7.2, yielding 22 mg IC1 with a monomer level of 77% according to SE-HPLC analysis. The sample was concentrated to 5 mL using a Vivaspin Turbo 15 ultrafiltration unit (Sartorius) followed by purification on a Superdex200 Increase 16/600 column (Cytiva) on an AKTA Purifier-10 (Cytiva) using PBS pH 7.4 as mobile phase. IC1 was obtained in a final yield of 12 mg and a monomer level of 95% according to SE-HPLC analysis. Isolation of **IC1** was confirmed by reducing SDS-PAGE analysis (Figure 18). The broad band observed for the heavy chain (HC) fused to IL-15 indicates heterogeneity, which may be due to glycosylation on the IL-15 portion.

### Example 41. Conjugation of tras-v1a-145 to NHO1-PF50 to obtain conjugate tras-v1a-145-NHO1-PF50 (2:1)

To a solution of **tras-v1a-145** (247 µL, 2 mg, 8.1 mg/mL in PBS pH 7.4) was added **NHO1-PF50** (150 µL, 3.7 mg/mL in PBS pH 7.4, 2.6 equiv. compared to IgG). The reaction was incubated overnight at room temperature. The reaction was diluted 20-fold using 20 mM Tris-HCl pH 8.0 and loaded onto a 1 mL HiTrap Q HP column (Cytiva) using 20 mM Tris-HCl pH 8.0, 10 mM NaCl as mobile phase. Next the product was eluted using a linear gradient of 50 CV to 20 mM Tris-HCl pH 8.0, 1 M NaCl. Fractions containing the desired 2:1 format were collected and concentrated to 1 mL using centrifugal filters (Amicon Ultra-0.5 mL MWCO 10 kDa, Merck Millipore). Next, the sample was purified on a Superdex200 Increase 10/300 GL column (Cytiva) on an AKTA Purifier-10 (Cytiva) using PBS pH 7.4 as mobile phase. Mass spectral analysis of the intact sample showed one major product (observed mass 162697 Da), corresponding to conjugate

### tras-v1a-145-NHO1-PF50 (2:1).

### Example 42. Conjugation of mPD-L1-v1a to 218-NHO1-PF51 to obtain conjugate mPD-L1-v1a-218-NHO1-PF51 (2:2)

To a solution of **mPD-L1-v1a** (659 µL, 21 mg, 31.9 mg/mL in PBS pH 7.4) was added **218-NHO1-PF51** (1352 µL, 7.8 mg/mL in PBS pH 7.4, 5 equiv. compared to IgG). The reaction was incubated overnight at 37 °C followed by purification according to the standard procedure. SDS-PAGE analysis confirmed formation of **mPD-L1-v1a-218-NHO1-PF51 (2:2)** (Figure 18).

### Example 43. Conjugation of mPD-L1-v1a to 218-NHO2-PF52 to obtain conjugate mPD-L1-v1a-218-NHO2-PF52 (2:2)

To a solution of **mPD-L1-v1a** (785 µL, 25 mg, 31.9 mg/mL in PBS pH 7.4) was added **218-NHO2-PF52** (2077 µL, 6.0 mg/mL in PBS pH 7.4, 5 equiv. compared to IgG). The reaction was incubated overnight at 37 °C followed by purification according to the standard procedure. SDS-PAGE analysis confirmed formation of **mPD-L1-v1a-218-NHO2-PF52 (2:2)** (Figure 18). In addition, mass spectral analysis of the IdeS digested sample showed one major product (observed mass 39295 Da), corresponding to conjugated Fc/2-fragment.

### Example 44. Conjugation of mPD-L1-v1a to 218-NHO2-PF53 to obtain conjugate mPD-L1-v1a-218-NHO2-PF53 (2:2)

To a solution of **mPD-L1-v1a** (606 µL, 19.3 mg, 31.9 mg/mL in PBS pH 7.4) was added **218-NHO2-PF53** (2639 µL, 3.64 mg/mL in PBS pH 7.4, 5 equiv. compared to IgG). The reaction was incubated overnight at 37 °C followed by purification according to the standard procedure. SDS-PAGE analysis confirmed formation **of mPD-L1-v1a-218-NHO2-PF53 (2:2)** (Figure 18). In addition, mass spectral analysis of the IdeS digested sample showed one major product (observed mass 39296 Da), corresponding to conjugated Fc/2-fragment.

### Example 45. Conjugation of mPD-L1-v1a to 218-NHO2-PF54 to obtain conjugate mPD-L1-v1a-218-NHO2-PF54 (2:2)

To a solution of **mPD-L1-v1a** (942 µL, 30 mg, 31.9 mg/mL in PBS pH 7.4) was added **218-NHO2-PF54** (3261 µL, 5.39 mg/mL in PBS pH 7.4, 6 equiv. compared to IgG). The reaction was incubated overnight at 37 °C followed by purification according to the standard procedure. SDS-PAGE analysis confirmed formation of **mPD-L1-v1a-218-NHO2-PF54 (2:2)** (Figure 18). In addition, mass spectral analysis of the IdeS digested sample showed one major product (observed mass 39012 Da), corresponding to conjugated Fc/2-fragment.

### Example 46. Conjugation of mPD-L1-v1a to 218-NHO2-PF56 to obtain conjugate mPD-L1-v1a-218-NHO2-PF56 (2:2)

To a solution of **mPD-L1-v1a** (691 µL, 22 mg, 31.9 mg/mL in PBS pH 7.4) was added **218-NHO2-PF56** (1667 µL, 6.56 mg/mL in PBS pH 7.4, 5 equiv. compared to IgG). The reaction was incubated overnight at 37 °C followed by purification according to the standard procedure. SDS-PAGE analysis confirmed formation **of mPD-L1-v1a-218-NHO2-PF56 (2:2)** (Figure 18). In addition, mass spectral analysis of the IdeS digested sample showed one major product (observed mass 39280 Da), corresponding to conjugated Fc/2-fragment.

### Example 47. Conjugation of mPD-L1-v1a-145 to NHO2-PF52 to obtain conjugate mPD-L1-v1a-145-NHO2-PF52 (2:1)

To a solution of **mPD-L1-v1a-145** (869 µL, 23.6 mg, 27.1 mg/mL in PBS pH 7.4) was added **NHO2-PF52** (910 µL, 7.47 mg/mL in PBS pH 7.4, 3 equiv. compared to IgG). The reaction was incubated overnight at room temperature followed by purification according to the standard procedure. SDS-PAGE analysis confirmed formation of **mPD-L1-v1a-145-NHO2-PF52 (2:1)** (Figure 18). In addition, mass spectral analysis of the IdeS digested sample showed one major product (observed mass 64226 Da), corresponding to crosslinked and conjugated Fc/2-fragment (Fc/2-IL-15-Fc/2).

### Example 48. Conjugation of mPD-L1-v1a-XL14 to NHO2-PF52 to obtain conjugate mPD-L1-v1a-XL14-NHO2-PF52 (2:2)

To a solution of **mPD-L1-v1a-XL14** (1541 µL, 45 mg, 29.21 mg/mL in PBS pH 7.4) was added **NHO2-PF52** (1739 µL, 7.47 mg/mL in PBS pH 7.4, 3 equiv. compared to IgG). The reaction was incubated overnight at 37 °C followed by purification according to the standard procedure. Mass spectral analysis of the IdeS digested sample showed one major product (observed mass 39495 Da), corresponding to conjugated Fc/2-fragment.

### Example 49. Conjugation of tras-v1a-145 to NHO1-PF57 to obtain conjugate tras-v1a-145-NHO1-PF57 (2:1)

To a solution **oftrast-v1a-145** (1600 µL, 16 mg, 10 mg/mL in PBS pH 7.4) was added **NHO1-PF57** (727 µL, 6.20 mg/mL in PBS pH 7.4, 1.6 equiv. compared to IgG). The reaction was incubated overnight at room temperature. The reaction was diluted 20-fold using 20 mM Tris-HCl pH 8.0 and loaded onto a 1 mL HiTrap Q HP column (Cytiva) using 20 mM Tris-HCl pH 8.0, 10 mM NaCl as mobile phase. Next the product was eluted using a linear gradient of 50 CV to 20 mM Tris-HCl pH 8.0, 1 M NaCl. Fractions containing the desired 2:1 format were collected and concentrated to 1 mL using centrifugal filters (Amicon Ultra-0.5 mL MWCO 10 kDa, Merck Millipore). Next, the sample was purified on a Superdex200 Increase 10/300 GL column (Cytiva) on an AKTA Purifier-10 (Cytiva) using PBS pH 7.4 as mobile phase. Mass spectral analysis of the intact sample showed one major product (observed mass 173099 Da), corresponding to conjugate **tras-v1a-145-NHO1-PF57 (2:1).**

### Example 50. Conjugation of tras-v1a-145 to NHO1-PF58 to obtain conjugate tras-v1a-145-NHO1-PF58 (2:1)

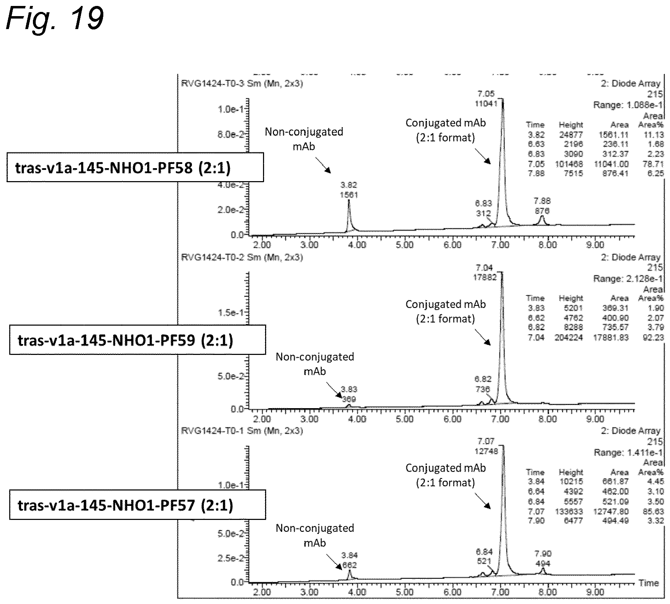

To a solution of **trast-v1a-145** (86 µL, 0.7 mg, 8.1 mg/mL in PBS pH 7.4) was added **NHO1-PF58** (42 µL, 7.94 mg/mL in PBS pH 7.4, 3 equiv. compared to IgG). The reaction was incubated overnight at room temperature. Next, the sample was purified on a Superdex200 Increase 10/300 GL column (Cytiva) on an AKTA Purifier-10 (Cytiva) using PBS pH 7.4 as mobile phase. RP-UPLC analysis of the intact sample confirmed formation of the conjugate **tras-v1a-145-NHO1-PF58 (2:1)** (Figure 19).

### Example 51. Conjugation of tras-v1a-145 to NHO1-PF59 to obtain conjugate tras-v1a-145-NHO1-PF59 (2:1)

To a solution **of trast-v1a-145** (85 µL, 0.9 mg, 10 mg/mL in PBS pH 7.4) was added **NHO1-PF59** (140 µL, 2.16 mg/mL in PBS pH 7.4, 2 equiv. compared to IgG). The reaction was incubated overnight at room temperature. Next, the sample was purified on a Superdex200 Increase 10/300 GL column (Cytiva) on an AKTA Purifier-10 (Cytiva) using PBS pH 7.4 as mobile phase. RP-UPLC analysis of the intact sample confirmed formation of the conjugate **tras-v1a-145-NHO1-PF59 (2:1)** (Figure 19).

### Example 52. Conjugation of tras-v1a-145 to NHO2-PF57 to obtain conjugate tras-v1a-145-NHO2-PF57 (2:1)

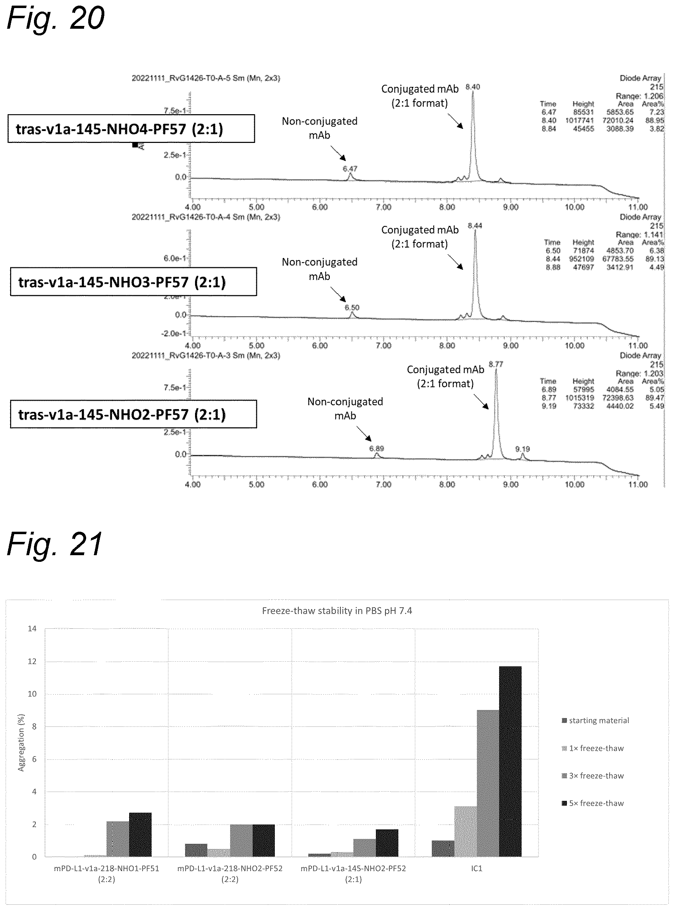

To a solution **oftrast-v1a-145** (95 µL, 1.0 mg, 10.6 mg/mL in PBS pH 7.4) was added **NHO2-PF57** (69 µL, 7.49 mg/mL in PBS pH 7.4, 3 equiv. compared to IgG). The reaction was incubated overnight at room temperature. Next, the sample was purified on a Superdex200 Increase 10/300 GL column (Cytiva) on an AKTA Purifier-10 (Cytiva) using PBS pH 7.4 as mobile phase. RP-UPLC analysis of the intact sample confirmed formation of the conjugate **tras-v1a-145-NHO2-PF57 (2:1)** (Figure 20).

### Example 53. Conjugation of tras-v1a-145 to NHO3-PF57 to obtain conjugate tras-v1a-145-NHO3-PF57 (2:1)

To a solution **oftrast-v1a-145** (95 µL, 1.0 mg, 10.6 mg/mL in PBS pH 7.4) was added **NHO3-PF57** (86 µL, 5.96 mg/mL in PBS pH 7.4, 3 equiv. compared to IgG). The reaction was incubated overnight at room temperature. Next, the sample was purified on a Superdex200 Increase 10/300 GL column (Cytiva) on an AKTA Purifier-10 (Cytiva) using PBS pH 7.4 as mobile phase. RP-UPLC analysis of the intact sample confirmed formation of the conjugate **tras-v1a-145-NHO3-PF57 (2:1)** (Figure 20).

### Example 54. Conjugation of tras-v1a-145 to NHO4-PF57 to obtain conjugate tras-v1a-145-NHO4-PF57 (2:1)

To a solution **oftrast-v1a-145** (95 µL, 1.0 mg, 10.6 mg/mL in PBS pH 7.4) was added **NHO4-PF57** (73 µL, 7.06 mg/mL in PBS pH 7.4, 3 equiv. compared to IgG). The reaction was incubated overnight at room temperature. Next, the sample was purified on a Superdex200 Increase 10/300 GL column (Cytiva) on an AKTA Purifier-10 (Cytiva) using PBS pH 7.4 as mobile phase. RP-UPLC analysis of the intact sample confirmed formation of the conjugate **tras-v1a-145-NHO4-PF57 (2:1)** (Figure 20).

### Evaluation of antibody-cytokine conjugates

### Example 55: Freeze-thaw stability study

Antibody-cytokine conjugates **mPD-L1-v1a-218-NHO1-PF51 (2:2), mPD-L1-v1a-218-NHO2-PF52 (2:2), mPD-L1-v1a-145-NHO2-PF52 (2:1)** and **IC1** were diluted to 1 mg/mL in PBS pH 7.4. Next, samples were snap-frozen using liquid nitrogen and thawed again. This step was performed 5 times. Aggregate levels were determined by SE-HPLC after 0, 1, 3 and 5 cycles (Figure 21). Conjugates **mPD-L1-v1a-218-NHO1-PF51 (2:2), mPD-L1-v1a-218-NHO2-PF52 (2:2)** and **mPD-L1-v1a-145-NHO2-PF52 (2:1)** were less prone to aggregation compared to genetic fusion IC1.

### Example 56: Stability study for antibody-cytokine conjugates prepared with IL-15 variants with variable N-terminal IL-15 sequence and using different hydroxylamines

Antibody-cytokine conjugates were diluted to 1 mg/mL in PBS pH 7.4 and incubated at 37°C. At the indicated time points, samples were analyzed by RP-UPLC to determine the stability (Table 2). The variants prepared with hydroxylamines **HO2, HO3** and **HO4** showed improved stability over the variant prepared using **HO1.**

**Table 2. Stability of antibody-cytokine conjugates as determined by RP-UPLC. Stability is plotted as percentage of the cytokine-to-antibody ratio compared to T0.**

| | day 1 | day 3 | day 7 | day 10 | day 14 | day 21 | day 28 |
|---|---|---|---|---|---|---|---|
| **mPD-L1-v1a-218-NHO1-PF51 (2:2)** | 98% | 92% | 87% | 81% | 76% | 62% | 55% |
| **mPD-L1-v1a-218-NHO2-PF52 (2:2)** | 99% | 97% | 95% | 94% | 92% | 88% | 85% |
| **mPD-L1-v1a-218-NHO2-PF53 (2:2)** | 99% | 98% | 95% | 94% | 93% | 89% | 84% |
| **mPD-L1-v1a-145-NHO2-PF52 (2:1)** | 100% | 98% | 96% | 94% | 91% | 87% | 83% |
| **mPD-L1-v1a-218-NHO2-PF54 (2:2)** | 99% | 97% | 95% | 93% | 92% | 86% | 85% |
| **mPD-L1-v1a-218-NHO2-PF56 (2:2)** | 99% | 98% | 95% | 94% | 93% | 89% | 84% |

### Example 57: Cleavage of antibody-cytokine conjugates by matriptase (MT-SP1)

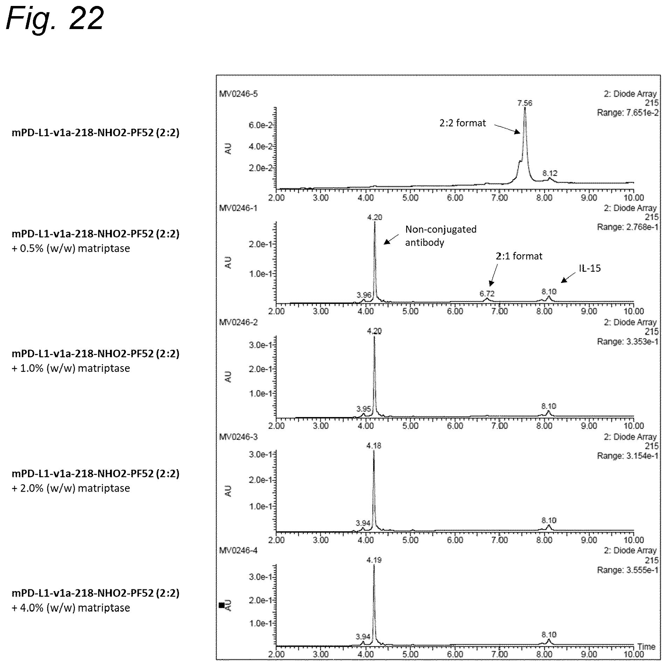

**mPD-L1-v1a-218-NHO2-PF52 (2:2)** was incubated at a final concentration of 1 mg/mL with 0.5, 1.0, 2.0 and 4.0 (w/w) % of recombinant human matriptase (commercially available from Bio-techne) for 2 hours at 37 °C followed by RP-UPLC analysis. Full cleavage was observed using 1.0% of matriptase (Figure 22).

### Example 58. Treatment of antibody-cytokine conjugates with MT-SP1 prior to in vitro evaluation

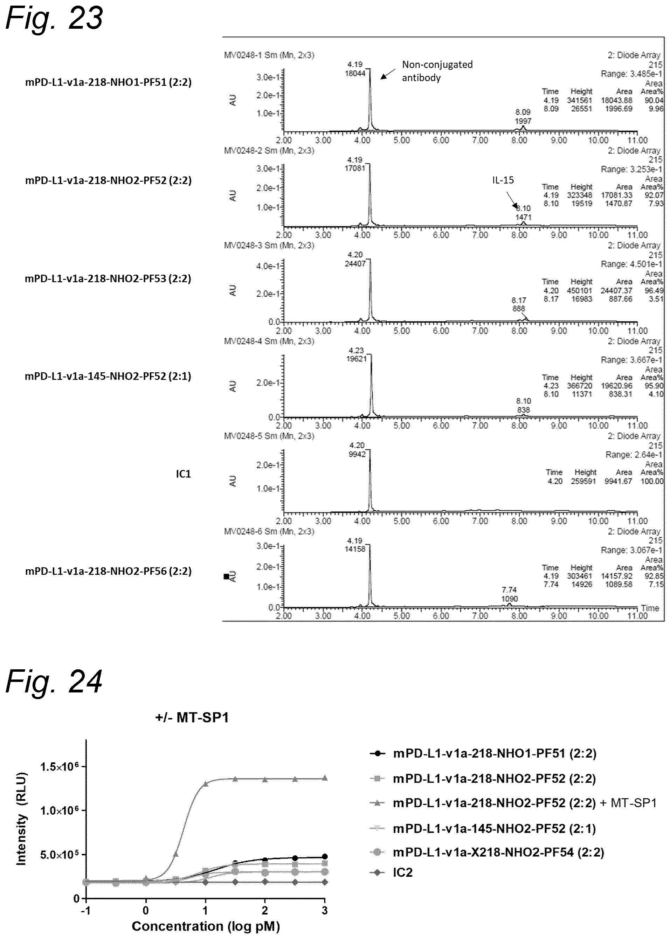

A set of antibody-cytokine conjugates (35 µg each) as indicated in figure 23 was incubated at a final concentration of 1 mg/mL with 2 (w/w) % of recombinant human matriptase (commercially available from Bio-techne) for 2 hours at 37 °C followed by RP-UPLC analysis. Full cleavage was observed in all cases (Figure 23). The cleaved constructs were used for evaluation of IL-15 activity in the in vitro IL-15 bioassay.

### Example 59: Cleavage of antibody-cytokine conjugates by urokinase (uPA)

**Tras-v1a-145-NHO1-PF50 (2:1)** was incubated at a final concentration of 1 mg/mL with 0.1, 1.0, and 10.0 (w/w) % of recombinant human urokinase (uPA, commercially available from Bio-techne) for 3 hours at room temperature. Intact samples were analyzed by MS. For the condition with 10% (w/w) of uPA, mass spectral analysis of the intact sample showed one major product (observed mass 149118 Da), corresponding to the cleaved antibody without IL-15, and one minor product (observed mass 13598 Da), corresponding to free IL-15. For conditions with 0.1% and 1.0% (w/w) uPA, mass spectral analysis of the intact sample showed uncleaved **tras-v1a-145-NHO1-PF50 (2:1)** as major product (observed mass 162708 Da). The sample treated with 10% (w/w) of uPA was used for evaluation of IL-15 activity in the in vitro IL-15 bioassay.

### In vitro evaluation

### General procedure for the in vitro IL-15 bioassay

IL-15 activity was monitored using an IL-15 bioassay (Promega, cat.# JA2015). This is a bioluminescent cell-based assay designed to measure IL-15 stimulation or inhibition using the STAT-5 response element as a readout. When IL-15 binds to its receptor, receptor-mediated pathway signalling induces luminescence that can be detected upon addition of a substrate and quantified with a luminometer. The assay was performed according to the manufacturer's instructions. In short, cleaved or intact antibody-cytokine conjugates or controls were added in duplo to IL-15 cells in a square root of 10-fold dilution series to obtain a final concentration ranging from 0.1 to 1000 pM unless stated otherwise (in case of cleaved constructs the concentration refers to the initial antibody-cytokine conjugate). IL-15 cells were incubated for 6 hours in a humidified atmosphere at 37°C and 5% CO₂. Detection reagent (Bio-Gio^{™} luciferase Assay reagent) was then added and chemiluminescence intensity was recorded with a microplate reader (Envision, PerkinElmer). Data analysis was performed using Graphpad prism software. EC₅₀ values were calculated by non-linear regression and maximum fold induction was calculated by dividing the relative luminescence units (RLU) of treated cells by the RLU of untreated cells (both background substracted).

### Example 60. In vitro IL-15 activity assay of intact and cleavad antibody-cytokine conjugates

The activity of 5 intact antibody-cytokine conjugates was measured via the in vitro IL-15 bioassay. **mPD-L1-v1a-218-NHO2-PF52 (2:2)** was included both with and without pretreatment with MT-SP1 for comparison. MT-SP1 treatment was performed according to example 58. All intact antibody-cytokine conjugates showed only minimal activation ranging from 1.0 to 2.5 fold induction, while the cleaved variant of **mPD-L1-v1a-218-NHO2-PF52 (2:2)** showed 7.2 fold induction Figure 24 and Table 3), demonstrating that IL-15 is inactivated or attenuated when linked to the antibody but becomes activated upon cleavage of the linker by MT-SP1.

**Table 3. EC₅₀ values and maximum fold induction of intact and cleaved antibody-cytokine conjugates as determined by the IL-15 bioassay.**

| Compound | MT-SP1 | EC₅₀ (pM) | Fold induction |
|---|---|---|---|
| **mPD-L1-v1a-218-NHO1-PF51 (2:2)** | - | 14.9 | 2.5 |
| **mPD-L1-v1a-218-NHO2-PF52 (2:2)** | - | 8.1 | 2.1 |
| **mPD-L1-v1a-218-NHO2-PF52 (2:2)** | + | 4.3 | 7.2 |
| **mPD-L1-v1a-145-NHO2-PF52 (2:1)** | - | 12.3 | 1.6 |
| **IC1** | - | - | 1.0 |
| **mPD-L1-v1a-218-NHO2-PF54 (2:2)** | - | 5.9 | 1.6 |

### Example 61. In vitro IL-15 activity assay of cleaved antibody-cytokine conjugates

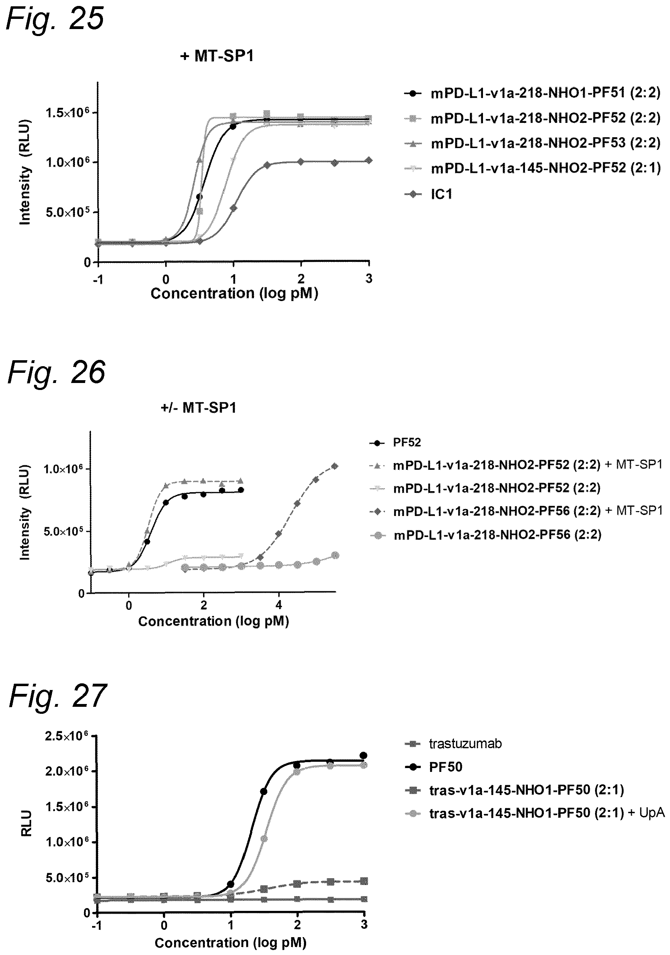

Antibody-cytokine conjugates were pre-treated with MT-SP1 according to example 58 to obtain the free cytokines. The activity of 5 cleaved antibody-cytokine conjugates was measured via the in vitro IL-15 bioassay according to the general procedure. Both **mPD-L1-v1a-218-NHO1-PF51 (2:2)** and **mPD-L1-v1a-218-NHO2-PF52 (2:2)** showed higher potency in terms of EC₅₀ values and maximum fold induction compared to the genetic fusion **IC1** comprising the same cleavable linker, IL-15 variant and stoichiometry (Figure 25 and Table 4). The corresponding 2:1 format **mPD-L1-v1a-145-NHO2-PF52 (2:1)** showed approximately a 2-fold higher EC₅₀ value compared to the 2:2 format as would be expected based on the amount of IL-15 which could be released. The variant with enhanced **IL-15Rβ-binding mPD-L1-v1a-218-NHO2-PF53 (2:2)** showed comparable in vitro potency as **mPD-L1-v1a-218-NHO2-PF52 (2:2),** which could be explained by fact that this assay is performed using a mouse cell line and this mutation binds with higher affinity to human IL-15Rβ but not murine IL-15Rβ.

**Table 4. EC₅₀ values and maximum fold induction of antibody-cytokine conjugates pre-treated with MT-SP1 determined by the IL-15 bioassay**

| Compound | MT-SP1 | EC₅₀ (pM) | Fold induction |
|---|---|---|---|
| **mPD-L1-v1a-218-NHO1-PF51 (2:2)** | + | 3.8 | 7.5 |
| **mPD-L1-v1a-218-NHO2-PF52 (2:2)** | + | 3.4 | 7.8 |
| **mPD-L1-v1a-218-NHO2-PF53 (2:2)** | + | 2.6 | 7.5 |
| **mPD-L1-v1a-145-NHO2-PF52 (2:1)** | + | 7.7 | 7.3 |
| IC1 | + | 11.0 | 5.3 |

### Example 62. In vitro IL-15 activity assay of intact and cleaved antibody-cytokine conjugates

**mPD-L1-v1a-218-NHO2-PF52 (2:2)** and **mPD-L1-v1a-218-NHO2-PF56 (2:2)** were pre-treated with MT-SP1 according to example 58 to obtain the free cytokines. The activity of both compounds was measured with and without pre-treated with MT-SP1 using the in vitro IL-15 bioassay. Non-modified cytokine **PF52** was included as a reference. Cleaved and intact **mPD-L1-v1a-218-NHO2-PF56 (2:2)** was added in a higher final concentration ranging from 32 pM to 316 nM (square root of 10-fold dilution series). Both antibody-cytokine conjugates showed activation upon pre-treatment with MT-SP1 as demonstrated by the increased fold induction (Figure 26 and table 5). **mPD-L1-v1a-218-NHO2-PF56 (2:2)** showed an >1000 fold higher EC₅₀ value, indicating that the high affinity binding to IL-15Rα has been annihilated while binding to IL-15Rβ/γ retained.

**Table 5. EC₅₀ values and maximum fold induction of intact and cleaved antibody-cytokine conjugates as determined by the IL-15 bioassay. Non-modified cytokine P52 was included as a reference.**

| Compound | MT-SP1 | EC₅₀ | Fold induction |
|---|---|---|---|
| **PF52** | - | 4.0 pM | 4.2 |
| **mPD-L1-v1a-218-NHO2-PF52 (2:2)** | - | 11.4 pM | 1.5 |
| | + | 3.4 pM | 4.6 |
| **mPD-L1-v1a-218-NHO2-PF56 (2:2)** | - | - | 1.5 |
| | + | 20.9 nM | 5.2 |

### Example 63. In vitro IL-15 activity assay with alternative antibody and cleavable linker

**Tras-v1a-145-NHO1-PF50 (2:1)** was pre-treated with urokinase (UpA) according to example 59 to obtain the free cytokines. The activity of **tras-v1a-145-NHO1-PF50 (2:1)** was measured with and without pre-treatment with UpA using the in vitro IL-15 bioassay. Non-modified cytokine **PF50** and the non-modified antibody trastuzumab were included as controls. Intact **tras-v1a-145-NHO1-PF50 (2:1)** showed only minimal activation (Table 6 and Figure 27), while cleaved **tras-v1a-145-NHO1-PF50 (2:1)** showed a similar response as the modified cytokine **PF50.**

**Table 6. EC₅₀ values and maximum fold induction of intact and cleaved antibody-cytokine conjugates as determined by the IL-15 bioassay. Non-modified cytokine P52 was included as a reference.**

| Compound | MT-SP1 | EC₅₀ | Fold induction |
|---|---|---|---|
| trastuzumab | - | - | 0.9 |
| **PF50** | - | 21 pM | 10.1 |
| **tras-v1a-145-NHO1-PF50 (2:1)** | - | 34 pM | 2.0 |
| | + | 34 pM | 9.5 |

### In vivo evaluation

### Example 64. In vivo tolerability in female BALB/c Mice

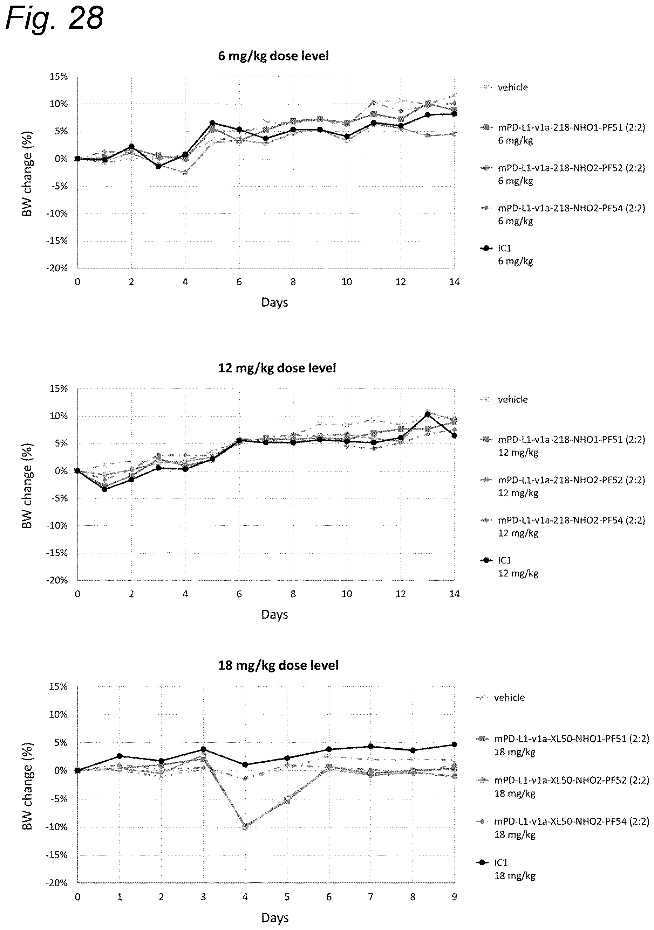

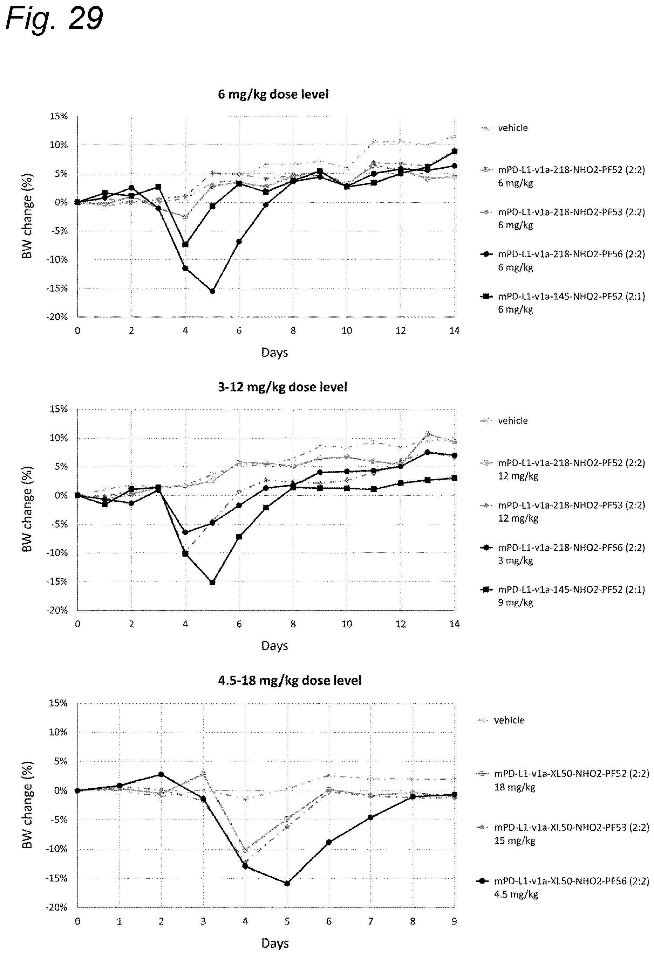

Female BALB/c mice (groups of 3 mice, 6- to 8-week-old at study initiation, obtained from Vital River Laboratory Animal Technology Co., Ltd., China), were treated with vehicle (PBS pH 7.4) or the indicated antibody-cytokine conjugate (at 6 mg/kg), and compared to **IC1** (at 6 mg/kg). In all cases a single dose was administered via s.c. injection. After dosing, all animals were measured daily for body weight (Figures 28 and 29, top graphs). Based on the read-out of this initial study, a second tolerability study was performed according to the same procedure but using dosing concentrations ranging from 3 to 12 mg/kg (Figures 28 and 29, middle graphs). Finally, a third study was performed according to the same procedure using dosing concentrations ranging from 4.5 to 18 mg/kg (Figures 28 and 29, bottom graphs). Antibody-cytokine conjugates with cleavable IL-15 **mPD-L1-v1a-218-NHO1-PF51 (2:2)** and **mPD-L1-v1a-218-NHO2-PF52 (2:2)** showed no signs of toxicity at 6 and 12 mg/kg, but moderate body weight loss (≤10% mean body weight loss) when dosed at 18 mg/kg, indicating an MTD of ~18mg/kg (Figure 28). The variant lacking IL-15Rα-binding **mPD-L1-v1a-218-NHO2-PF56 (2:2)** showed an MTD of ~4.5 mg/kg, and for the initial dose of 6 mg/kg 1 out of 3 mice was found death on day 5. Compared to **mPD-L1-v1a-218-NHO2-PF52 (2:2),** variants **mPD-L1-v1a-145-NHO2-PF52 (2:1)** and **mPD-L1-v1a-218-NHO2-PF53 (2:2)** also showed a reduced tolerability with an estimated MTD of 9 and 15 mg/kg, respectively (Figure 29).

### Example 65. In vivo efficacy in CT26 syngeneic colon cancer model

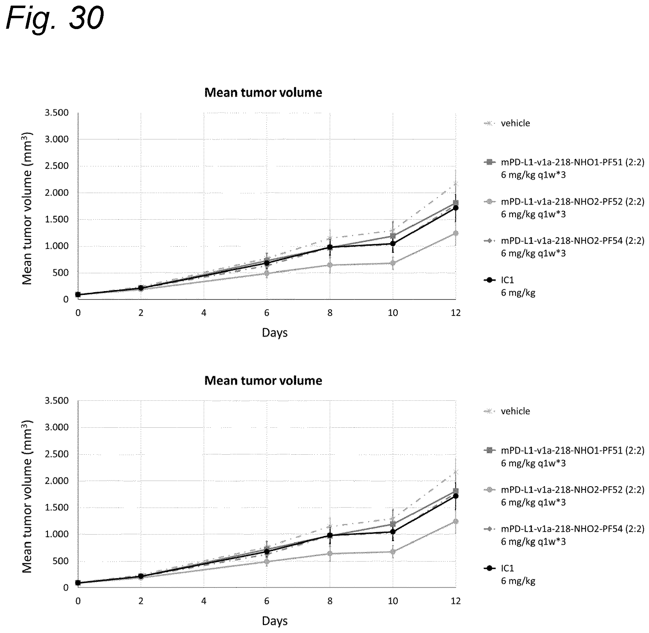

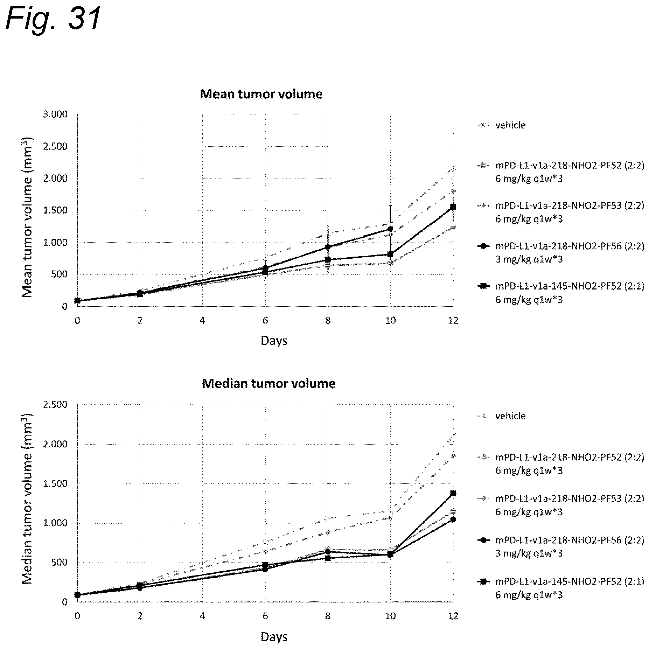

Female BALB/c mice (5- to 8-week-old at study initiation, obtained from Vital River Laboratory Animal Technology Co., Ltd., China) were inoculated subcutaneously it the right rear flank region with 5 × 10⁵ CT26 tumor cells in 0.1 ml of PBS for tumor development. When the tumor volume was in the range of 70 to 120 mm³, groups of eight mice were injected s.c. with either vehicle (PBS pH 7.4) or the indicated antibody-cytokine conjugate (either 3 or 6 mg/kg as indicated). In all cases a dose was administered on day 0 and 7. Tumor volume and body weight was measured 2-3 times per week after randomization. **mPD-L1-v1a-218-NHO2-PF52 (2:2)** showed improved anti-tumor efficacy compared to the corresponding genetic fusion **IC1** and the non-cleavable variant **mPD-L1-v1a-218-NHO2-PF54 (2:2)** (Figure 30). Additionally, variants **mPD-L1-v1a-145-NHO2-PF52 (2:1)** and **mPD-L1-v1a-218-NHO2-PF53 (2:2)** and **mPD-L1-v1a-218-NHO2-PF56 (2:2)** showed anti-tumor efficacy compared to the vehicle group (Figure 31).

### Sequence list

*Sequence identification* of protease-cleavable linker 1 *(SEQ. ID NO: 1):*
   GSSGGSGGSGGSGLSGRSDNHGSSGS
*Sequence identification* of protease-cleavable linker 2 *(SEQ. ID NO: 2):*
   GGGGSGGGGSRASRANGS
*Sequence identification of human IL-15 (SEQ. ID NO: 3):* 
NWVNVISDLKKIEDLIQSMHIDATLYTESDVHPSCKVTAMKCFLLELQVISLESGDASIHDTVENLIILANNSL
SSNGNVTESGCKECEELEEKNIKEFLQSFVHIVQMFINTS
*Sequence identification of* IL-15Rα-IL-15 fusion protein *(SEQ. ID NO: 4):* 
ITCPPPMSVEHADIWVKSYSLYSRERYICNSGFKRKAGTSSLTECVLNKATNVAHWTTPSLKCIRDPALVH
QRPAPPSGGSGGGGSGGGSGGGGSLQNWVNVISDLKKIEDLIQSMHIDATLYTESDVHPSCKVTAMKCF
LLELQVISLESGDASIHDTVENLIILANNSLSSNGNVTESGCKECEELEEKNIKEFLQSFVHIVQMFINTS
*Sequence identification of* SYR-(G₄S)₃-IL15 **(PF18)** *(SEQ. ID NO: 5):* 
SYRGGGGSGGGGSGGGGSNWVNVISDLKKIEDLIQSMHIDATLYTESDVHPSCKVTAMKCFLLELQVISL
ESGDASIHDTVENLIILANNSLSSNGNVTESGCKECEELEEKNIKEFLQSFVHIVQMFINTS
*Sequence identification of* SYR-cleavable linker 1-IL15 **(PF50)** *(SEQ. ID NO: 6):* 
MSYRGSSGGSGGSGGSGLSGRSDNHGSSGSNWVNVISDLKKIEDLIQSMHIDATLYTESDVHPSCKVTA
MKCFLLELQVISLESGDASIHDTVENLIILANNSLSSNGNVTESGCKECEELEEKNIKEFLQSFVHIVQMFIN
TS
*Sequence identification* of SYR-cleavable linker 2-IL15 **(PF51)** *(SEQ. ID NO: 7):* 
MSYRGGGGSGGGGSRASRANGSNWVNVISDLKKIEDLIQSMHIDATLYTESDVHPSCKVTAMKCFLLEL
QVISLESGDASIHDTVENLIILANNSLSSNGNVTESGCKECEELEEKNIKEFLQSFVHIVQMFINTS
*Sequence identification of* ST-cleavable linker 2-IL1 5 **(PF52)** *(SEQ. ID NO: 8):* 
STGGGGSGGGGSRASRANGSNWVNVISDLKKIEDLIQSMHIDATLYTESDVHPSCKVTAMKCFLLELQVI
SLESGDASIHDTVENLIILANNSLSSNGNVTESGCKECEELEEKNIKEFLQSFVHIVQMFINTS
*Sequence identification* of ST-cleavable linker 2-IL1 5(N72D) (PF53) *(SEQ. ID NO: 9):* 
STGGGGSGGGGSRASRANGSNWVNVISDLKKIEDLIQSMHIDATLYTESDVHPSCKVTAMKCFLLELQVI
SLESGDASIHDTVENLIILANDSLSSNGNVTESGCKECEELEEKNIKEFLQSFVHIVQMFINTS
*Sequence identification of* ST-(G₄S)₃GGS-IL15 **(PF54)** *(SEQ. ID NO: 10):* 
STGGGGSGGGGSGGGGSGGSNWVNVISDLKKIEDLIQSMHIDATLYTESDVHPSCKVTAMKCFLLELQVI
SLESGDASIHDTVENLIILANNSLSSNGNVTESGCKECEELEEKNIKEFLQSFVHIVQMFINTS
*Sequence identification* of SYR-(G₄S)₈-IL15 **(PF55)** *(SEQ. ID NO: 11):* 
SYRGGGGSGGGGSGGGGSGGGGSGGGGSGGGGSGGGGSGGGGSNWVNVISDLKKIEDLIQSMHIDA
TLYTESDVHPSCKVTAMKCFLLELQVISLESGDASIHDTVENLIILANNSLSSNGNVTESGCKECEELEEKN
IKEFLQSFVHIVQMFINTS
*Sequence identification of* ST-cleavable linker 2-IL1 5(E46G,V49R) **(PF56)** *(SEQ. ID NO: 12):* 
MSTGGGGSGGGGSRASRANGSNWVNVISDLKKIEDLIQSMHIDATLYTESDVHPSCKVTAMKCFLLGLQ
RISLESGDASIHDTVENLIILANNSLSSNGNVTESGCKECEELEEKNIKEFLQSFVHIVQMFINTS
*Sequence identification* of SYR-(G₄S)₈-IL15Ra-IL-15 **(PF57)** *(SEQ. ID NO: 13):* 
(M)SYRGGGGSGGGGSGGGGSGGGGSGGGGSGGGGSGGGGSGGGGSITCPPPMSVEHADIWVKSY
SLYSRERYICNSGFKRKAGTSSLTECVLNKATNVAHWTTPSLKCIRDPALVHQRPAPPSGGSGGGGSGG
GSGGGGSLQNWVNVISDLKKIEDLIQSMHIDATLYTESDVHPSCKVTAMKCFLLELQVISLESGDASIHDT
VENLIILANNSLSSNGNVTESGCKECEELEEKNIKEFLQSFVHIVQMFINTS
*Sequence identification of* S-(G₄S)₈-IL15Rα-IL-15 **(PF58)** *(SEQ. ID NO: 14):* 
SGGGGSGGGGSGGGGSGGGGSGGGGSGGGGSGGGGSGGGGSITCPPPMSVEHADIWVKSYSLYSR
ERYICNSGFKRKAGTSSLTECVLNKATNVAHWTTPSLKCIRDPALVHQRPAPPSGGSGGGGSGGGSGG
GGSLQNWVNVISDLKKIEDLIQSMHIDATLYTESDVHPSCKVTAMKCFLLELQVISLESGDASIHDTVENLII
LANNSLSSNGNVTESGCKECEELEEKNIKEFLQSFVHIVQMFINTS
*Sequence identification of* ST-(G₄S)₈-IL15Rα-IL-15 **(PF59)** *(SEQ. ID NO: 15):* 
STGGGGSGGGGSGGGGSGGGGSGGGGSGGGGSGGGGSGGGGSITCPPPMSVEHADIWVKSYSLYS
RERYICNSGFKRKAGTSSLTECVLNKATNVAHWTTPSLKCIRDPALVHQRPAPPSGGSGGGGSGGGSG
GGGSLQNWVNVISDLKKIEDLIQSMHIDATLYTESDVHPSCKVTAMKCFLLELQVISLESGDASIHDTVENL
IILANNSLSSNGNVTESGCKECEELEEKNIKEFLQSFVHIVQMFINTS
*Sequence identification* of mPD-L1 light chain *(SEQ. ID NO: 16):* 
QSALTQPASVSGSPGQSITISCTGTSSDVGAYNYVSWYQQHPGKAPKLMIYDVSNRPSGVSNRFSGSKS
GNTASLTISGLQAEDEADYYCSSYTSSSTRVFGTGTKVTVLGQPKANPTVTLFPPSSEELQANKATLVCLI
SDFYPGAVTVAWKADGSPVKAGVETTKPSKQSNNKYAASSYLSLTPEQWKSHRSYSCQVTHEGSTVEK
TVAPTEC
*Sequence identification* of mPD-L1 heavy chain *(SEQ. ID NO: 17):* 
EVQLLESGGGLVQPGGSLRLSCAASGFTFSAYRMFWVRQAPGKGLEWVSSIYPSGGITFYADSVKGRFT
ISRDNSKNTLYLQMNSLRAEDTAIYYCARIKLGTVTTVDYWGQGTLVTVSSASTKGPSVFPLAPSSKSTSG
GTAALGCLVKDYFPEPVTVSWNSGALTSGVHTFPAVLQSSGLYSLSSVVTVPSSSLGTQTYICNVNHKPS
NTKVDKRVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPRE
PQVYTLPPSREEMTKNQVSLTCLVKGFYPSDIAVEWESNGQPENNYKTTPPVLDSDGSFFLYSKLTVDKS
RWQQGNVFSCSVMHEALHNHYTQKSLSLSPGK
*Sequence identification* of genetic fusion (IC1) heavy chain *(SEQ. ID NO: 18):* 
EVQLLESGGGLVQPGGSLRLSCAASGFTFSAYRMFWVRQAPGKGLEWVSSIYPSGGITFYADSVKGRFT
ISRDNSKNTLYLQMNSLRAEDTAIYYCARIKLGTVTTVDYWGQGTLVTVSSASTKGPSVFPLAPSSKSTSG
GTAALGCLVKDYFPEPVTVSWNSGALTSGVHTFPAVLQSSGLYSLSSVVTVPSSSLGTQTYICNVNHKPS
NTKVDKRVEPKSCDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPRE
PQVYTLPPSREEMTKNQVSLTCLVKGFYPSDIAVEWESNGQPENNYKTTPPVLDSDGSFFLYSKLTVDKS
RWQQGNVFSCSVMHEALHNHYTQKSLSLSPGSCPPPMSVEHADIWVKSYSLYSRERYICNSGFKRKAG
TSSLTECVLNKATNVAHWTTPSLKCIRDPALVHQRPAPPSGGSGGGGSGGGSGGGGSLQNWVNVISDL
KKIEDLIQSMHIDATLYTESDVHPSCKVTAMKCFLLELQVISLESGDASIHDTVENLIILANNSLSSNGNVTE
SGCKECEELEEKNIKEFLQSFVHIVQMFINTS
*Sequence identification* of humane IL-15 *(SEQ. ID NO: 19):*

NWVNVISDLKKIEDLIQSMHIDATLYTESDVHPSCKVTAMKCFLLELQVISLESGDASIHDTVENLIILA
NNSLSSNGNVTESGCKECEELEEKNIKEFLQSFVHIVQMFINTS

## Claims

1. An antibody-cytokine conjugate having structure (**1**) or structure (**2**) 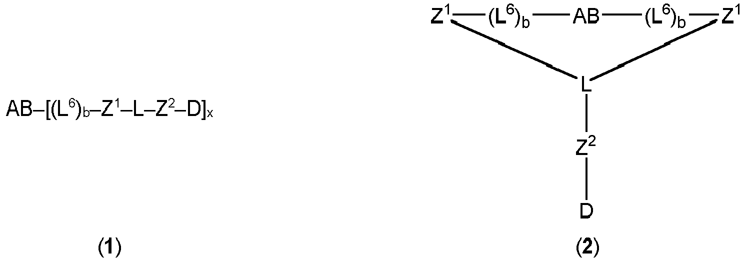 wherein:
- AB is an antibody;
- L is a linker that connects AB to D;
- x is an integer in the range of 1 - 10;
- Z¹ and Z² are connecting groups, wherein at least Z¹ comprises the product of a cycloaddition or a nucleophilic reaction;
- b is 0 or 1;
- L⁶ is -(H)ᵥ-S-(L⁷)_{w'}-, wherein:
∘ H is a monosaccharide,
∘ v is an integer in the range of 1 - 10,
∘ S is a sugar or a sugar derivative,
∘ w' is 0 or 1, and
∘ L⁷ is -N(H)C(O)-(CH₂-O)_{z}-(CH₂-CH₂-O)_{γ}-CH₂- or CH₂-(O-CH₂-CH₂)_{γ}-,wherein γ is an integer in the range of 0 - 100 and z is 0 or 1.
- D is a polypeptide according to -(D¹)_{α}-D², wherein:
∘ D¹ is a cleavable peptide linker;
∘ D² is an immune cell engager polypeptide specific for an immune cell receptor;
∘ α is 0 or 1;
- wherein L is a cleavable linker if α = 0.

2. The antibody-cytokine conjugate according to claim 1, wherein b = 1, preferably the chain of atoms between D² and AB is 10 - 100 atoms, preferably selected from B, C, O, N, S and P.

3. The antibody-cytokine conjugate according to any one of the preceding claims, wherein the antibody is specific for an extracellular receptor on a tumour cell, preferably wherein the extracellular receptor on the tumour cell is selected from the group consisting of 5T4 (TPBG), ADAM9, ALPP, ALPPL2, AMHRII, ASCT2 (SLC1A5), ASLG659, ASPHD1, av-integrin, avb3-integrin/ITGAV/CD51, Axl, B7-H3 (CD276), B7-H4 (VTCN1), BAFF-R, BCMA (CD269), BMPR1B, Brevican, c-KIT (CD117), c-Met, C4.4a (LYPD3), CA-IX (CA9)/MN, Cadherin-6, CanAg, CCR7, CD117 (c-KIT), CD123 (IL-3Rα), CD13, CD133, CD138/syndecan-1, CD166 (ALCAM), CD19, CD20, CD203C, CD205, Ly75, CD21, CD22, CD228 (P79, SEMF), CD25 (IL-2R-α), CD30 (TNFRSF8), CD324 (CDH1/E-cadherin), CD33, CD352 (SLAMF6, NTB-A), CD37, CD38, CD44v6, CD45, CD46, CD47, CD48a (SLAMF2), CD56 (NCAM), CD70, CD71 (TF-R), CD72, CD74, CD79a, CD79b, CDH6, Cadherin-6, CEACAM5 (CD66e), claudin, CLDN18.2, CLDN6 (claudin-6, Skullin), CLEC12A, CLL-1/CLEC12A, Cripto, CS1 (SLAMF7, CD319), CXCR5, DKL-1, DLL3 (delta-like 3), DPEP3, E16, EGFR, EGFRvlll, ENPP3, CD203c (AGS-16), EpCAM, EphA2, EphB2R, Ephrin-A4 (EFNA4), ETBR, FAP, FGFR2, FGFR3, fibronectin EDB , FLT3, FOLR1 (FR-a), Gal-3BP, GD3, GDNF-Ra1, GEDA, GFRA1, Globo H, GPC3 (glypican-3), gpNMB, GPR172A, GPR19, GPR54, GRP20, guanyl cyclase C (GCC), HER2, HER3, HLA-DOB, IGF-1R, IL13R, IL20Ra, Integrin avb6, KAAG-1, LAMP-1 (CD107a), Lewis Y (CD174), LGR5, LIV-1 (SLC39A6, ZIP6), LRRC15, LY64, Ly6E (lymph. antig 6), Ly6G6D, LY6K, mesothelin (MSLN), MFI2 (TAA), MICA/B, MOSPD2, MPF, MUC1 (CA6), MUC16/CA-125 , MUC1c, NaPi2b (SLC34A2), NCA, Nectin-4 (PVRL4), Notch3, P-cadherin (pCAD, CDH3), P2X5, PD-L1 (CD274, B7-H1), PMEL17, PRLR (prolactin), PSCA, PSMA, PTK7 (CCK4), RET, RNF43, RON, ROR1, ROR2, Serna 5b, SEZ6, SLITRK6 (SLC44A4), STEAP1, STEAP2, STn, TAG72, TENB2, TF (CD142, thromoplastin), TIM-1, TM4SF, TMEFF, TMEM118, TMEM46, TNF-alpha, TROP-2 (TACSTD2), TWEAKR, receptor tyrosine kinases (RTK), tenascin, or the antibody is specific for an extracellular protein resulting from a viral infection and/or for atumour-associated carbohydrate antigen (TACA) and/or wherein the immune cell-engaging polypeptide is selected from the group consisting of IL-1, IL-2, IL-3, IL-4, IL-5, IL-6, IL-7, IL-8, IL-9, IL-10, IL-11, IL-12, IL-13, IL-15, IL-16, IL-17, IL-18, IL-19, IL-20, IL-21, IL-22, IL-23,, IL-24, IL-26, IL-28, IL-29, IL-33, IL-36, IL37, IL-38, IFN-α (including IFN-α1/13, IFN-α2, IFN-α4, IFN-α5, IFN-α6, IFN-α7, IFN-α8, IFN-α10, IFN-α14, IFN-α16, IFN-α17, and IFN-α21), IFN- β, IFN-Y, IFN-A, TFN-α, TNF-β, TGF-β1, M-CSF, G-CSF, GM-CSF, and CXL10, more preferably the immune cell engaging polypeptide is IL-2 or IL-15, more preferably IL-15.

4. The antibody-cytokine conjugate according to any one of the preceding claims, wherein each Z¹ is individually selected from the structures (Z2) - (Z20c): 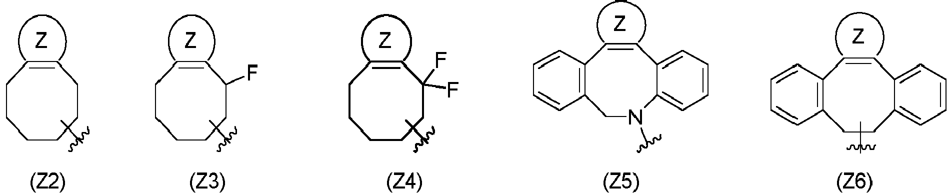 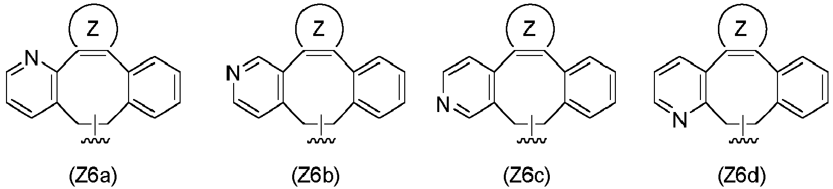 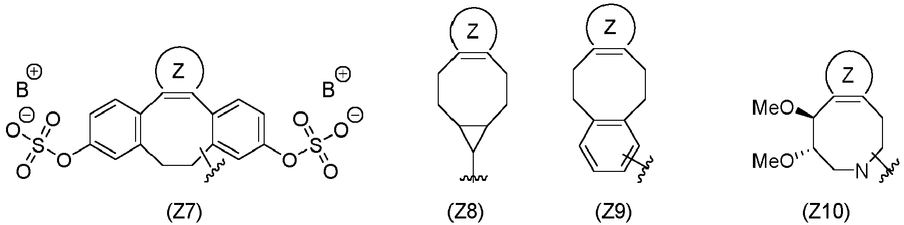 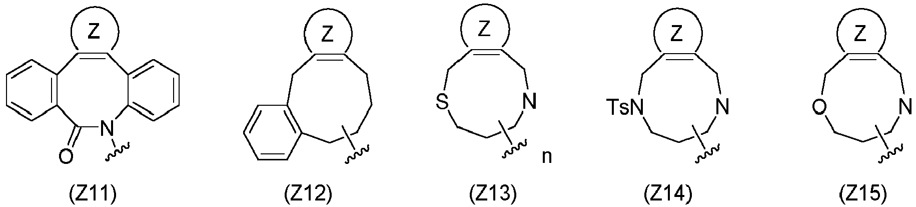 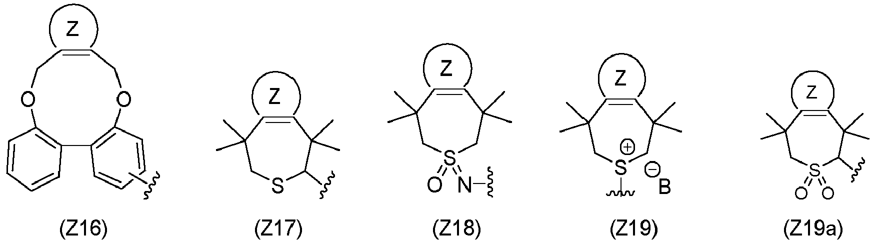 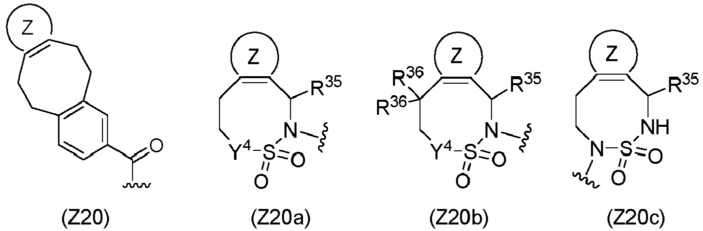 wherein the connection to L is represented by the wavy bond, B⁽⁻⁾ is an anion, B⁽⁺⁾ is a cation, the nitrogen atom of (Z10), (Z13), (Z14) and (Z15) may bear the connection to L, or contains a hydrogen atom or is substituted;
R³⁶ is an halogen selected from fluor, chlorine, bromine and iodine; Y⁴ is a heteroatom; R³⁵ is selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, Si, S and NR¹⁴ wherein R¹⁴ is independently selected from the group consisting of hydrogen and C₁ - C₄ alkyl groups;
ring Z is selected from a triazole, a cyclohexene, a cyclohexadiene, a [2.2.2]-bicyclooctadiene, a [2.2.2]-bicyclooctene, an isooxazoline, an isoxazolidine, a pyrazoline or a piperazine,
preferably Z¹ is selected from the structures (Z21) - (Z38a), more preferably Z¹ is (Z29): 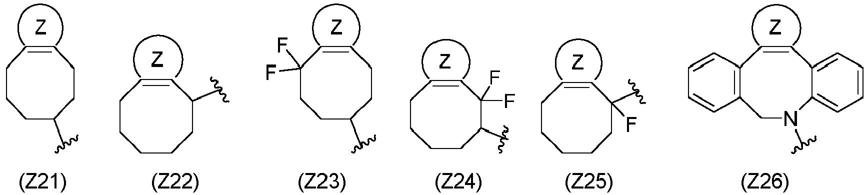 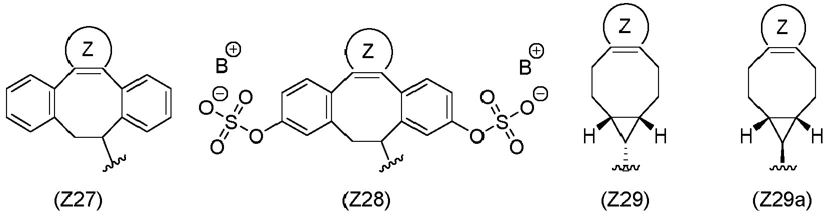 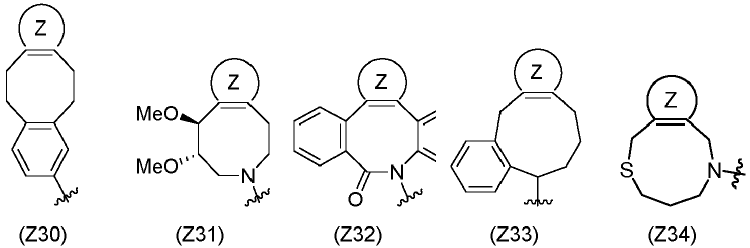 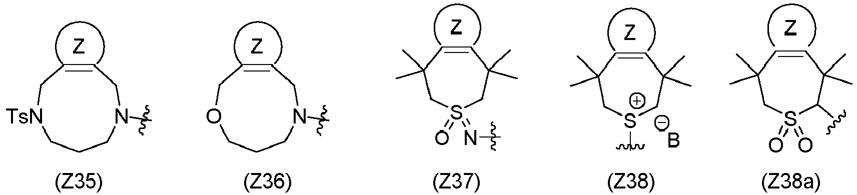 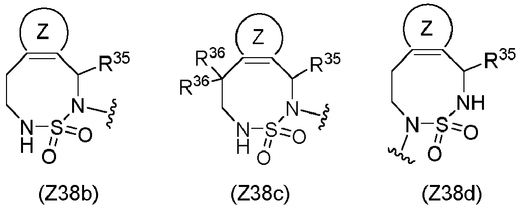 even more preferably, Z¹ is obtainable by a click reaction between cycloalkyne and azide and Z² is obtainable by a click reaction between cycloalkyne and nitrone.

5. The antibody-cytokine conjugate according to any one of the preceding claims, wherein linker L is a linker of structure
-(L¹)-(L²)ₒ-(L³)ₚ-(L⁴)_{q}- wherein:
- L¹ is connected to Z¹ and L⁴ if present is connected to Z²;
- L¹, L², L³ and L⁴ are each individually linkers that together link Z¹ to Z²;
- o, p and q are each individually 0 or 1, preferably o = p = q = 0; and preferably wherein:
a) linker L¹ is represented by:
-(W)ₖ-(A)_{d}-(B)ₑ-(A)_{f}-(B)_{e'}-(C(O))_{g}-, or
[-(W)ₖ-(A)_{d}-(B)ₑ-(A)_{f}-]₂BM-(C(O))_{g}- (A)_{d'}-(B)_{e'}-(A)_{f'}-(C(O))_{g'}-,
wherein:
- d and d' are individually 0 or 1,
- e and e' are individually an integer in the range 1-10,
- f and f are individually 0 or 1,
- g and g' are individually an integer in the range 0-10,
- k = 0 or 1 with the proviso that if k = 1 then d = 0,
- A is a sulfamide group according to structure (23) 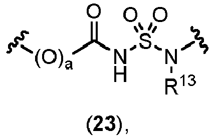 wherein a = 0 or 1, and R¹³ is selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, S and NR¹⁴ wherein R¹⁴ is independently selected from the group consisting of hydrogen and C₁ - C₄ alkyl groups;
- W is -OC(O)-, -C(O)O-, -C(O)NH-, -NHC(O)-, -OC(O)NH-, -NHC(O)O-, - (O)(CH2)ₘC(O)-, -C(O)(CH₂)ₘC(O)NH- or -(4-Ph)CH₂NHC(O)(CH₂)ₘC(O)NH-, wherein m is an integer in the range 0 - 10;
- B is a -CH₂-CH₂-O- or a -O-CH₂-CH₂- moiety, or (B)ₑ is a -(CH₂-CH₂-O)ₑ₁-CH₂-CH₂- or a -(CH₂-CH₂-O)ₑ₁-CH₂- moiety, wherein e1 is defined the same way as e;
- BM is a branching moiety, preferably selected from a carbon atom, a nitrogen atom, a phosphorus atom, a (hetero)aromatic ring, a (hetero)cycle or a polycyclic moiety, more preferably BM is a nitrogen atom.
and/or
b) linker L² is a peptide spacer, preferably wherein L² has a recognition sequence for proteases, preferably wherein L² comprises 1 - 5 amino acids, including optionally unnatural amino acids, more preferably a dipeptide, tripeptide or tetrapeptide spacer;
and/or
c) linker L³ is spacer that is associated with a cleaving mechanism, preferably L³ is selected from a para-aminobenzyoxycarbonyl derivative according to structure (L3a) or a glucuronic acid-functionalized *p*-hydroxybenzyloxycarbonyl (L3b): 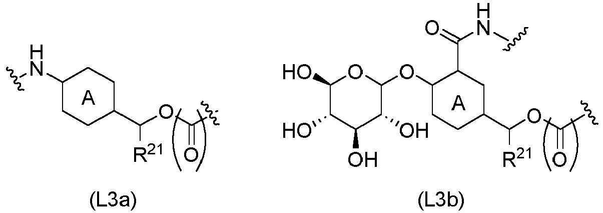 wherein the brackets indicate an optional carbonyl group, R²¹ is H, R²⁶ or C(O) R²⁶, wherein R²⁶ is C₁ - C₂₄ (hetero)alkyl groups, C₃ - C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NR²⁸ wherein R²⁸ is independently selected from the group consisting of hydrogen and C₁ - C₄ alkyl groups, preferably wherein R²¹ is H or C(O)R²⁶, wherein R²⁶ = 4-N-methyl-piperazine or morpholine, most preferably wherein R²¹ is H;
and/or
d) linker L⁴ is a connecting spacer, preferably L⁴ is a diamine according to -NR²²-(C_{z}-alkylene) - NR²²-(SO₂-NH-)ⱼ(C(O))ₕ-, wherein R²² is H or C₁ - C₄ alkyl, z is an integer in the range 1 - 10, j is 0 or 1, h is 0 or 1; or
linker L⁴ is a an ethyleneglycol spacer according to the structure -NR²²-(CH₂-CH₂-O)ₑ₆-(CH₂)ₑ₇-C(O)-, wherein e6 is an integer in the range 1 - 10, e7 is an integer in the range 1 - 3 and R²² is H or C₁ - C₄ alkyl; or
an aminoalkanoic acid spacer according to the structure - NR²²-(C_{z}-alkylene)-C(O)-, wherein x is an integer in the range 1 - 20 and R²² is H or C₁ - C₄ alkyl; or.

6. The antibody-cytokine conjugate according to any one of the preceding claims, wherein α = 1 and D is connected to Z² via the N-terminus of D¹.

7. The antibody-cytokine conjugate according to any one of the preceding claims, wherein D¹ contains a cleavable site that is recognized by one of a serine protease, a cysteine protease, an aspartate protease, a threonine protease, a glutamic acid protease, a metalloproteinase, a gelatinase, and an asparagine peptide lyase, a cathepsin B, a cathepsin C, a cathepsin D, a cathepsin E, a cathepsin G, a cathepsin K, a cathepsin L, a kallikrein, a hKI, a hKIO, a hKT5, a plasmin, a collagenase, a type IV collagenase, a stromelysin, a factor Xa, a chymotrypsin-like protease, a trypsin-like protease, a elastase-like protease, a subtilisin-like protease, an actinidain, a bromelain, a calpain, a caspase, a caspase-3, a mirl-CP, a papain, a HIV-I protease, a HSV protease, a CMV protease, a chymosin, a renin, a pepsin, a matriptase, a membrane type serine protease 1 (MT-SP1), a legumain, a plasmepsin, a nepenthesin, a metalloexopeptidase, a metalloendopeptidase, a matrix metalloprotease (MMP), a MMP1, a MMP2, a MMP3, a MMP7, a MMP8, a MMP9, a MMP1O, a MMP11, a MMP12, a MMP13, a MMP14, an ADAM10, an ADAM12, an urokinase plasminogen activator (uPA), an enterokinase, a prostate-specific target (PSA, hK3), an interleukin-1b converting enzyme, a thrombin, a FAP (FAP-α), a type II transmembrane serine protease (TTSP), a neutrophil elastase, a proteinase 3, a neutrophil serine protease 4, a mast cell chymase, a mast cell tryptase, a dipeptidyl peptidase and a dipeptidyl peptidase IV (DPPIV/CD26).

8. The antibody-cytokine conjugate according to any one of the preceding claims, wherein L is according to any one of the following structures: 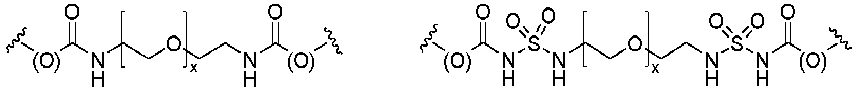 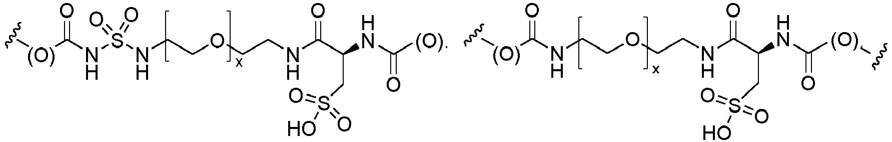 wherein x is in the range of 0 - 100, preferably x is in the range of 0 - 50, more preferably x is in the range of 1 - 30, most preferably x is 1 - 18.

9. The antibody-cytokine conjugate according to any one of claims 1 - 11, wherein L-Z² is as follows: 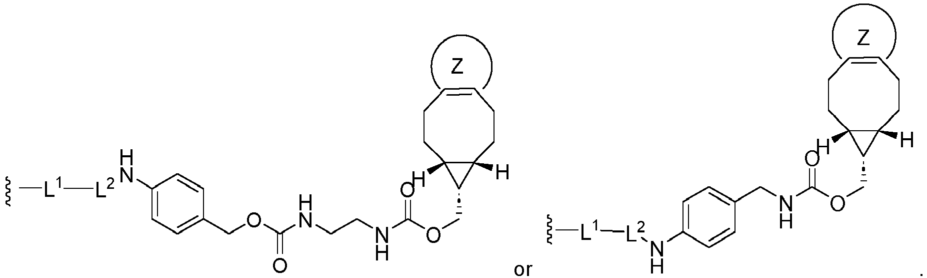 wherein
∘ the wavy bond connected to L¹ represents the connection to Z¹ and ring Z is defined as in claim 4, preferably wherein in the definition of D α is 0; and
∘ L¹ and L² are as defined in claim 5.

10. An antibody-cytokine conjugate according to any one of the preceding claims for use in medical treatment.

11. The antibody-cytokine conjugate according to any one of claims 1 -9 for use in the treatment of cancer or an autoimmune disease.

12. A pharmaceutical composition comprising the conjugate according to any one of claims 1 - 9 and a pharmaceutically acceptable carrier.

13. A process for preparing the antibody-cytokine conjugate according to any one of claims 1 - 9, wherein the process comprises following steps:
- reacting AB(F¹)ₓ, with Q¹-L-Q² or 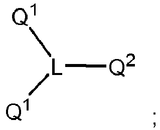
- and reacting the resulting compound with F²-D;
or the process comprises the following steps:
- reacting D-F² with Q¹-L-Q² or 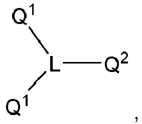
- and reacting the resulting compound with AB(F¹)ₓ
wherein AB(F¹)ₓ is a functionalized antibody containing x reactive moieties F¹ and x is an integer in the range of 1 -10, wherein Q¹ and F¹ are mutually reactive towards each other and Q² and F² are mutually reactive towards each other, wherein the reactions between Q¹ and F¹ and between Q² and F² form a covalent linkage between the reactants.

14. The process according to claim 13, wherein F¹ is an azide, Q¹ comprises a cycloalkyn, Q² comprises a trans-cyclooctene and F² comprises a nitrone or tetrazine.

15. The process according to claim 13 or 14, which is preceded by:
- a step in which a terminal serine or threonine of D is converted into a nitrone in order to obtain F²-D with F² being a nitrone, or
- a ligase-mediated ligation step to convert D into F²-D, preferably wherein the ligase is selected from tubulin tyrosine ligase, transglutaminase, lipoic acid ligase, farnesyl transferase, glycosyl transferase, formyl-glycine generating enzyme (FGE), trypsiligase/subtiligase, more preferably the ligase is sortase,
preferably wherein D is produced by microbial recombinant expression and then converted into D-F², more preferably the microbial is a prokaryote, most preferably an E. coli bacteria.
